# EUROPEAN PATENT SPECIFICATION

(11) **EP 3 565 846 B1**
(45) Date of publication and mention of the grant of the patent: **11.11.2026**
(21) Application number: 18736394.0
(22) Date of filing: 03.01.2018
(51) Int. Cl.: C07K 16/28, C07K 14/00, G01N 33/50, A61K 47/68

(54) **PROTEIN THERAPEUTICS FOR TREATMENT OF SENESCENT CELLS**
PROTEINTHERAPEUTIKA ZUR BEHANDLUNG VON SENESZENTEN ZELLEN
AGENTS THÉRAPEUTIQUES PROTÉIQUES POUR LE TRAITEMENT DE CELLULES SÉNESCENTES

(30) Priority: 03.01.2017 US 201762441745 P; 23.05.2017 US 201762509830 P
(43) Date of publication of application: 13.11.2019
(73) Proprietor: BioAtla, Inc., San Diego, CA 92121 (US)
(72) Inventor: SHORT, Jay M., Jackson, Wyoming 83001 (US)
(74) Representative: De Vries & Metman
(86) International application number: PCT/US2018/012136
(87) International publication number: WO 2018/129007

(56) References cited:
- WO-A1-2015/181526
- WO-A1-2016/036916
- WO-A1-2016/138071
- JP-A- 2012 523 239
- KR-A- 20160 144 846
- US-A1- 2012 258 865
- US-A1- 2016 207 989
- HANDAYANINGSIH, ANASTASIA-EVI ET AL.: "IGF-I enhances cellular senescence via the reactive oxygen species-p53 pathway", BIOCHEMICAL AND BIOPHYSICAL RESEARCH COMMUNICATIONS, vol. 425, no. 2, August 2012 (2012-08-01), pages 478 - 484, XP055511853

## Description

### FIELD OF THE DISCLOSURE

This disclosure relates to the field of treating or clearing senescent cells and/or treating diseases or disorders related to senescent cells. Particularly, this disclosure relates to conditionally active antibodies or antibody fragments that target senescent cells and to methods of generating such conditionally active senolytic antibodies or antibody fragments.

### BACKGROUND OF THE DISCLOSURE

Senescent cells are metabolically active but trapped in the G1 phase of cell growth cycle with their lifespan controlled by multiple dominant genes (Stanulis-Praeger, Mech. Ageing Dev., vol. 38, pp.1-48, 1987). Senescent cells differ from quiescent cells and terminal differentiated cells in several important aspects, having characteristic morphological changes such as enlargement, flattening, and increased granularity (Dimri et al., Proc. Nat. Acad. Sci. USA, vol. 92, pp.9363-9367, 1995). Senescent cells do not divide even if stimulated by mitogens (Campisi, Trends Cell Biol., vol. 11, pp. S27-S31, 2001). Senescence involves activation of p53 and/or Rb and their regulators such as p16INK4a, p21, and ARF. Except when p53 or Rb is inactivated, senescence is generally irreversible.

Senescent cells express increased levels of plasminogen activator inhibitor (PAI) and exhibit staining for β-galactosidase activity at pH 6 (Sharpless et al., J. Clin. Invest., vol. 113, pp.160-168, 2004). Irreversible G1 arrest is mediated by inactivation of cyclin dependent kinase (CdK) complexes which phosphorylate Rb. P21 accumulates in senescent cells, which inhibits CdK4-CdK6. P16 also inhibits CdK4-CdK6 and accumulates in senescent cells proportionally with β-galactosidase activity and cell volume (Stein et al., Mol. Cell. Biol., vol. 19, pp.2109-2117, 1999). Evidence suggests that p21 is expressed during initiation of senescence but not required for maintaining senescence, while p16 expression helps maintain senescence once initiated.

Since in some cases senescence is related to the progressive shortening of telomeres with each cell division, senescence is triggered when certain chromosomal telomeres reach a critical length (Mathon and Lloyd, Nat. Rev. Cancer, vol. 3, pp.203-213, 2001; Martins, U. M. Exp Cell Res., vol. 256, pp.291-299, 2000). Senescence can be abrogated by the expression of telomerase which lengthens telomeres. For example, human fibroblasts undergo replication indefinitely when the fibroblasts are transfected to express telomerase. Most cancer cells express telomerase in order to maintain telomere length and replicate indefinitely. The minority of cancer cells that do not express telomerase have alternative mechanisms for lengthening of telomeres (ALTs).

There are also other causes of senescence. Collectively, these other causes are often referred to as stress-induced premature senescence (SIPS). Oxidative stress can shorten telomeres thereby inducing senescence (von Zglinicki, Trends Biochem. Sci., vol. 27, pp.339-344, 2002). Hyperoxia has been shown to induce senescence. Gamma irradiation of human fibroblasts in early to mid G1 phase causes senescence in a p53-dependent manner (Di Leonardo et al., Genes Dev., vol. 8, pp.2540-2551, 1994). Ultraviolet radiation also induces senescence. Other agents that can induce senescence include hydrogen peroxide (Krtolica et al., Proc. Nat. Acad. Sci. USA, vol. 98, pp.12072-12077, 2001), sodium butyrate, 5-azacytadine, and transfection with the Ras oncogene (Tominaga, Mech. Ageing Dev., vol. 123, pp.927-936, 2002). Chemotherapeutic agents including doxorubicin, cisplatin, and a host of others have been shown to induce senescence in cancer cells (Roninson, Cancer Res., vol. 63, pp.2705-2715, 2003). 5-bromodeoxyuridine treatment results in senescence in both normal and malignant cells (Michishita et al., J. Biochem., vol. 126, pp.1052-1059, 1999). Generally speaking, agents that damage DNA are capable of causing senescence.

Evidence suggests a relationship between senescence and aging. Cultured cells from old donors exhibit senescence after fewer growth cycles than cells from young donors (Martin et al., Lab. Invest., vol. 23, pp.86-92, 1970; Schneider et al., Proc. Nat. Acad. Sci. USA, vol. 73, pp.3584-3588, 1976). Cells from short-lived species senesce after fewer growth cycles than cells from long-lived species (Rohme, D., Proc. Nat. Acad. Sci. USA, vol. 78, pp.5009-3320, 1981). Cultured cells from donors with hereditary premature aging syndromes such as Werner's syndrome show senescence after fewer growth cycles than cells from age-matched controls.

Senescence confers functional changes on the senescent cells which have been associated with various age-related diseases and disorders (Chang et al., Proc. Nat. Acad. Sci. USA, vol. 97, pp.4291-4296, 2000). Senescent cells accumulate in tissues and organs of individuals as they age and are found at sites of age-related pathologies. Given that senescent cells have been causally implicated in certain aspects of age-related decline in health and may contribute to certain diseases, and are also induced as a result of necessary life-preserving chemotherapeutic and radiation treatments, the presence of senescent cells may have deleterious effects to millions of patients worldwide. It is widely believed that selective elimination of senescent cells can prevent and treat age-related diseases and disorders.

Senescent cells can also promote tumorigenesis. Senescent stromal cells express tumor promoting factors that exert a paracrine effect on neighboring epithelial cells. These effects include mitogenicity and anti-apoptosis (Chang et al., Proc. Nat. Acad. Sci. USA, vol. 97, pp.4291-4296, 2000). Senescent fibroblasts have been shown to stimulate premalignant and malignant epithelial cells but not normal epithelial cells to form tumors in mice. This occurred when as few as 10% of the fibroblasts were senescent (Krtolica et al., Proc. Nat. Acad. Sci. USA, vol. 98, pp.12072-12077, 2001). Tumor promoting factors secreted by senescent cells are partly mediated by p21waf1/cip1/sdi1 (Roninson, Cancer Res., vol. 63, pp.2705-2715, 2003). A threshold of senescent stromal cells appears to provide a milieu allowing adjacent premalignant epithelial cells to survive, migrate, and divide (Campisi, Nat. Rev. Cancer, vol. 3, pp. 339-349, 2003).

Consequently, therapeutics targeting senescent cells are a promising treatment option for senescence-associated diseases and disorders. US 2016/0038576 discloses an immunogenic composition for inducing an adaptive immune response directed specifically at senescent cells for treatment and prophylaxis of age-related diseases and disorders, and other diseases and disorders associated with or exacerbated by the presence of senescent cells. The immunogenic composition comprises at least one or more of senescent cell-associated antigens, polynucleotides encoding senescent cell-associated antigens, and recombinant expression vectors comprising the polynucleotides for use in administering to a subject.

WO 2015116740 discloses a method of administering a therapeutically-effective amount of a small molecule senolytic agent that selectively kills senescent cells as compared with non-senescent cells for treatment of senescent cell-associated diseases and disorders. The senescent cell-associated diseases and disorders treatable by the method include cardiovascular diseases and disorders associated with or caused by arteriosclerosis, such as atherosclerosis, idiopathic pulmonary fibrosis, chronic obstructive pulmonary disease, osteoarthritis, senescence-associated ophthalmic diseases and disorders, and senescence-associated dermatological diseases and disorders.

US 2015/0064137 discloses a polypeptide and viruses comprising a polypeptide useful for selective elimination of senescent cells. The polypeptide and viruses can induce apoptosis in senescent cells. The polypeptide is selected from products of pro-apoptotic genes. The viruses comprise the pro-apoptotic gene for which expression is regulated by the p16 promoter. The p16 promoter can be a canonical p16 promoter or a non-canonical p16 promoter.

These therapeutics target one or more proteins of senescent cells to kill or remove senescent cells. However, these targeted proteins of senescent cells may also be present on other types of cells which may lead to undesirable side-effects. Thus, it would be advantageous to develop a class of therapeutic proteins that preferentially and/or specifically bind to a target on senescent cells, while minimizing or eliminating binding to the same target on other types of cells.

### SUMMARY OF THE DISCLOSURE

The invention provides a method of producing a conditionally active senolytic antibody or antibody fragment that binds to a target associated with a senescent cell according to the appended claims. The method of producing a conditionally active senolytic antibody or antibody fragment that binds to a target associated with a senescent cell from a parent antibody or antibody fragment that binds to the target associated with the senescent cell comprises the steps of:
(i) evolving a DNA encoding the parent antibody or antibody fragment using one or more evolutionary techniques to create mutant DNAs;
(ii) expressing the mutant DNAs to obtain mutantantibodies or antibody fragments;
(iii) subjecting the mutant antibody or antibody fragments to an assay of binding activity to the target under an extracellular pH of the senescent cell of from 5.5-7.0 and an assay of binding activity to the target under a normal physiological pH of 7.2-7.8; and
(iv) selecting the conditionally active senolyticsenolytic antibody or antibody fragment from the mutant antibodies or antibody fragments subjected to the assays in step (iii) that exhibits at least one of:
   (a) a decrease in binding activity to the target in the assay under the normal physiological pH of 7.2-7.8 compared to the same binding activity of the parent antibody or antibody fragment in the same assay, and an increase in binding activity to the target in the assay under the extracellular pH of 5.5-7.0 of the senescent cell compared to the same binding activity to the target of the conditionally active senolyticsenolytic antibody or antibody fragment in the assay under the normal physiological pH of 7.2-7.8; and
   (b) a decrease in the binding activity to the target in the assay under the normal physiological pH of 7.2-7.8 compared to the same binding activity to the target of the parent antibody or antibody fragment in the same assay and an increase in the binding activity of the target in the assay under the extracellular pH of 5.5-7.0 of the senescent cell compared to the same binding activity to the target of the parent antibody or antibody fragment in the assay under the extracellular pH of 5.5-7.0 of the senescent cell; and wherein the target is selected from at least one of APC, ARHGAP1, ARMCX-3, AXL, B2MG, BCL2L1, CAPNS2, CD261, CD39, CD54, CD73, CD95, CDC42, CDKN2C, CLYBL, COPG1, CRKL, DCR1, DCR2, DCR3, DEP1, DGKA, EBP, EBP50, FASL, FGF1, GBA3, GIT2, ICAM1, ICAM3, IGF1, ISG20, ITGAV, KITLG, LaminB1, LANCL1, LCMT2, LPHN1, MADCAM1, MAG, MAP3K14, MAPK, MEF2C, miR22, MMP3, MTHFD2, NAIP, NAPG, NCKAP1, Nectin4, NNMT, NOTCH3, NTAL, OPG, OSBPL3, p16, pl6INK4a, p19, p21, p53, PAI1, PARK2, PFN1, PGM, PLD3, PMS2, POU5F1, PPP1A, PPP1CB, PRKRA, PRPF19, PRTG, RAC1, RAPGEF1, RET, Smurf2, STX4, VAMP3, VIT, VPS26A, WEE1, YAP1, YH2AX, and YWHAE. In addition, it should be recognized that the target may be any combination of the preceding.

In each of the foregoing embodiments, a ratio of the activity of the conditionally active antibody or antibody fragment in the assay under the extracellular pHof the senescent cell to the activity of the conditionally active antibody or antibody fragment in the assay under the normal physiological pHmay be at least about1.3:1, or at least about 2:1, or at least about 3:1, or at least about 4:1, or at least about 5:1, or at least about 6:1, or at least about 7:1, or at least about 8:1, or at least about 9:1, or at least about 10:1, or at least about 11:1, or at least about 12:1, or at least about 13:1, or at least about 14:1, or at least about 15:1, or at least about 16:1, or at least about 17:1, or at least about 18:1, or at least about 19:1, or at least about 20:1, or at least about 30:1, or at least about 40:1, or at least about 50:1, or at least about 60:1, or at least about 70:1, or at least about 80:1, or at least about 90:1, or at least about 100:1.

In each of the embodiments, the extracellular pHof the senescent cell is a pH in a range of from about 5.5 to about 7.0, or from about 6.0 to about 7.0, or from about 6.2 to about 6.8.

In each of the foregoing embodiments, the normal physiological pH is a pH in a range of from about 7.2 to about 7.8, or from about 7.2 to about 7.6, or from about 7.4 to about 7.6.

In each of the foregoing embodiments, the assay under the normal physiological pH and the assay under the extracellular pH of the senescent cell may be performed in assay solutions containing at least one component selected from an inorganic compound, an ion and an organic molecule. In this embodiment, the at least one component may have substantially the same concentration in the assay solutions for both the assay under the normal physiological pH and the assay under the extracellular pH of the senescent cell. In these embodiments, the at least one component may be the inorganic compound and is selected from boric acid, calcium chloride, calcium nitrate, di-ammonium phosphate, magnesium sulfate, mono-ammonium phosphate, mono-potassium phosphate, potassium chloride, potassium sulfate, copper sulfate, iron sulfate, manganese sulfate, zinc sulfate, magnesium sulfate, calcium nitrate, calcium chelate, copper chelate, iron chelate, iron chelate, manganese chelate, zinc chelate, ammonium molybdate, ammonium sulphate, calcium carbonate, magnesium phosphate, potassium bicarbonate, potassium nitrate, hydrochloric acid, carbon dioxide, sulfuric acid, phosphoric acid, carbonic acid, uric acid, hydrogen chloride, and urea. In these embodiments, the at least one component may be the ion and is selected from a phosphorus ion, a sulfur ion, a chloride ion, a magnesium ion, a sodium ion, a potassium ion, an ammonium ion, an iron ion, a zinc ion, and a copper ion. In these embodiments, the at least one component may be selected from one or more of uric acid in concentration range of 2-7.0 mg/dL, calcium ion in a concentration range of 8.2-11.6 mg/dL, chloride ion in a concentration range of 355-381 mg/dL, iron ion in a concentration range of 0.028-0.210 mg/dL, potassium ion in a concentration range of 12.1-25.4 mg/dL, sodium ion in a concentration range of 300-330 mg/dL, and carbonic acid in a concentration range of 15-30 mM. In these embodiments, the at least one component may be the organic molecule and is an amino acid selected from Histidine, Alanine, Isoleucine, Arginine, Leucine, Asparagine, Lysine, Aspartic acid, Methionine, Cysteine, Phenylalanine, Glutamic acid, Threonine, Glutamine, Tryptophan, Glycine, Valine, Pyrrolysine, Proline, Selenocysteine, Serine, and Tyrosine. In these embodiments, the at least one component may be an organic acid selected from citric acid, α-ketoglutaric acid, succinic acid, malic acid, fumaric acid, acetoacetic acid, β-hydroxybutyric acid, lactic acid, pyruvic acid, α-ketonic acid, acetic acid, and volatile fatty acids. In these embodiments, the at least one component may be a sugar selected from glucose, pentose, hexose, xylose, ribose, mannose, galactose, lactose, GlcNAcβ1-3Gal, Galα1-4Gal, Manα1-2Man, GalNAcβ1-3Gal, and O-, N-, C-, and S-glycosides. In these embodiments, the at least one component may be selected from magnesium ion, sulfate ion, bisulfate ion, carbonate ion, bicarbonate ion, nitrate ion, nitrite ion, phosphate ion, hydrogen phosphate ion, dihydrogen phosphate ion, persulfate ion, monopersulfate ion, borate ion, and ammonium ion.

In each of the foregoing embodiments, the extracellular pH of the senescent cell may be a first pH in a range of from about 5.5 to about 7.0 and the normal physiological pH may be a second pH in a range of from about 7.2 to about 7.8, and the one or more assays may be performed in assay solutions containing at least one species having a molecular weight of less than 900 a.m.u. and a pKa up to 0.5, 1, 2, 3, or 4 pH units away from said first pH.

In each of the foregoing embodiments, the extracellular pH of the senescent cell may be a first pH in a range of from about 5.5 to about 7.0 and the normal physiological pH may be a second pH in a range of from about 7.2 to about 7.8, the one or more assays may be performed in assay solutions containing at least one species having a molecular weight of less than 900 a.m.u., and said species may have a pKa between said first pH and said second pH.

In each of the foregoing embodiments, the extracellular pH of the senescent cell may be a first pH in a range of from about 5.5 to about 7.0 and the normal physiological pH may be a second pH in a range of from about 7.2 to about 7.8, and the one or more assays may be performed in assay solutions containing at least one species selected from histidine, histamine, hydrogenated adenosine diphosphate, hydrogenated adenosine triphosphate, citrate, bicarbonate, acetate, lactate, bisulfide, hydrogen sulfide, ammonium, and dihydrogen phosphate.

In each of the foregoing embodiments, the selecting step (iv) may comprise selecting a conditionally active senolytic antibody or antibody fragment that exhibits (a) a decrease in binding activity to the target in the assay under the normal physiological pH of 7.2-7.8 compared to the same binding activity to the target of the parent antibody or antibody fragment in the same assay, and an increase in the binding activity to the target in the assay under the extracellular pH of 5.5-7.0 of the senescent cell compared to the same binding activity to the target of the conditionally active senolytic antibody or antibody fragment in the assay under the normal physiologicalpH of 7.2-7.8.

In each of the foregoing embodiments, the selecting step (iv) may comprise selecting a conditionally active senolytic antibody or antibody fragment that exhibits (b) a decrease in the binding activity to the target in the assay under the normal physiological pH of 7.2-7.8 compared to the same activity of the parent antibody or antibody fragment in the same assay and an increase in the activity in the assay under the extracellular pH of 5.5-7.0 of the senescent cell compared to the same activity of the parent antibody or antibody fragment in the assay under the extracellular pH of 5.5-7.0 of the senescent cell.

The disclosure provides a conditionally active senolytic antibody or antibody fragment produced by any of the foregoing methods. The antibody may be a single chain antibody or an antibody fragment. The antibody may be suitable to be engineered as part of chimeric antigen receptor of T-cells. The antibody may be a humanized antibody, a bispecific antibody, or a multispecific antibody.

The conditionally active senolytic antibody or antibody fragment may be conjugated to a masking moiety by a linker. The masking moiety reduces a binding activity of the conditionally active antibody to the target by at least at least 50%, 55%, 60%, 65%, 70%, 75%, 80%, 85%, 90%, 92%, 93%, 94%, 95%, 96%, 97%, 98%, 99% or even 100%.

In each of the foregoing embodiments, the linker may be covalently bonded to a variable region of the conditionally active antibody.

In each of the foregoing embodiments, the masking moiety may specifically bind to a variable region of the conditionally active antibody.

In each of the foregoing embodiments, the linker may comprise a flexible region and a cleavage site.

In each of the foregoing embodiments, the cleavage site may be cleaved by a protease in the extracellular environment of the senescent cell.

In each of the foregoing embodiments, the conditionally active senolytic antibody or antibody fragment may be conjugated to a cytotoxic drug, a cytostatic drug, or an anti-proliferative drug by a linker.

In each of the foregoing embodiments, the linker may comprise a cleavage site of at least one protease in the extracellular environment of the senescent cell. The at least one protease is selected from ADAM10, ADAM12, ADAM17, ADAMTS, ADAMTS5, BACE, Caspase 1-14, Cathepsin A, Cathepsin B, Cathepsin D, Cathepsin E, Cathepsin K, Cathepsin S, FAP, MT1-MMP, Granzyme B, Guanidinobenzoatase, Hepsin, Human Neutrophil Elastase, Legumain, Matriptase 2, Meprin, MMP1-17, MT-SP1, Neprilysin, NS3/4A, Plasmin, PSA, PSMA, TRACE, TMPRSS 3, TMPRSS 4, and uPA.

In another embodiment, the disclosure provides pharmaceutical composition comprising an effective amount of any of the foregoing conditionally active senolytic antibodies or antibody fragments and a pharmaceutically acceptable carrier.

In still another embodiment, the disclosure provides a method of treatment of aging, or of a senescent cell-associated disease or disorder comprising a step of administering any of the foregoing conditionally active antibodies or antibody fragments or any of the foregoing pharmaceutical compositions. In the foregoing embodiment, the senescent cell-associated disease or disorder may be selected from cognitive diseases, cardiovascular disease, metabolic diseases and disorders, motor function diseases and disorders, cerebrovascular disease, emphysema, osteoarthritis, pulmonary diseases, inflammatory/autoimmune diseases and disorders, ophthalmic diseases or disorders, metastasis, a chemotherapy or radiotherapy side effect, aging-related diseases and disorders , fibrotic diseases and disorders.

In yet another embodiment, the disclosure provides a method for generating a conditionally active molecule that has a molecular weight of less than about 3000 a.m.u from a parent organic compound. The method includes steps of modifying the parent organic compound by introducing one or more partially charged or charged groups into the parent organic compound to produce one or more modified organic compounds; and selecting the modified organic compound that exhibits a higher activity in the assay under the aberrant pHcompared to the same activity in the assay under the normal physiological condition.

In yet another embodiment, the disclosure provides a method for generating a conditionally active molecule that has a molecular weight of less than about 3000 a.m.u from a parent organic compound, comprising steps of: modifying the parent organic compound by removing one or more partially charged or charged groups from the parent organic compound to produce one or more modified organic compounds; and selecting the modified organic compound that exhibits a higher activity in the assay under the aberrant pH compared to the same activity in the assay under the normal physiological condition.

In yet another embodiment, the disclosure provides a method for generating a conditionally active molecule that has a molecular weight of less than about 3000 a.m.u from a parent organic compound, comprising steps of: modifying the parent organic compound by replacing one or more groups of the parent organic compound with one or more partially charged or charged groups to produce one or more modified organic compounds; and selecting the modified organic compound that exhibits a higher activity in the assay under the aberrant pH compared to the same activity in the assay under the normal physiological condition.

In each of the foregoing methods, the parent organic compound may have a molecular weight in a range of from about 100 a.m.u. to about 3000 a.m.u, or from about 100 a.m.u., to about 1500 a.m.u., or from about 150 a.m.u., to about 1250 a.m.u., or from about 300 a.m.u., to about 1100 a.m.u., or from about 400 a.m.u., to about 1000 a.m.u.

In each of the foregoing methods, the aberrant pH may be a value of an extracellular pH of a senescent cell and the normal physiological pH is different value of a same extracellular pH of a normal cell.

In each of the foregoing methods, the aberrant pH may be a pH in the range of from about 5.0 to about 7.0, or from about 5.5 to about 7.0, or from about 6.0 to about 7.0, or from about 6.2 to about 6.8, and the normal physiological pH is a pH in the range of from about 7.0 to about 7.8, or from about 7.2 to about 7.8, or from about 7.2 to about 7.6.

In each of the forgoing methods, the conditionally active antibody or antibody fragment may be conjugated to an agent selected from toxic agents, radioactive agents, or D retro inverso peptides.

In each of the foregoing embodiments, the D retro inverso peptides may comprise LTLRKEPASE IAQSILEAYS QNGWANRRSG GKRP (SEQ ID NO:5), LTLRKEPASE IAQSILEAYS QNGWANRRSG GKRPPPRRRQ RRKKRG (SEQ ID NO:6), or SEIAQSILEAYSQNGW (SEQ ID NO:7).

### BRIEF DESCRIPTION OF THE DRAWINGS

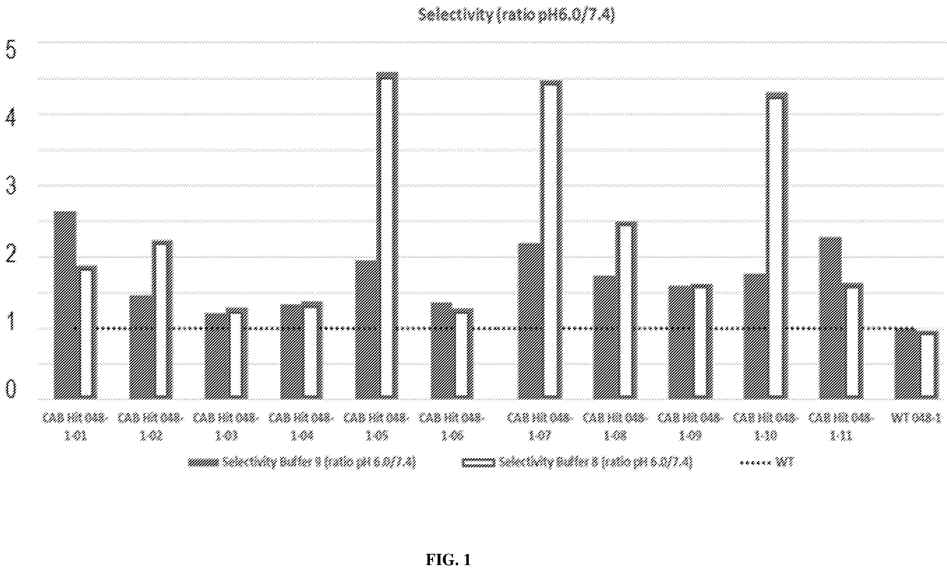

FIG. 1 is a plot showing the selectivity of the conditionally active antibodies selected in Example 9 at pH 6.0 over pH 7.4.
FIG. 2 is a diagram showing the formation of salt bridges in deoxyhemoglobin, where three amino acid residues form two salt bridges stabilize the T quaternary structure of the deoxyhemoglobin, leading to lower affinity to oxygen.
FIG. 3 is a diagram showing the structure of a chimeric antigen receptor (CAR).
FIG. 4 shows the binding activity of conditionally active antibodies to an antigen assayed in different buffer solutions.
FIG. 5 shows the effects of changing the composition of Krebs buffer on the binding activity of a conditionally active antibody.
FIG. 6 shows that the binding activities of three different conditionally active antibodies were dependent on the presence and concentration of bicarbonate at pH 7.4, as described in Example 12.
FIG. 7 shows the design principle for a D retro inverso (DRI) peptide of a natural or wild-type peptide.
FIG. 8 shows signaling pathways that regulate the FOXO family, including FOXO4. "+p" indicates phosphorylation, "-p" indicates dephosphorylation, "+m" indicates methylation, an arrow indicates activation, and a line with a cross bar at its end indicates inhibition, each relating to a target gene.
FIG. 9A shows untreated MCF-7 cells.
Fig. 9B shows MCF-7 cells treated with 1 µM of Palbociclib Isethionate.
FIG. 9C shows separation of untreated and treated MCF-7 cells by fluorescence activated cell sorting (FACS).
FIG. 9D shows target expression profiles for untreated MCF-7 cells and MCF-7 cells treated with Palbociclib Isethionate.
FIG. 10A shows untreated MDA-MB231 cells.
FIG. 10B shows MDA-MB231 cells treated with 1 µM of Palbociclib Isethionate.
FIG. 10C shows separation of untreated MDA-MB231 cells and MDA-MB231 cells treated with Palbociclib Isethionate by FACS.
FIG. 10D shows target expression profiles for untreated MDA-MB231 cells untreated and MDA-MB231 cells treated with Palbociclib Isethionate.
FIG. 11A shows untreated MDA-MB468 cells.
FIG. 11B shows MDA-MB468 cells treated with 1 µM of Palbociclib Isethionate.
FIG. 11C shows that the untreated MDA-MB468 cells and the MDA-MB468 cells treated with Palbociclib Isethionate were not separated by FACS.
FIG. 11D shows similar target expression profiles for untreated MDA-MB468 cells and MDA-MB468 cells treated with Palbociclib Isethionate.
FIG. 12A shows untreated MDA-MB231 cells.
FIG. 12B shows MDA-MB231 cells treated with Palbociclib Isethionate.
FIG. 13A shows untreated MDA-MB468 cells.
FIG. 13B shows MDA-MB468 cells treated with Palbociclib Isethionate.
FIG. 14A shows FACS cell sorting of untreated MDA-MB231 cells that were B-gal staining negative.
FIG. 14B shows FACS cell sorting of MDA-MB231 cells treated with Palbociclib Isethionate that were B-gal staining negative.
FIG. 14C shows FACS cell sorting of untreated MDA-MB231 cells that were B-gal staining positive.
FIG. 14D shows FACS cell sorting of MDA-MB231 cells treated with Palbociclib Isethionate that were B-gal staining positive.
FIG. 15A shows FACS sorting of untreated MDA-MB231 cells.
FIG. 15B shows FACS sorting of MDA-MB231 cells treated with Palbociclib Isethionate.
FIG. 16A shows FACS cell sorting of untreated MDA-MB468 cells that were B-gal staining negative.
FIG. 16B shows FACS cell sorting of MDA-MB468 cells treated with Palbociclib Isethionate that were B-gal staining negative.
FIG. 16C shows FACS cell sorting of untreated MDA-MB468 cells that were B-gal staining positive.
FIG. 16D shows FACS cell sorting of MDA-MB468 cells treated with Palbociclib Isethionate that were B-gal staining positive.
FIG. 17A shows FACS sorting of untreated MDA-MB468 cells.
FIG. 17B shows FACS sorting of MDA-MB468 cells treated with Palbociclib Isethionate.
FIG. 18A shows CD73 expression levels in MDA-MB231 and MDA-MB468 cells before and after the Palbociclib Isethionate treatment.
FIG. 18B shows senescent cell killing by an anti-CD73 conditionally active antibody.

### DEFINITIONS

In order to facilitate understanding of the examples provided herein, certain frequently occurring methods and/or terms will be defined herein.

The definitions of the terms "about," "activity," "agent," "ambiguous base requirement," "amino acid," "amplification," "chimeric property," "cognate," "comparison window," "conservative amino acid substitutions," "corresponds to," "degrading effective," "defined sequence framework," "digestion," "directional ligation," "DNA shuffling," "drug" or "drug molecule," "effective amount," "electrolyte," "epitope," "enzyme," "evolution" or "evolving," "fragment," "derivative," "analog," "full range of single amino acid substitutions," "gene," "genetic instability," "heterologous," "homologous" or "homeologous," "industrial applications," "identical" or "identity," "areas of identity," "isolated," "isolated nucleic acid," "ligand," "ligation," "linker" or "spacer," "microenvironment," "molecular property to be evolved," "mutations," "naturally-occurring," "normal physiological conditions" or "wild type operating conditions," "nucleic acid molecule," "nucleic acid molecule," "nucleic acid sequence coding for" or "DNA coding sequence of" or a "nucleotide sequence encoding," "promotor sequence," "nucleic acid encoding an enzyme (protein)" or "DNA encoding an enzyme (protein)" or "polynucleotide encoding an enzyme (protein)," "specific nucleic acid molecule species," "assembling a working nucleic acid sample into a nucleic acid library," "nucleic acid library," "nucleic acid construct" or "nucleotide construct" or "DNA construct," "construct," "oligonucleotide" or "oligo," "homologous," "operably linked," "operably linked to," "parental polynucleotide set," "patient" or "subject," "physiological conditions," "population," "pro-form," "pre-pro-form," "pseudorandom," "quasi-repeated units," "random peptide library," "random peptide sequence," "receptor," "recombinant," "synthetic," "related polynucleotides," "reductive reassortment," "reference sequence," "comparison window," "sequence identity," "percentage of sequence identity," "substantial identity," "reference sequence," "repetitive index (RI)", "restriction site," "selectable polynucleotide," "sequence identity," "similarity," "specifically bind," "specific hybridization," "specific polynucleotide," "stringent hybridization conditions," "substantially identical," "substantially pure enzyme," "substantially pure," "treating," "variable segment," "variant," "wild-type," "wild-type protein" or "wild-type biologic protein," "parent molecule" or "target protein," "working," "conditionally active antibody," "antibody-dependent cell-mediated cytotoxicity" or "ADCC," "cancer" and "cancerous," "multispecific antibody," "full length antibody," "library," "recombinant antibody," and "individual" or "subject" are the same as in WO 2016/138071.

The term "antibody", as used herein, refers to intact immunoglobulin molecules, as well as fragments of immunoglobulin molecules, such as Fab, Fab', (Fab')₂, Fv, and SCA fragments, that are capable of binding to an epitope of an antigen. These antibody fragments, which retain some ability to selectively bind to an antigen (e.g., a polypeptide antigen) of the antibody from which they are derived, can be made using well known methods in the art (see, e.g., Harlow and Lane, supra), and are described further, as follows. Antibodies useful in the practice of the claimed invention may be IgG1, IgG2, IgG3, IgG4, IgM, IgA1, IgA2, sIgA, IgD or IgE. Antibodies can be used to isolate preparative quantities of the antigen by immunoaffinity chromatography. Various other uses of such antibodies are to diagnose and/or stage disease (e.g., neoplasia) and for therapeutic application to treat disease, such as for example: neoplasia, autoimmune disease, AIDS, cardiovascular disease, infections, and the like. Chimeric, human-like, humanized or fully human antibodies are particularly useful for administration to human patients.

An Fab fragment consists of a monovalent antigen-binding fragment of an antibody molecule, and can be produced by digestion of a whole antibody molecule with the enzyme papain, to yield a fragment consisting of an intact light chain and a portion of a heavy chain.

An Fab' fragment of an antibody molecule can be obtained by treating a whole antibody molecule with pepsin, followed by reduction, to yield a molecule consisting of an intact light chain and a portion of a heavy chain. Two Fab' fragments are obtained per antibody molecule treated in this manner.

An (Fab')2 fragment of an antibody can be obtained by treating a whole antibody molecule with the enzyme pepsin, without subsequent reduction. A (Fab')₂ fragment is a dimer of two Fab' fragments, held together by two disulfide bonds.

An Fv fragment is defined as a genetically engineered fragment containing the variable region of a light chain and the variable region of a heavy chain expressed as two chains.

A single chain antibody ("SCA" or scFv) is a genetically engineered single chain molecule containing the variable region of a light chain and the variable region of a heavy chain, linked by a suitable, flexible polypeptide liner, and which may include additional amino acid sequences at the amino- and/or carboxyl- termini. For example, a single chain antibody may include a tether segment for linking to the encoding polynucleotide. A functional single chain antibody generally contains a sufficient portion of the variable region of a light chain and a sufficient region of the variable region of a heavy chain so as to retain the property of a full-length antibody for binding to a specific target molecule or epitope.

The term "antigen" or "Ag" as used herein is defined as a molecule that is capable of triggering an immune response. This immune response may involve either antibody production, or the activation of specific immunologically-competent cells, or both. A person skilled in the art will understand that any macromolecule, including virtually all proteins or peptides, can serve as an antigen. It is readily apparent that an antigen can be generated, synthesized or can be derived from a biological sample. Such a biological sample can include, but is not limited to a tissue sample, a tumor sample, a cell or a biological fluid.

The term "apoptosis", as used herein, refers to a mechanism of cell death affecting single cells, marked by shrinkage of the cell, condensation of chromatin, and fragmentation of the cell into membrane-bound bodies that are eliminated by phagocytosis. The term "apoptosis" is often used synonymously with the term "programmed cell death".

The term "apoptosis-inducing activity", as used herein, refers to the intrinsic property of a compound to selectively invoke apoptosis in a (i) particular cell type and/or (ii) cell in a particular stage of development or differentiation, due to internal or external stimuli. A skilled person is aware of the existence of *in vitro* standard assays for determining apoptosis-inducing activity of a compound in a cell culture, for example tests that assess levels of cytoplasmic Cytochrome C (marker for apoptosis) and levels of TUNEL (marker for apoptosis). Using these standard assays, the skilled person can easily assess and compare the apoptosis-inducing activity of different compounds with regard to different cell type or cells in a different developmental stage, e.g. senescent vs. non-senescent cells. Other standard apoptosis assays are an Annexin V assay and a cleaved caspase-3 staining.

The term "biosimilar" or "follow-on biologic" is used in a manner that is consistent with the working definition promulgated by the U.S. Food and Drug Administration (FDA), which defines a biosimilar to be a product that is "highly similar" to a reference product (despite minor differences in clinically inactive components). In practice, there can be no clinically meaningful differences between the reference product and the biosimilar product in terms of safety, purity, and potency (Public Health Service (PHS) Act §262). A biosimilar can also be one that satisfies one or more guidelines adopted May 30, 2012 by the Committee for Medicinal Products for Human Use (CHMP) of the European Medicines Agency and published by the European Union as "Guideline on similar biological medicinal products containing monoclonal antibodies- non-clinical and clinical issues" (Document Reference EMA/CHMP/BMWP/403543/2010). For example, a "biosimilar antibody" refers to a subsequent version of an innovator's antibody (reference antibody) typically made by a different company. Differences between a biosimilar antibody and a reference antibody can include post-translational modification, e.g. by attaching to the antibody other biochemical groups such as a phosphate, various lipids and carbohydrates; by proteolytic cleavage following translation; by changing the chemical nature of an amino acid (e.g., formylation); or by many other mechanisms. Other post-translational modifications can be a consequence of manufacturing process operations- for example, glycation may occur with exposure of the product to reducing sugars. In some cases, storage conditions may be permissive for certain degradation pathways such as oxidation, deamidation, or aggregation to occur. As all of these product-related variants may be included in a biosimilar antibody.

The terms "cancer" and "cancerous" refer to or describe the physiological condition in mammals that is typically characterized by unregulated cell growth/proliferation. A "tumor" comprises one or more cancerous cells. Examples of cancer include, but are not limited to, carcinoma, lymphoma, blastoma, sarcoma, and leukemia or lymphoid malignancies. More particular examples of such cancers include squamous cell cancer (e.g., epithelial squamous cell cancer), lung cancer including small cell lung cancer, non-small cell lung cancer ("NSCLC"), adenocarcinoma of the lung and squamous carcinoma of the lung, cancer of the peritoneum, hepatocellular cancer, gastric or stomach cancer including gastrointestinal cancer, pancreatic cancer, glioblastoma, cervical cancer, ovarian cancer, liver cancer, bladder cancer, hepatoma, breast cancer, colon cancer, rectal cancer, colorectal cancer, endometrial or uterine carcinoma, salivary gland carcinoma, kidney or renal cancer, prostate cancer, vulvar cancer, thyroid cancer, hepatic carcinoma, anal carcinoma, penile carcinoma, as well as head and neck cancer.

The term "conditionally active protein" refers to a variant, or mutant, of a parent protein which is more or less active under one or more aberrant conditions as compared to the same activity of a control or under a normal physiological condition. This conditionally active protein also exhibits activity in selected regions of the body and/or exhibits increased or decreased activity under aberrant, or permissive, physiological conditions. Normal physiological conditions are those which would be considered within a normal range at a location in a subject such as at the site of administration, or at the tissue or organ at the site of action, in a subject. An aberrant condition is that which deviates from the normally acceptable range for that condition at that location. In one aspect, the conditionally active protein is virtually inactive at a normal physiological condition but is active at the aberrant or permissive condition. For example, in one aspect, an evolved conditionally active protein is virtually inactive at body temperature, but is active at lower or higher temperatures. In another aspect, the conditionally active protein may be reversibly or irreversibly inactivated at the normal physiological or control condition. In a further aspect, the conditionally active protein is a therapeutic protein. In another aspect, the conditionally active protein is used as a drug, or therapeutic agent. In yet another aspect, the conditionally active protein is more or less active in highly oxygenated blood, such as, for example, after passage through the lung or in the lower pH environments found in the kidney. A conditionally active protein may be a conditionally active biologic protein.

As used herein, the term "cyclic peptide" refers to a polypeptide chain whose amino and carboxyl termini are themselves linked together with a peptide bond that forms a circular chain (i.e., between the alpha carboxyl of one residue and the alpha amine of another). For purposes of this application, cyclic peptides may also include a linkage other than a peptide bond such as non-alpha amide linkage, and a thioether linkage between Trp and Cys residues. The length of the cyclic peptide may be in the range of from about 5 to about 500 amino acids, or from about 8 to about 300 amino acids, or from about 8 to about 200 amino acids, or from about 10 to about 100 amino acids, or from about 10 to about 50 amino acids. Additionally, amino acids other than naturally-occurring amino acids, for example β-alanine, phenyl glycine and homoarginine, may be included in the cyclic peptides.

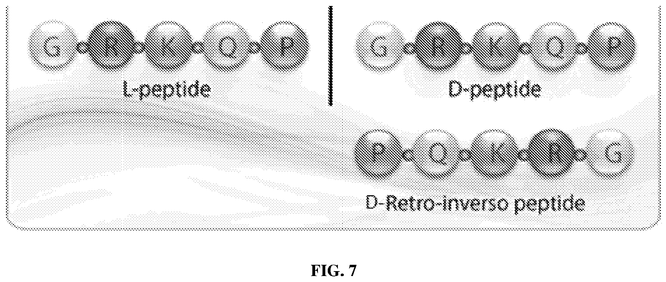

The abbreviation "DRI", as used herein, refers to the D retro inverso isoform of an L-peptide, in which the amino acid sequence is reversed in comparison with a fragment or the full-length of a natural or wild-type protein, and at least a portion of the amino acid residues in the DRI peptide are D amino acid residues instead of the L amino acid residues in the natural or wild-type protein (FIG. 7). The D retro inverso peptide can be made by identifying the amino acid sequence of a fragment or the full-length of a natural protein, reversing the sequence and synthesizing the D retro reverse peptide using known methods to provide a peptide having the reverse of the amino acid sequence of the fragment or the full-length of a natural protein and including in the D retro reverse peptide a sufficient number of D amino acids to provide the desired function.

The terms "diseases or conditions where the removal of senescent cells is beneficial", "diseases or conditions associated with the presence of senescent cells" and "disorders where the removal of senescent cells is beneficial" are used interchangeable, referring to any disease or condition in a mammalian, for example a human, subject where removal or clearance or reduced viability of senescent cells is beneficial to the subject suffering from said disease or condition. The term encompasses the situation where senescent cells are one, or the only, cause of a disease or contribute to the progression of a disease. The term further relates to the situation where senescent cells might become, in the future, the cause of a disease or condition in said subject. For example, the treatment of a disease or condition where the removal of senescent cells is beneficial, is a disease or condition prevented, preventable or ameliorated by removing senescent cells. For example, it is known that chemotherapeutic agents and radiation therapy induce cellular senescence. It is advantageous to remove these senescent cells in order to prevent the onset of diseases or conditions associated with cellular senescence. The term further encompasses diseases or conditions where removal of senescent cells alleviates or reduces symptoms of a disease or condition.

Removal of senescent cells is beneficial if *inter alia* the disease or condition can be healed, prevented or if the symptoms of the disease or condition or can be reduced or alleviated. Removal of senescent cells may be achieved by induction of apoptosis in the senescent cells. For example, the disease or condition where the removal of senescent cells is beneficial, is selected from the group formed by atherosclerosis, chronic inflammatory diseases such as arthritis or arthrosis, cancer, osteoarthritis, diabetes, diabetic ulcers, kyphosis, sclerosis, hepatic insufficiency, cirrhosis, Hutchinson-Gilford progeria syndrome (HGPS), laminopathies, osteoporosis, dementia, (cardio)vascular diseases, obesity, metabolic syndrome, acute myocardial infarction, emphysema, insulin sensitivity, boutonneuse fever, sarcopenia, neurodegenerative diseases such as Alzheimer's , Huntington's or Parkinson's disease, cataracts, anemia, hypertension, fibrosis, age-related macular degeneration, COPD, asthma, renal insufficiency, incontinence, hearing loss such as deafness, vision loss such as blindness, sleeping disturbances, pain such as joint pain or leg pain, imbalance, fear, depression, breathlessness, weight loss, hair loss, muscle loss, loss of bone density, frailty and/or reduced fitness. A disease or condition where the removal of senescent cells is beneficial is a disease or condition associated with or linked to inflammation, specifically chronic inflammation, in a mammalian, for example human, subject, where said inflammation is caused or mediated by senescent cells. In some embodiments, said senescent cells causing or mediating said inflammation are at least partially co-localized in the same organ, more preferably in the same tissue, as the organ or tissue, affected by said disease or condition.

The term "diseases or conditions associated with the presence of senescent cells", as used herein, refers to any disease or condition in a mammalian, for example human, subject where the presence of senescent cells, or presence of cellular senescence, in a mammalian, for example human, subject is linked to said disease or condition in said subject. In this context, "linked to" can *inter alia* refer to the senescent cells or cellular senescence (i) as the at least partial cause of a disease or condition, (ii) or as at least a partial cause of a symptom. In some embodiments, the disease or condition associated with the presence of senescent cells, is selected from the group formed by atherosclerosis, chronic inflammatory diseases such as arthritis or arthrosis, cancer, osteoarthritis, diabetes, diabetic ulcers, kyphosis, sclerosis, hepatic insufficiency, cirrhosis, Hutchinson-Gilford progeria syndrome (HGPS), laminopathies, osteoporosis, dementia, (cardio)vascular diseases, obesity, metabolic syndrome, acute myocardial infarction, emphysema, insulin sensitivity, boutonneuse fever, sarcopenia, neurodegenerative diseases such as Alzheimer's , Huntington's or Parkinson's disease, cataracts, anemia, hypertension, fibrosis, age-related macular degeneration, COPD, asthma, renal insufficiency, incontinence, hearing loss such as deafness, vision loss such as blindness, sleeping disturbances, pain such as joint pain or leg pain, imbalance, fear, depression, breathlessness, weight loss, hair loss, muscle loss, loss of bone density, frailty and/or reduced fitness. A specific disease or condition where the removal of senescent cells is beneficial is a disease or condition associated with or linked to inflammation, typically chronic inflammation, for example in a mammalian, such as human, subject, where said inflammation is caused or mediated by senescent cells. In some embodiments, said senescent cells causing or mediating said inflammation is at least partially co-localized in the same organ, for example in the same tissue, as the organ or tissue, affected by said disease or condition.

The term "extracellular condition of a senescent cell" as used herein refers to a condition in the extracellular environment immediately surrounding one or more senescent cells and which differs from the same condition surrounding non-senescent cells. The extracellular environment of a senescent cell can include, for example, any extracellular matrix or fluid adjacent to the senescent cell.

The terms "FOXO4 peptide" and "FOXO4 protein" as used herein refer to a protein translated from a transcript of the forkhead box protein O4 (FOXO4) gene. The FOXO4 has two variants (SEQ ID NO:1 and SEQ ID NO:2). The term "FOXO4 DRI peptide" refers to a D retro inverso peptide that has the reverse amino acid sequence of at least a fragment of the FOXO4 protein and contains some, for example all D amino acid residues.

The term "full length antibody" refers to an antibody which comprises an antigen-binding variable region (V_{H} or V_{L}) as well as a light chain constant domain (CL) and heavy chain constant domains, CH1, CH2 and CH3. The constant domains may be native sequence constant domains (e.g. human native sequence constant domains) or amino acid sequence variants thereof. Depending on the amino acid sequence of the constant domain of their heavy chains, full length antibodies can be assigned to different "classes". There are five major classes of full length antibodies: IgA, IgD, IgE, IgG, and IgM, and several of these may be further divided into "subclasses" (isotypes), e.g., IgG1, IgG2, IgG3, IgG4, IgA, and IgA2. The heavy-chain constant domains that correspond to the different classes of antibodies are called alpha, delta, epsilon, gamma, and mu, respectively.

An "individual" or "subject" is a mammal. Mammals include, but are not limited to, domesticated animals (e.g., cows, sheep, cats, dogs, and horses), primates (e.g., humans and non-human primates such as monkeys), rabbits, and rodents (e.g., mice and rats).

The term "library" as used herein refers to a collection of proteins in a single pool. The library may be generated using DNA recombinant technology. For example, a collection of cDNAs or any other protein coding DNAs may be inserted in an expression vector to generate a protein library. A collection of cDNAs or protein coding DNAs may also be inserted into a phage genome to generate a bacteriophage display library of wild-type proteins. The collection of cDNAs may be produced from a selected cell population or a tissue sample, such as by the methods disclosed by Sambrook et al. (Molecular Cloning, Cold Spring Harbor Laboratory Press, 1989). cDNA collections from selected cell types are also commercially available from vendors such as Stratagene^{®}. The library of wild-type proteins as used herein is not a collection of biological samples.

The term "ligand" as used herein refers to a molecule that is recognized by a particular receptor and specifically binds the receptor in one or more binding sites. Examples of ligands include, but not restricted to, agonists and antagonists for cell membrane receptors, toxins and venoms, viral epitopes, hormones, hormone receptors peptides, enzymes, enzyme substrates, co factors, drugs (e.g. opiates, steroids, etc.), lectins, sugars, polynucleotides, nucleic acids, oligosaccharides, proteins, and monoclonal antibodies. Typically, a ligand comprises two structural portions: a first portion that is involved in binding of the ligand to its receptor and a second portion that is not involved in such binding.

The term "multispecific antibody" as used herein is an antibody having binding specificities for at least two different epitopes. Exemplary multispecific antibodies may bind both a BBB-R and a brain antigen. Multispecific antibodies can be prepared as full-length antibodies or antibody fragments (e.g. F(ab')₂ bispecific antibodies). Engineered antibodies with two, three or more (e.g. four) functional antigen binding sites are also contemplated (see, e.g., US 2002/0004587 A1).

The term "non-naturally occurring amino acid" as used herein refers to any amino acid that is not found in nature. Non-natural amino acids include any D-amino acids, amino acids with side chains that are not found in nature, and peptidomimetics. Examples of peptidomimetics include, but are not limited to, b-peptides, g-peptides, and d-peptides; oligomers having backbones which can adopt helical or sheet conformations, such as compounds having backbones utilizing bipyridine segments, compounds having backbones utilizing solvophobic interactions, compounds having backbones utilizing side chain interactions, compounds having backbones utilizing hydrogen bonding interactions, and compounds having backbones utilizing metal coordination. Non-naturally occurring amino acids also include residues that have side chains that resist non-specific protein adsorption, which may be designed to enhance the presentation of the antimicrobial peptide in biological fluids, and/or polymerizable side chains, which enable the synthesis of polymer brushes using the non-natural amino acid residues within the peptides as monomeric units.

The term "parent protein" as used herein refers to a polypeptide or protein that may be evolved to produce a conditionally active polypeptide or protein using the methods of the present invention. The parent protein may be a wild-type protein or a non-naturally occurring protein. For example, a therapeutic polypeptide or protein or a mutant or variant polypeptide or protein may be used as a parent polypeptide or protein. Parent protein may also be a fragment of another naturally occurring protein, wild-type protein, therapeutic protein or mutant protein. Examples of parent proteins include antibodies, antibody fragments, enzymes, enzyme fragments cytokines and fragments thereof, hormones and fragments thereof, ligands and fragments thereof, receptors and fragments thereof, regulatory proteins and fragments thereof, and growth factors and fragments thereof.

The term "polypeptide" as used herein refers to a polymer in which the monomers are amino acids and are joined together through peptide or disulfide bonds. A polypeptide may be a full-length naturally-occurring amino acid chain or a fragment, mutant or variant thereof, such as a selected region of the amino acid chain that is of interest in a binding interaction. A polypeptide may also be a synthetic amino acid chain, or a combination of a naturally-occurring amino acid chain or fragment thereof and a synthetic amino acid chain. A fragment refers to an amino acid sequence that is a portion of a full-length protein, and will be typically between about 8 and about 500 amino acids in length, preferably about 8 to about 300 amino acids, more preferably about 8 to about 200 amino acids, and even more preferably about 10 to about 50 or 100 amino acids in length. Additionally, amino acids other than naturally-occurring amino acids, for example β-alanine, phenyl glycine and homoarginine, may be included in the polypeptides. Commonly-encountered amino acids which are not gene-encoded may also be included in the polypeptides. The amino acids may be either the D- or L-optical isomer. The D-isomers are preferred for use in a specific context, further described below. In addition, other peptidomimetics are also useful, e.g. in linker sequences of polypeptides (see Spatola, 1983, in Chemistry and Biochemistry of Amino Acids. Peptides and Proteins, Weinstein, ed., Marcel Dekker, New York, p. 267). In general, the term "protein" is not intended to convey any significant difference from the term "polypeptide" other than to include structures which comprise two or several polypeptide chains held together by covalent or non-covalent bonds.

The term "protein" as used herein refers to a polymer in which the monomers are amino acids and are joined together through peptide or disulfide bonds. A protein may be a full-length naturally-occurring amino acid chain or a fragment, mutant or variant thereof, such as a selected region of the amino acid chain that is of interest in a binding interaction. A protein may be a cyclic peptide with the amino acid polymer forming a cyclic structure using the entire or part of the polymer. A protein may also be a synthetic amino acid chain, an amino acid chain containing a non-natural amino acid or a combination of a naturally-occurring amino acid chain or fragment thereof and a synthetic amino acid chain. A fragment refers to an amino acid sequence that is a portion of a full-length protein, and will be typically between about 8 and about 500 amino acids in length, preferably about 8 to about 300 amino acids, more preferably about 8 to about 200 amino acids, and even more preferably about 10 to about 50 or 100 amino acids in length. Additionally, amino acids other than naturally-occurring amino acids, for example β-alanine, phenyl glycine and homoarginine, may be included in the polypeptides. Commonly-encountered amino acids which are not gene-encoded may also be included in the polypeptides. The amino acids may be either the D- or L- optical isomer. The D-isomers are preferred for use in a specific context, further described below. In addition, other peptidomimetics are also useful, e.g. in linker sequences of polypeptides (see Spatola, 1983, in Chemistry and Biochemistry of Amino Acids. Peptides and Proteins, Weinstein, ed., Marcel Dekker, New York, p. 267). In general, the term "protein" is not intended to convey any significant difference from the term "polypeptide" other than to include structures which comprise two or several polypeptide chains held together by covalent or non-covalent bonds.

The term "receptor" as used herein refers to a molecule that has an affinity for a given ligand. Receptors can be naturally occurring or synthetic molecules. Receptors can be employed in an unaltered state or as aggregates with other species. Receptors can be attached, covalently or non-covalently, to a binding member, either directly or via a specific binding substance. Examples of receptors include, but are not limited to, antibodies, including monoclonal antibodies and antisera reactive with specific antigenic determinants (such as on viruses, cells, or other materials), cell membrane receptors, complex carbohydrates and glycoproteins, enzymes, and hormone receptors. The binding of a ligand to its receptor indicates a combination of the ligand and its receptor molecule through specific molecular recognition to form a complex, which can be detected by a variety of ligand receptor binding assays known to a skilled person.

The term "senescence" or "cellular senescence" as used herein means the progression from an actively dividing cell to a metabolically active, non-dividing cell. The term "senescence" also refers to the state cells enter after multiple rounds of division and in which state future cell division is prevented from occurring even though the cell remains metabolically active.

The term "senescent cell" as used herein means a cell that is metabolically active but permanently withdrawn from the cell cycle (see, e.g., Campisi, Cell, vol. 120, pp.513-522, 2005). Senescent cells do not replicate and possess one or more of the following additional characteristics attributed to senescent cells: cell cycle arrest in the G1 phase; an enlarged, flattened morphology; increased granularity; staining for β-galactosidase activity at pH 6; senescence associated heterochromatic foci; and characteristic gene expression that is in part regulated by p16 and p21. Examples of senescent cells include senescent preadipocytes, senescent endothelial cells, senescent fibroblasts, senescent neurons, senescent epithelial cells, senescent mesenchymal cells, senescent smooth muscle cells, senescent macrophages, and senescent chondrocytes.

The term "senolytic agent" as used herein refers to an agent that selectively (preferentially or to a greater degree) destroys, kills, removes, or facilitates selective destruction of senescent cells. In other words, the senolytic agent destroys or kills a senescent cell in a biologically, clinically, and/or statistically significant manner compared with its capability to destroy or kill a non-senescent cell. The senolytic agent may be a small compound or a biological molecule such as proteins, polynucleotides. A senolytic agent is used in an amount and for a time sufficient that selectively kills established senescent cells but is insufficient to kill (destroy, cause the death of) non-senescent cells in a clinically significant or biologically significant number. In certain embodiments, the senolytic agents described herein alter at least one signaling pathway in a manner that induces (initiates, stimulates, triggers, activates, promotes) and results in (i.e., causes, leads to) death of the senescent cell. The senolytic agent may alter, for example, either or both of a cell survival signaling pathway (e.g., Akt pathway) or an inflammatory pathway, for example, by antagonizing a protein within the cell survival and/or inflammatory pathway in a senescent cell.

The term "small molecule" as used herein refers to molecules or ions that have a molecular weight of less than 900 a.m.u., or more preferably less than 500 a.m.u. or more preferably less than 200 a.m.u. or even more preferably less than 100 a.m.u. In the assays and environments of the present invention, small molecules may often be present as a mixture of the molecule and a deprotonated ion of the molecule, depending primarily on the pH of the assay or environment.

The term "target associated with a senescent cell" as used herein means a molecule, for example a protein, that is located on the surface of a senescent cell (e.g., a cellular membrane protein), or present in the senescent cell, or secreted by the senescent cell into the extracellular environment of the senescent cell.

The term "therapeutic protein" as used herein refers to any protein and/or polypeptide that can be administered to a mammal to elicit a biological or medical response of a tissue, system, animal or human that is being sought, for instance, by a researcher or clinician. A therapeutic protein may elicit more than one biological or medical response. Examples of therapeutic proteins include antibodies, enzymes, hormones, cytokines, regulatory proteins, and fragments thereof.

The term "therapeutically effective amount" as used herein means any amount which, as compared to a corresponding subject who has not received such amount, results in, but is not limited to, healing, prevention, or amelioration of a disease, disorder, or side effect, or a decrease in the rate of advancement of a disease or disorder. The term also includes within its scope amounts effective to enhance normal physiological function as well as amounts effective to cause a physiological function in a patient which enhances or aids in the therapeutic effect of a second pharmaceutical agent.

The terms "treat" and "treatment" as used herein refer to medical management of a disease, disorder, or condition of a subject (i.e., patient) (see, e.g., Stedman's Medical Dictionary). In general, an appropriate dose and treatment regimen provide the senolytic agent in an amount sufficient to provide therapeutic and/or prophylactic benefit. Therapeutic benefit for subjects to whom the senolytic agents described herein are administered, includes, for example, an improved clinical outcome, wherein the object is to prevent or slow or retard (lessen) an undesired physiological change associated with the disease, or to prevent or slow or retard (lessen) the expansion or severity of such disease.

The term "tumor microenvironment" as used herein refers to a microenvironment in and surrounding a solid tumor to support the growth and metastasis of the tumor cells. The tumor microenvironment includes surrounding blood vessels, immune cells, fibroblasts, other cells, soluble factors, signaling molecules, an extracellular matrix, and mechanical cues that can promote neoplastic transformation, support tumor growth and invasion, protect the tumor from host immunity, foster therapeutic resistance, and provide niches for dormant metastases to thrive. The tumor and its surrounding microenvironment are closely related and interact constantly. Tumors can influence their microenvironment by releasing extracellular signals, promoting tumor angiogenesis and inducing peripheral immune tolerance, while the immune cells in the microenvironment can affect the growth and evolution of cancerous cells. See Swarts et al. "Tumor Microenvironment Complexity: Emerging Roles in Cancer Therapy," Cancer Res, vol., 72, pages 2473-2480, 2012; Weber et al., "The tumor microenvironment," Surgical Oncology, vol. 21, pages 172-177, 2012; Blagosklonny, "Antiangiogenic therapy and tumor progression," Cancer Cell, vol. 5, pages 13-17, 2004; Siemann, "Tumor microenvironment," Wiley, 2010; and Bagley, "The tumor microenvironment," Springer, 2010.

### DETAILED DESCRIPTION

It must be noted that as used herein and in the appended claims, the singular forms "a", "an", and "the" include plural references unless the context clearly dictates otherwise. Furthermore, the terms "a" (or "an"), "one or more," and "at least one" can be used interchangeably herein. The terms "comprising," "including," "having," and "constructed from" can also be used interchangeably.

Unless otherwise indicated, all numbers expressing quantities of ingredients, properties such as molecular weight, percent, ratio, reaction conditions, and so forth used in the specification and claims are to be understood as being modified in all instances by the term "about," whether or not the term "about" is present. Accordingly, unless indicated to the contrary, the numerical parameters set forth in the specification and claims are approximations that may vary depending upon the desired properties sought to be obtained by the present disclosure. At the very least, and not as an attempt to limit the application of the doctrine of equivalents to the scope of the claims, each numerical parameter should at least be construed in light of the number of reported significant digits and by applying ordinary rounding techniques. Notwithstanding that the numerical ranges and parameters setting forth the broad scope of the disclosure are approximations, the numerical values set forth in the specific examples are reported as precisely as possible. Any numerical value, however, inherently contains certain errors necessarily resulting from the standard deviation found in their respective testing measurements.

It is to be understood that each component, compound, substituent or parameter disclosed herein is to be interpreted as being disclosed for use alone or in combination with one or more of each and every other component, compound, substituent or parameter disclosed herein.

It is also to be understood that each amount/value or range of amounts/values for each component, compound, substituent or parameter disclosed herein is to be interpreted as also being disclosed in combination with each amount/value or range of amounts/values disclosed for any other component(s), compounds(s), substituent(s) or parameter(s) disclosed herein and that any combination of amounts/values or ranges of amounts/values for two or more component(s), compounds(s), substituent(s) or parameters disclosed herein are thus also disclosed in combination with each other for the purposes of this description.

It is further understood that each range disclosed herein is to be interpreted as a disclosure of each specific value within the disclosed range that has the same number of significant digits. Thus, a range of from 1-4 is to be interpreted as an express disclosure of the values 1, 2, 3 and 4. It is further understood that each lower limit of each range disclosed herein is to be interpreted as disclosed in combination with each upper limit of each range and each specific value within each range disclosed herein for the same component, compounds, substituent or parameter. Thus, this disclosure to be interpreted as a disclosure of all ranges derived by combining each lower limit of each range with each upper limit of each range or with each specific value within each range, or by combining each upper limit of each range with each specific value within each range.

Furthermore, specific amounts/values of a component, compound, substituent or parameter disclosed in the description or an example is to be interpreted as a disclosure of either a lower or an upper limit of a range and thus can be combined with any other lower or upper limit of a range or specific amount/value for the same component, compound, substituent or parameter disclosed elsewhere in the application to form a range for that component, compound, substituent or parameter.

The present invention provides a method for producing a conditionally active antibody or antibody fragment having activity on senescent cells from a parent antibody or antibody fragment that binds to a target associated with a senescent cell according to the appended claims. The method comprises the steps of
(i) evolving a DNA encoding the parent antibody or antibody fragment using one or more evolutionary techniques to create mutant DNAs;
(ii) expressing the mutant DNAs to obtain mutant antibodies or antibody fragments;
(iii) subjecting the mutant antibodies or antibody fragments to an assay under an extracellular pH of a senescent cell and an assay under a normal physiologic pH; and
(iv) selecting the conditionally active senolytic antibody or antibody fragment from the mutant antibodies or antibody fragments that exhibits at least one of:
   (a) a decrease in an activity in the assay under the normal physiological pH compared to the same activity of the parent antibody or antibody fragment in the same assay, and an increase in the activity in the assay under the extracellular pH of the senescent cell compared to the same activity of the conditionally active antibody or antibody fragment in the assay under the normal physiological condition; and
   (b) a decrease in the activity in the assay under the normal physiological pH compared to the same activity of the parent antibody or antibody fragment in the same assay, and an increase in the activity in the assay under the extracellular pH of the senescent cell compared to the same activity of the parent antibody or antibody fragment in the assay under the extracellular pH of the senescent cell.

The parent antibody or antibody fragment binds to the target associated with the senescent cell and the activity is the binding activity to the target..In some embodiments, the parent antibody or antibody fragment may be a therapeutic antibody or antibody fragment or a biosimilar.

The target associated with the senescent cell is typically a protein of a senescent cell. The target is in some examples a protein on the cellular membrane of the senescent cell. In some embodiments, the target is selected from DEP-1, NTAL, EBP50, STX4, VAMP3, ARMCX-3, LANCL1, B2MG, PLD3, and VPS26A. These proteins are recognized as biomarkers of senescent cells as described in WO 2015/181526. In some embodiments, the target is selected from ITGAV, RAC1, ARHGAP1, RAPGEF1, CRKL, NCKAP1, CDC42, CAPNS2, EBP, FGF1, ISG20, KITLG, LPHN1, MAG, MEF2C, OSBPL3, PFN1, POU5F1, PPP1CB, pl6INK4a, PRKRA, APC, AXL, BCL2L1, CDKN2C, CLYBL, COPG1, DGKA, GBA3, GIT2, IGF1, LCMT2, MADCAM1, MAP3K14, MTHFD2, NAIP, NAPG, NNMT, PARK2, PMS2, PRPF19, PRTG, RAPGEF1, RET, VIT, WEE1, YAP1, and YWHAE.

In some embodiments, the target is an Fas protein or a death receptor (DR). Fas is also sometimes referred to as a tumor necrosis factor receptor superfamily member 6A (TNFSF6). This is a membrane receptor that is easily accessible from outside of senescent cells. DRs are TNF-related apoptosis-inducing ligands (TRAILs), see Guicciardi et al., "Life and death by death receptors," FASEB J. vol. 23, pp. 1625-1637, 2009. Examples of DRs include DR4 and DR5.

In some embodiments, the target associated with the senescent cell is selected from MDM2, AKT (AKT1, AKT2, and AKT3), NOTCH3, DcR2 (TNFRSF10D), and a protein of the BCL-2 anti-apoptotic protein family. The proteins in this family have BH1-BH4 domains (BCL-2 (i.e., the BCL-2 protein member of the BCL-2 anti-apoptotic protein family), BCL-xL, BCL-w, Al, MCL-1, and BCL-B); or BH1, BH2, and BH3 domains (BAX, BAK, and BOK); or a BH3 domain only (BIK, BAD, BID, BIM, BMF, HRK, NOXA, and PUMA) (see, e.g., Cory et al., Nature Reviews Cancer, vol. 2, pp. 647-56, 2002; Cory et al., Cancer Cell, vol. 8, pp. 5-6, 2005; Adams et al, Oncogene, vol. 26, pp. 1324-1337, 2007). More targets associated with senescent cells suitable for use in the present invention are described in Althubiti et al, Cell Death and Disease, vol. 5, p. el528, 2014.

In some embodiments, the target associated with the senescent cell is selected from a misfolded form of a protein selected from prion protein (PrP), CD38, Notch-1, CD44, CD59, Fas ligand, TNF receptor, and EGF receptor as described in US 2016/0115237. The target may also be p16INK4a, or a protein selected from Tables 1-3 of US 2016/0038576.

Once the target associated with the senescent cell is selected, the parent antibody or antibody fragment that binds to the target is selected

The parent antibody or antibody fragment may be selected from a library, as described in WO 2016/138071. In some embodiments, the parent antibody or antibody fragment is selected from the library for example by use of an assay under a condition with a pH below 7.0, for example, in a pH range of from 5.0 to below 7.0, or from 5.5 to below 7.0, or from 6.0 to below 7.0, or from 6.2 to 6.8.

In some other embodiments, the parent antibody or antibody fragment is selected from the library for example using a screening solution that does not contain a small molecule having a pKa between 6 and 7.5, preferably between 6 and 7, and more preferably between 6.2 and 6.8. Examples of such small molecules are described in this application.

In some embodiments, the parent antibody or antibody fragment has one or more favorable characteristics based upon which it is chosen for use as the parent antibody. For example, in certain embodiments, the parent antibody may be selected based on having a good binding activity at one or more extracellular conditions of a senescent cell such as at a pH in the range of 5.0 to less than 7.0.

In some embodiments, the parent antibody is selected for its binding activity to a specific epitope. Selection based on binding activity to a specific epitope may be combined with one or more other selection criteria such as selection for good binding activity at one or more extracellular conditions of a senescent cell.

In other embodiments, the parent antibody is selected based on internalization efficiency. Selection based on internalization efficiency may be combined with one or more other selection criteria such as binding activity to a specific epitope or good binding activity at one or more extracellular conditions of a senescent cell.

In other embodiments, the parent antibody may have similar binding activity and/or characteristics under both the normal physiological pH and an extracellular pH of a senescent cell. In such embodiments, the parent antibody is selected based on having the most similar binding activity and/or the most similar combination of one or more characteristics under both the normal physiological pH and the extracellular pH of the senescent cell. For example, if the normal physiological pH and the extracellular pH of the senescent cell may be pH 7.4 and pH 6.4 respectively, the antibody that has the most similar binding activity at pH 7.4 and 6.4, may be selected as the parent antibody over an antibody having a less similar binding activity at pH 7.4 and 6.4.

In some embodiments, the parent antibody or antibody fragment may be a fragment of a naturally occurring antibody or antibody fragment. For Example, the parent antibody or antibody fragment may be the variable region of an antibody.

After the parent antibody or antibody fragment is selected, a DNA encoding the parent antibody or antibody fragment is evolved using a suitable evolutionary technique to produce mutant DNAs, which may then be expressed to produce mutant antibodies or antibody fragments for screening to identify a conditionally active senolytic antibody or antibody fragment. Suitable techniques for evolving the DNA encoding the parent antibody or antibody fragment, expressing the mutant DNAs to produce mutant antibodies or antibody fragments, and screening the mutant antibodies or antibody fragments are described in WO 2016/138071.

Once selected, the conditionally active senolytic antibody or antibody fragment may be optionally synthesized in "mimetic" and "peptidomimetic" forms, as described in WO 2016/138071.

The selected conditionally active senolytic antibody or antibody fragment may also be produced using a polypeptide expression cell production host or an organism. To make the production process more efficient, the DNA encoding the conditionally active senolytic antibody or antibody fragment may be subjected to codon optimization for the cell production host or organism. Codon optimization has been described previously, such as in, Narum et al., "Codon optimization of gene fragments encoding Plasmodium falciparum merzoite proteins enhances DNA vaccine protein expression and immunogenicity in mice," Infect. Immun., vol. 69, pp, 7250-3, 2001, which describes codon-optimization in the mouse system; Outchkourov et al., "Optimization of the expression of Equistatin in Pichia pastoris, protein expression and purification," Protein Expr. Purif., vol. 24, pp. 18-24, 2002, which describes codon-optimization in the yeast system; Feng et al., "High level expression and mutagenesis of recombinant human phosphatidylcholine transfer protein using a synthetic gene: evidence for a C-terminal membrane binding domain" Biochemistry, vol. 39, pp. 15399-409, 2000, which describes codon-optimization in E. coli; Humphreys et al., "High-level periplasmic expression in Escherichia coli using a eukaryotic signal peptide: importance of codon usage at the 5' end of the coding sequence", Protein Expr. Purif., vol. 20, pp. 252-64, 2000, which describes how codon usage affects protein secretion in *E. coli.*

The cell production host may be a mammalian cell production host selected from one of the group consisting of CHO, HEK293, IM9, DS-I, THP-I, Hep G2, COS, NIH 3T3, C33a, A549, A375, SK-MEL-28, DU 145, PC-3, HCT 116, Mia PACA-2, ACHN, Jurkat, MML-1, Ovcar 3, HT 1080, Panc-1, U266, 769P, BT-474, Caco-2, HCC 1954, MDA-MB-468, LnCAP, NRK-49F, and SP2/0 cell lines; and mouse splenocytes and rabbit PBMC. The mammalian cell production host is for example selected from a CHO or HEK293 cell line. In one specific aspect, the mammalian cell production host is a CHO-S cell line. In another embodiment, the mammalian cell production host is a HEK293 cell line.

In some embodiments, the cell production host is a yeast cell, for example *S*. *cerevisiae* yeast cells or pichia yeast cells. In some embodiments, the cell production host is a prokaryotic cell such as *E*. *coli* (Owens, R.J. and Young, R.J., J. Immunol. Meth., vol. 168, p.149, 1994; Johnson S and Bird RE, Methods Enzymol., vol. 203, p.88, 1991). The conditionally active senolytic antibody or antibody fragment may also be produced in plant cells or plants (Firek et al., Plant Mol. Biol., vol. 23, p.861, 1993).

The conditionally active senolytic antibody or antibody fragment may be modified through a natural process or using a chemical modification technique, as described in WO 2016/138071. The conditionally active senolytic antibody or antibody fragment may be synthesized using a solid-phase chemical peptide synthesis method, also as described in WO 2016/138071.

The conditionally active senolytic antibody or antibody fragment is selected using assays under an extracellular pH of a senescent cell and assays under a normal physiological condition. The selected conditionally active senolytic antibody or antibody fragment exhibits at least one of:
(a) a decrease in an activity in the assay under the normal physiological pH compared to the same activity of the parent antibody or antibody fragment in the same assay, and an increase in the activity in the assay under the extracellular pH of 5.5-7.0 of the senescent cell compared to the same activity of the conditionally active senolytic antibody or antibody fragment in the assay under the normal physiological pH of 7.2-7.8; and
(b) a decrease in the activity in the assay under the normal physiological pH of 7.2-7.8 compared to the same activity of the parent antibody or antibody fragment in the same assay and an increase in the activity in the assay under the extracellular pH of 5.5-7.0 of the senescent cell compared to the same activity of the parent antibody or antibody fragment in the assay under the extracellular pH of 5.5-7.0 of the senescent cell.

The pH has a different value in the assay under the extracellular pH of the senescent cell as compared to the assay at the normal physiologicalpH, e.g. the value of the pH at the normal physiological condition may be a pH of 7.2-7.8, or 7.2-7.6 and the value of the pH at the extracellular condition of the senescent cell may be pH 6.0-7.0, or 6.2-6.8.

The activity is binding activity of the conditionally active antibody or antibody fragment to the target, or a specific epitope.

The extracellular pHof a senescent cell is selected from one or more of the differences caused in the extracellular environment immediately adjacent to the senescent cell that are the result of special characteristic(s) of senescent cells, as compared to, for example the characteristics of normal cells. One group of special characteristics of senescent cells that is useful in the present invention is the metabolic activities of senescent cells. For example, senescent cells may exhibit one or more of the following special characteristics: (1) growth arrest of senescent cells is essentially permanent and cannot be reversed by known physiological stimuli; (2) senescent cells increase in size, sometimes enlarging more than twofold relative to the size of their non-senescent counterparts; (3) senescent cells express a senescence-associated β-galactosidase (SAP-gal), which partly reflects the increase in lysosomal mass; (4) many senescent cells express pl6INK4a, which is not commonly expressed by quiescent or terminally differentiated cells. (5) some senescent cells with persistent DNA damaging response (DDR) signaling harbor persistent nuclear foci, termed DNA segments, with chromatin alterations reinforcing senescence (DNA-SCARS such as dysfunctional telomeres or telomere dysfunction-induced foci (TIF)) and contain activated DDR proteins and are distinguishable from transient damage foci; (6) senescent cells express and may secrete molecules associated with senescence, which in certain instances may be observed in the presence of persistent DDR signaling; and (7) the nuclei of senescent cells lose structural proteins such as Lamin B 1 or chromatin-associated proteins such as histones and HMGB1. See, e.g., Freund et al, Mol. Biol. Cell, vol. 23, pp. 2066-75, 2012; Davalos et al, J. Cell Biol., vol. 201, pp. 613-29, 2013; Ivanov et al, J. Cell Biol., DOI:10.1083/jcb.201212110, pp. 1-15, 2013; Funayama et al, J. Cell Biol., vol. 175, pp. 869-80, 2006.

In some embodiments, the extracellular pH of the senescent cell is a low pH caused by increased glycolytic metabolism in the senescent cells (James et al., "Senescent human fibroblasts show increased glycolysis and redox homeostasis with extracellular metabolomes that overlap with those of irreparable DNA damage, aging, and disease," J Proteome Res., vol. 14, pp. 1854-71, 2015). Glycolysis involves breaking down glucose to form two pyruvates and two ATP, where the pyruvate may be converted to lactate and excreted, thus lowers the pH in the extracellular environment of the senescent cells (Wiley and Campisi, "From Ancient Pathways to Aging Cells-Connecting Metabolism and Cellular Senescence," Cell Metab., vol. 23, pp. 1013-21, 2016). This is similar to the tumor microenvironment where the glycolytic metabolism in cancer cells lowers the pH in tumor microenvironment. Thus, the extracellular pH of the senescent cells is may be an acidic pH in a range of from about 5.5 to about 7.2, or from about 6.0 to about 7.0, or from about 6.2 to about 7.0, or from about 6.2 to about 6.8, or from about 6.4 to about 6.8. The corresponding normal physiological condition is a normal physiological pH in a range of from about 7.2 to about 7.8, preferably from about 7.2 to about 7.6, or more preferably from about 7.4 to about 7.6.

Senescent cells secrete a variety of different proteins, which are collectively called senescence-associated secretory phenotype (SASP). These secreted proteins include, for example, GM-CSF, GROa, GRC-α,β,γ, IGFBP-7, IL-lα, IL-6, IL-7, IL-8, MCP-1, MCP-2, MIP-la, MMP-1, MMP-10, MMP-3, Amphiregulin, ENA-78, Eotaxin-3, GCP-2, GITR, HGF, ICAM-1, IGFBP-2, IGFBP-4, IGFBP-5, IGFBP-6, IL-13, IL-Iβ, MCP-4, MIF, MIP-3a, MMP-12, MMP-13, MMP-14, NAP2, oncostatin M, osteoprotegerin, PIGF, RANTES, sgpl30, TIMP-2, TRAIL-R3, Acrp30, angiogenin, Axl, bFGF, BLC, BTC, CTACK, EGF-R, Fas, FGF-7, G-CSF, GDNF, HCC-4, I-309, IFN-y, IGFBP-1, IGFBP-3, IL-1 Rl, IL-11, IL-15, IL-2R-a, IL-6 R, I-TAC, leptin, LIF, MMP-2, MSP-a, PAI-1, PAI-2, PDGF-BB, SCF, SDF-1, sTNF RI, sTNF RII, thrombopoietin, TIMP-1, tPA, uPA, uPAR, VEGF, MCP-3, IGF-1, TGF-β3 , MIP-1 -delta, IL-4, FGF-7, PDGF-BB, IL-16, BMP-4, MDC, MCP-4, IL-10, TIMP-1, Fit-3 Ligand, ICAM-1, Axl, CNTF, INF-γ, EGF, and BMP-6. Additional proteins secreted by senescent cells include IGF-2, and IGF-2R, IGFBP-3, IGFBP-7, TGF-β, WNT2, CXCR2-binding chemokines, WNT16B, SFRP2, SPINK1, ENPP5, EREG, ANGPTL4, CSGALNACT, CCL26, AREG, ANGPT1, CCK, THBD, CXCL14, NOV, GAL, NPPC, FAM150B, CST1, MUCL1, NPTX2, TMEM155, EDN1, PSG9, ADAMTS3, CD24, PPBP, CXCL3, CST2, PSG8, PCOLCE2, PSG7, TNFSF15, C17orf67, CALCA, FGF18, BMP-2, MATN3, TFP1, SERPINI 1, TNFRSF25, and IL-23A. The conditionally active antibody or antibody fragment of the present invention may be used as a senolytic agent to kill or remove senescent cells from a subject. The interaction between the conditionally active senolytic antibody or antibody fragment and the senescent cell may inhibit or even kill the senescent cell through inhibiting a cell survival signaling pathway and/or an inflammatory pathway that are activated during cellular senescence. Inhibition of the cell survival signaling pathway and/or inflammatory pathway can induce (i.e., initiate, trigger, stimulate or in some manner remove or inhibit suppression of) a cell death pathway, such as an apoptotic pathway, in the senescent cell that will lead to the death of the senescent cell.

Cell survival signaling pathways that are activated during senescence include the src kinase signaling pathway, a PI3K/Akt pathway, a PBK/Akt/mTor pathway, a p38/MAPK pathway, an ERK/MAPK pathway, a mTOR pathway, an insulin/IGF-1 signaling pathway, and a TGF-β signaling pathway. Inflammatory pathways that are activated during senescence include a p38/MAPK signaling pathway, an ERK/MAPK pathway, an src kinase signaling pathway, and an NF-kB pathway.

The src kinase signaling pathway is involved in regulation of cell proliferation, differentiation, apoptosis, cell adhesion, and stress responses (see, e.g., Wang, Oncogene, vol. 19, pp. 5643-50, 2000 and Thomas et al, Annu. Rev. Cell Dev. Biol., vol. 13, pp. 513-609, 1997). The src kinase signaling pathway is also involved in inflammatory responses, including macrophage mediated immune responses (see, e.g., Byeon et al, Mediators of Inflammation, vol. 2012, article ID 512926, 2012) and acute inflammatory responses (see, e.g., Okutani et al, Am. J. Physiol. Lung Cell Mol. Physiol., vol. 291, pp. L129-L141, 2006). Accordingly, a conditionally active senolytic antibody or antibody fragment that alters an src kinase signaling pathway may alter both a signaling pathway and an inflammatory pathway.

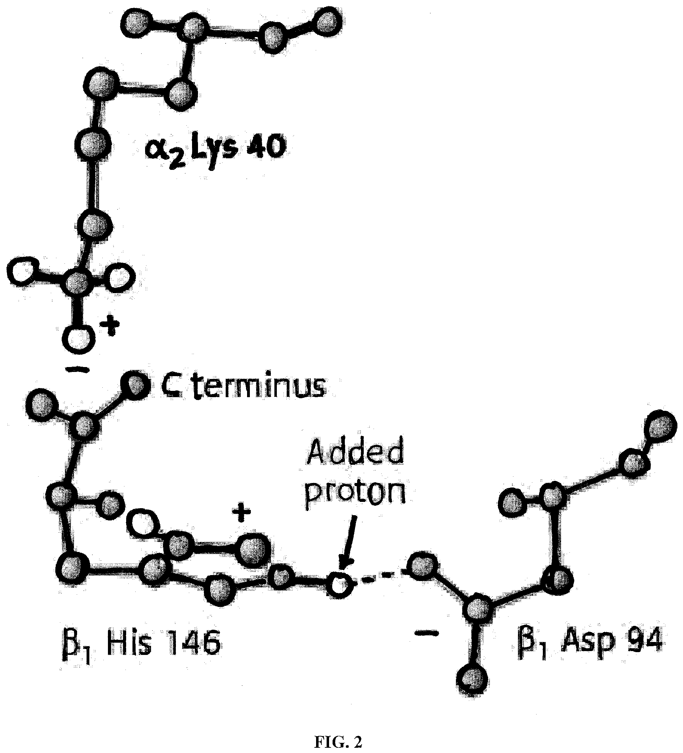

Altering a signaling pathway and/or an inflammatory pathway of a cell may affect a function of one or more downstream proteins or may affect the interaction of one or more downstream proteins with other components of the respective cell signaling or inflammatory pathway. For example, a conditionally active senolytic antibody or antibody fragment that alters a src kinase signaling pathway or a PBK/Akt pathway may alter a function of one or more downstream proteins in the respective pathway or may affect the interaction of the one or more downstream proteins with another component of the respective pathway (see, e.g., Example 1; Figures 2B-2D). Exemplary proteins that are upregulated in senescent cells include P38/MAPK, ERKl/2, and PBK (complex). In certain embodiments, the PBK/Akt pathway, which is a cell signaling pathway, is activated during senescence and a conditionally active senolytic antibody or antibody fragment described herein inhibits the PBK/Akt pathway to enhance or induce apoptosis in the senescent cells.

The assay solutions for the assay under the extracellular pH of senescent cell and the assay under the normal physiological pH for example include a component selected from citrate buffers such as sodium citrate, phosphate buffers, bicarbonate buffers such as the Krebs buffer, phosphate buffered saline (PBS) buffer, Hank's buffer, Tris buffer, HEPES buffer, etc. Other buffers known to a person skilled in the art to be suitable for the assays may be used.

The assay solutions may contain at least one component selected from inorganic compounds, ions and organic molecules, for example ones that are commonly found in a bodily fluid of a mammal such as a human or animal. These inorganic compounds, ions and organic molecules are described in detail in WO 2016/138071.

The conditionally active senolytic antibody or antibody fragment may interact with one or more of the inorganic compounds, ions, and organic molecules. Such interactions between the conditionally active senolytic antibody or antibody fragment and the component may be selected from inorganic compounds, ions and organic molecules include hydrogen bond bonding, hydrophobic interaction, and Van der Waals interactions.

In some embodiments, the extracellular pH of the senescent cell is a lower pH in the range of from 5.5 to 7.2, or from 6.0 to 7.0, or from 6.2 to 6.8, and the normal physiological pH is the normal physiological pH, for example in the range of from 7.2 to 7.8. The assay solutions for pH as the extracellular pH may include a component with a pKa between the lower pH of the extracellular pH and the normal physiological pH. The pKa is for example up to 0.5, 1, 1.5, 2, 2.5, or 3 units away from the lower pH of the extracellular condition. This component in some embodiments has a molecular weight of less than 900 a.m.u. and may for example be selected from histidine, histamine, hydrogenated adenosine diphosphate, hydrogenated adenosine triphosphate, citrate, bicarbonate, acetate, lactate, bisulfide, hydrogen sulfide, ammonium, dihydrogen phosphate and any combination thereof.

It has been observed that certain conditionally active antibodies or antibody fragments contain an increased number (or proportion) of charged amino acid residues in comparison to the amino acid residues of the parent antibody or antibody fragment from which the conditionally active antibodies or antibody fragments are derived. There are three positively charged amino acid residues: lysine, arginine and histidine; and two negatively charged amino acid residues: aspartate and glutamate. These charged amino acid residues are over-represented in certain conditionally active antibodies or antibody fragments in comparison with the parent antibody or antibody fragment from which the conditionally active antibodies or antibody fragments are derived. As a result, the conditionally active antibodies or antibody fragments are more likely to interact with charged species in the assay solution since the number of charged amino acid residues in the conditionally active antibodies or antibody fragments has increased. This, in turn, influences the activity of the conditionally active antibodies or antibody fragments.

It has also been observed that certain conditionally active antibodies or antibody fragments typically have different activities in the presence of different species in the assay solutions. Species that have at least two ionization states: an uncharged or less charged state at one value of a pH and a charged or more charged state at different value of the pH may alter the activity of the conditionally active antibody or antibody fragment. The charged or more charged state of the species may increase the interaction of the species with charged amino acid residues present in the conditionally active antibodies or antibody fragments. This mechanism may be employed to enhance the selectivity and/or pH-dependent activity of the conditionally active senolytic senolytic antibodies or antibody fragments.

The nature of the charge(s) on the conditionally active antibodies or antibody fragments may be one factor used to determine suitable species for influencing the activity of the conditionally active antibodies or antibody fragments. In some embodiments, the conditionally active antibodies or antibody fragments may have more positively charged amino acid residues: lysine, arginine and histidine, in comparison with the parent antibody or antibody fragment. The conditionally active antibodies or antibody fragments can thus be selected to have the desired level of interaction with a particular species present in the extracellular environment of senescent cells where the activity is desired and or to have the desired level of interaction with a particular species present in the normal physiological condition where a reduced activity is desired.

The location of the charged amino acid residues on the conditionally active antibodies or antibody fragments may also have an influence on the activity. For example, the proximity of charged amino acid residues to a binding site of the conditionally active antibodies or antibody fragments may be used to influence the activity of the conditionally active antibodies or antibody fragments.

In some embodiments, it may be the case that the interaction of the charged species with the conditionally active antibodies or antibody fragments may form salt bridges between different moieties on the antibody or antibody fragment, especially the moieties that are charged or polarized. The formation of salt bridges is known to stabilize polypeptide structures (Donald, et al., "Salt Bridges: Geometrically Specific, Designable Interactions," Proteins, 79(3): 898-915, 2011; Hendsch, et al., "Do salt bridges stabilize proteins? A continuum electrostatic analysis," Protein Science, 3:211-226, 1994). The salt bridges can stabilize or fix the protein structure which normally undergoes constant minor structural variation called "breathing" (Parak, "Proteins in action: the physics of structural fluctuations and conformational changes," Curr Opin Struct Biol., 13(5):552-557, 2003). The protein structural "breathing" is important for protein function and its binding with its partner because the structural fluctuation permits the conditionally active protein to efficiently recognize and bind to its partner (Karplus, et al., "Molecular dynamics and protein functions," PNAS, vol. 102, pp. 6679-6685, 2015). By forming salt bridges, the binding site, especially the binding pocket, on the conditionally active antibody or antibody fragment may be less accessible to its partner, possible because the salt bridges may directly block the partner from accessing the binding site. Even with salt bridges remote from the binding site, the allosteric effect may alter the conformation of the binding site to inhibit binding. Therefore, after the salt bridges stabilize (fix) the structure of the conditionally active antibody or antibody fragment, the antibody or antibody fragment may become less active in binding to its partner, leading to decreased activity.

One known example of protein and how its structure is stabilized by salt bridges is hemoglobin. Structural and chemical studies have revealed that at least two sets of chemical groups are responsible for the salt bridges: the amino termini and the side chains of histidines β146 and α122, which have pKa values near pH 7. In deoxyhemoglobin, the terminal carboxylate group of β146 forms a salt bridge with a lysine residue in the α subunit of the other αβ dimer. This interaction locks the side chain of histidine β146 in a position where it can participate in a salt bridge with negatively charged aspartate 94 in the same chain, provided that the imidazole group of the histidine residue is protonated (FIG. 2). At a high pH, the side chain of histidine β146 is not protonated and the salt bridges do not form. As the pH drops, however, the side chain of histidine β146 becomes protonated, the salt bridge between histidine β146 and aspartate β94 forms, which stabilizes the quaternary structure of deoxyhemoglobin, leading to a greater tendency for oxygen to be released at actively metabolizing tissues (with lower pH). The hemoglobin shows a pH-dependent binding activity for oxygen where at a low pH, the binding activity for oxygen is reduced because of the formation of salt bridges. On the other hand, at a high pH, the binding activity for oxygen is increased because of the absence of salt bridges.

Similarly, small molecules such as bicarbonate may reduce the binding activity of the conditionally active antibody or antibody fragment to its partner by forming salt bridges in the conditionally active antibody or antibody fragment. For example, at a pH lower than its pKa of 6.4, bicarbonate is protonated and thus not charged. The uncharged bicarbonate is not capable of forming salt bridges, thus has little effect on the binding of the conditionally active antibody or antibody fragment with its partner. Hence, the conditionally active antibody or antibody fragment has high binding activity with its partner at the low pH. On the other hand, at a high pH greater than the pKa of bicarbonate, bicarbonate is ionized by losing the proton, thus becoming negatively charged. The negatively charged bicarbonate will form salt bridges between positively charged moieties or polarized moieties on the conditionally active antibody or antibody fragment to stabilize the structure of the conditionally active antibody or antibody fragment. This will block or reduce the binding of the conditionally active antibody or antibody fragment with its partner. Hence the conditionally active antibody or antibody fragment has low activity at the high pH. The conditionally active antibody or antibody fragment thus has a pH-dependent activity at the presence of bicarbonate with higher binding activity at low pH than at high pH.

When a species such as bicarbonate is absent from the assay solutions, the conditionally active antibody or antibody fragment may lose its conditional activity. This is likely due to the lack of salt bridges on the conditionally active antibody or antibody fragment to stabilize (fix) the structure of the antibody or antibody fragment. Thus, the partner will have similar access to the binding site on the conditionally active antibody or antibody fragment at any pH, producing similar activity at the first pH and second pH.

It is to be understood that, though the salt bridges (ion bonds) are the strongest and most common manner for the species to affect the activity of the conditionally active antibodies or antibody fragments, other interactions between such species and the conditionally active antibodies or antibody fragments may also contribute to stabilize (fix) the structure of the conditionally active antibodies or antibody fragments. The other interactions include hydrogen bonds, hydrophobic interactions, and van der Waals interactions.

In some embodiments, to select a suitable compound or ion as the species, the conditionally active senolytic antibody or antibody fragment is compared with the parent antibody or antibody fragment from which it is evolved to determine whether the conditionally active senolytic antibody or antibody fragment has a higher proportion of negatively charged amino acid residues or positively charged amino acid residues. A compound with a suitable charge at the normal physiological pH may then be chosen to influence the activity of the conditionally active senolytic antibody or antibody fragment. For example, when the conditionally active senolytic antibody or antibody fragment has a higher proportion of positively charged amino acid residues than the parent antibody or antibody fragment, the suitable compound should typically be negatively charged at the normal physiological pH to interact with the conditionally active senolytic antibody or antibody fragment. On the other hand, when the conditionally active senolytic antibody or antibody fragment has a higher proportion of negatively charged amino acid residues than the parent antibody or antibody fragment, the suitable small molecule should typically be positively charged at the normal physiological pH to interact with the conditionally active senolytic antibody or antibody fragment.

Thus, a suitable species may be an inorganic or organic molecule that transits from an uncharged or less charged state at the lower pH of extracellular condition of senescent cells to charged or more charged state at the normal physiological pH. The species should typically have a pKa between the lower pH and normal physiological pH. For example, bicarbonate has pKa at 6.4. Thus, at a higher pH such as pH 7.4, the negatively charged bicarbonate will bind to the charged amino acid residues in the conditionally active senolytic antibodies or antibodies fragments and reduce the activity. On the other hand, at a lower pH such as pH 6.0-6.2, the less charged bicarbonate will not bind in the same quantity to the conditionally active senolytic antibodies or antibodies fragments and thus allow a higher activity of the conditionally active senolytic antibodies or antibodies fragments.

Bisulfide has a pKa 7.05. Thus, at a higher pH such as pH 7.4, the more negatively charged bisulfide will bind to the positively charged amino acid residues in the conditionally active senolytic antibodies or antibody fragments and reduce its activity. On the other hand, at a lower pH such as pH 6.0-6.8, the less charged hydrogen sulfide/bisulfide will not bind at the same level to the conditionally active senolytic antibodies or antibody fragments and thus allow a higher activity of the conditionally active senolytic antibodies or antibody fragments.

Some species are selected from bisulfide, hydrogen sulfide, histidine, histamine, citrate, bicarbonate, acetate, and lactate. Each of these small molecules has a pKa between 6.2 and 7.0. Other suitable small molecules may be found in textbooks using the principles of the present application, such as CRC Handbook of Chemistry and Physics, 96th Edition, by CRC press, 2015; Chemical Properties Handbook, McGraw-Hill Education, 1998.

The species for example have a low molecular weight and/or a relatively small conformation to ensure maximum access to small pockets on conditionally active senolytic antibody or antibody fragment by minimizing steric hindrance. For this reason, small molecules typically have a molecular weight of less than 900 a.m.u., or more preferably less than 500 a.m.u. or more preferably less than 200 a.m.u. or even more preferably less than 100 a.m.u. For example, hydrogen sulfide, bisulfide and bicarbonate all have low molecular weights and small structures that provide access to pockets on conditionally active senolytic antibody or antibody fragment.

The concentration of the species in the assay solutions is for example at or near the physiological concentration of the species in a subject. For example, the physiological concentration of bicarbonate (in human serum) is in the range of 15 to 30 mM. Thus, the concentration of bicarbonate in the assay solutions may be from 10 mM to 40 mM, or from 15 mM to 30 mM, or from 20 mM to 25 mM, or about 20 mM. The physiological concentration of bisulfide is also low. The concentration of bisulfide in the assay solutions may be from 3 to 500 nM, or from 5 to 200 nM, or from 10 to 100 nM, or from 10 to 50 nM.

The species may be present in the assay solution for the extracellular condition of senescent cells and the assay solution for the normal physiological condition at substantially the same concentration, e.g. about 20 µM for bicarbonate.

In some embodiments, the conditionally active senolytic antibody or antibody fragment is pH-dependent when two or more different small molecules are present, for example, a combination of bicarbonate and histidine. Therefore, these two or more small molecules are present in the assay solutions.

The species in the assay solutions may be formed *in situ* from a component of the assay solutions or be directly included in the assay solutions. For example, CO₂ from the air may dissolve in the assay solutions to provide bicarbonate as the species in the assay solutions. For another example, sodium dihydrogen phosphate may be added to the assay solution to provide dihydrogen phosphate as the species in the assay solutions.

When the species is absent, the conditionally active senolytic antibodies or antibody fragments may lose the pH-dependency. Thus, in the absence of the species, the conditionally active antibodies or antibody fragments may have similar activity between the lower pH of extracellular condition of senescent cells and the normal physiological pH in the absence of the species. This same result can be achieved based on any extracellular condition of a senescent cell that differs from a normal physiological condition.

In some embodiments, the conditionally active senolytic antibody or antibody fragment shows an increased activity at the lower pH of an extracellular condition of senescent cells in comparison with the same activity at the normal physiological pH, in the presence of an ancillary protein. The ancillary protein may be a protein present in blood or human serum. One suitable protein may be albumin, particularly mammalian albumin, such as bovine albumin or human albumin.

In one aspect, the ancillary protein such as albumin is present in the assay solutions used for screening and selecting the conditionally active senolytic antibody or antibody fragment from the mutant antibodies or antibody fragments produced by the evolving step. In another aspect, the assay solutions with the ancillary protein such as albumin are also used to test the activity of the selected conditionally active senolytic antibody or antibody fragment under the same or different conditions.

In some embodiments, the two or more of these inorganic compounds, ions, and organic molecules discussed in this application are added at substantially the same concentrations to both assay solutions for normal physiological pH and extracellular pH of senescent cells. For example, both bicarbonate and histidine are added to both assay solutions.

In one embodiment, human serum may be added to both assay solutions for normal physiological pH and extracellular pH of senescent cells at substantially the same concentration. Because the human serum has a large number of inorganic compounds, ions, organic molecules (including proteins), the assay solutions will have multiple and large number of components selected from inorganic compounds, ions, organic molecules presented at substantially the same concentrations between the two assay solutions.

In some other embodiments, at least one of the two or more components is added to the assay solutions for normal physiological pH and extracellular pH of senescent cells at different concentrations. For example, both bicarbonate and histidine are added to the assay solutions. The bicarbonate concentration may be different between the assay solutions, while the histidine may have the same concentration in both assay solutions.

In some embodiments, the assay solutions may be designed for selecting conditionally active biological senolytic antibodies or antibody fragments with an activity dependent on two or more conditions. In one exemplary embodiment, the conditionally active senolytic antibody or antibody fragment may have binding activity dependent on both pH and bicarbonate. The assay solutions for selecting such a conditionally active senolytic antibody or antibody fragment may be an assay solution for the normal physiological condition with pH at 7.2-7.6, bicarbonate at a concentration in the range of from 25 to 30 mM. The assay solution for the extracellular condition of senescent cells may be with pH at 6.4-6.8, bicarbonate at a concentration in the range of from 10 to 20 mM. Optionally the assay solutions for both normal physiological condition and extracellular condition of senescent cells may also comprise an ion to assist the binding between the mutant antibodies or antibody fragments and the binding partner, thus to increase the number of hits for conditionally active senolytic antibodies or antibody fragments.

In some embodiments, certain components of serum may be purposely minimized or omitted from the assay solutions. For example, when screening antibodies, components of serum that bind with or adsorb antibodies can be minimized in or omitted from the assay solutions. Such bound antibodies may give false positives thereby including bound mutant antibodies that are not conditionally active but rather are merely bound to a component present in serum under a variety of different conditions. Thus, careful selection of assay components to minimize or omit components that can potentially bind with mutant antibodies or antibody fragments in the assay may reduce the number of false positive mutant antibodies or antibody fragments that may be inadvertently identified as positive for conditional activity due to binding to a component in the assay other than the desired binding partner. For example, in some embodiments where mutant antibodies or antibody fragments having a propensity to bind with components in human serum are being screened, bovine serum albumin may be used in the assay solution in order to reduce or eliminate the possibility of false positives caused by mutant antibodies or antibody fragments binding to components of human serum. Other similar replacements can also be made in particular cases to achieve the same goal, which is well appreciated by skilled person in the art.

In some embodiments, the evolving step may produce mutant antibodies or antibody fragments that may simultaneously have other desired properties besides the conditionally active characteristics discussed above. Suitable other desired properties that may be evolved may include binding affinity, expression, humanization, etc. Therefore, the present invention may be employed to produce a conditionally active senolytic antibody or antibody fragment that also has an improvement in at least one or more of these other desired properties.

In some embodiments, the conditionally active senolytic antibody or antibody fragment may be further mutated using one of the mutagenesis techniques disclosed herein in, for example, a second evolving step, to improve another property of the conditionally active antibody or antibody fragment such as binding affinity, expression, humanization, etc. After the second evolving step, the mutant antibodies or antibody fragments may be screened for both the conditional binding activity and the improved property.

In some embodiments, after evolving the parent antibody or antibody fragment to produce mutant antibodies or antibody fragments, a first conditionally active antibody or antibody fragment is selected, which exhibits at least one of:
(a) a decrease in binding activity to the target in the assay under the normal physiological pH of 7.2-7.8 compared to the same activity of the parent antibody or antibody fragment in the same assay, and an increase in the binding activity to the target in the assay under the extracellular pH of 5.5-7.0 of the senescent cell compared to the same activity of the conditionally active antibody or antibody fragment in the assay under the normal physiological pH of 7.2-7.8; and
(b) a decrease in the binding activity to the target in the assay under the normal physiological pH of 7.2-7.8 compared to the same activity of the parent antibodies or antibody fragment in the same assay and an increase in the binding activity to the target in the assay under the extracellular pH of 5.5-7.0 of the senescent cell compared to the same activity of the parent antibody or antibody fragment in the assay under the extracellular pH of 5.5-7.0 of the senescent cell.

The selected first conditionally active antibodies or antibody fragment may then be further subjected to one or more additional evolving, expressing and selecting steps to select at least a second conditionally active antibodies or antibody fragment that also exhibits at least one of:
(a) a decrease in an activity in the assay under the normal physiological pH of 7.2-7.8 compared to the same activity of the parent antibodies or antibody fragment in the same assay, and an increase in the activity in the assay under the extracellular pH of 5.5-7.0 of the senescent cell compared to the same activity of the conditionally active antibodies or antibody fragment in the assay under the normal physiological pH; and
(b) a decrease in the activity in the assay under the normal physiological pH of 7.2-7.8 compared to the same activity of the parent antibodies or antibody fragment in the same assay and an increase in the activity in the assay under the extracellular pH of 5.5-7.0 of the senescent cell compared to the same activity of the parent antibodies or antibody fragment in the assay under the extracellular pH of 5.5-7.0 of the senescent cell. The second activity may be the same as the first activity, in which case it is desirable for the second conditionally active antibodies or antibody fragment to have a larger ratio between the binding activity at the extracellular pH and the binding activity at the normal physiological pH, in comparison with the first conditionally active senolytic antibodies or antibody fragment. In some embodiments, the second activity may be a different activity than the first activity in which case an activity such as internalization efficiency or binding to a specific epitope may be the second activity.

In certain embodiments, the present invention is aimed at producing conditionally active senolytic antibodies or antibody fragments with a ratio of the binding activity at the extracellular pH of the senescent cell to the binding activity at the normal physiological pH greater than 1.0 (e.g., a high selectivity between the two pHs). The ratio of binding activity, or selectivity, at the extracellular pH of the senescent cell to the binding activity at the normal physiological pH may be at least about 1.3:1, or at least about 2:1, or at least about 3:1, or at least about 4:1, or at least about 5:1, or at least about 6:1, or at least about 7:1, or at least about 8:1, or at least about 9:1, or at least about 10:1, or at least about 11:1, or at least about 12:1, or at least about 13:1, or at least about 14:1, or at least about 15:1, or at least about 16:1, or at least about 17:1, or at least about 18:1, or at least about 19:1, or at least about 20:1, or at least about 30:1, or at least about 40:1, or at least about 50:1, or at least about 60:1, or at least about 70:1, or at least about 80:1, or at least about 90:1, or at least about 100:1.

In one embodiment, the conditionally active senolytic antibody or antibody fragment is an antibody, which may have a ratio of the binding activity at the extracellular pH of the senescent cell to the binding activity at the normal physiological pH of at least about 5:1, or at least about 6:1, or at least about 7:1, or at least about 8:1, or at least about 9:1, or at least about 10:1, or at least about 20:1, or at least about 40:1, or at least about 70:1, or at least about 100:1.

In some embodiments, the conditionally active senolytic antibody or antibody fragment is a probody that comprises an antibody or an antibody fragment (collectively referred to as an "antibody") conjugated to a masking moiety (MM) through a linker (L). The probody is more active in the extracellular environment of senescent cells in comparison with the extracellular environment of normal cells. Particularly, in the extracellular environment of normal cells, the masking moiety of the probody will mask the activity of the antibody, which will, as a result, have a lower binding activity to the target senescent cells. The masking moiety will be cleaved from the antibody by a protease present in the extracellular environment of senescent cells. The antibody is thereby unmasked and free to bind to the target senescent cells. Therefore, the probody has an increased binding activity to the target senescent cells in the extracellular environment of the senescent cells than the binding activity to the same target in the extracellular environment of normal cells.

The antibody fragment that may be included in the probody may include variable or hypervariable regions of light and/or heavy chains of an antibody (V_{L}, V_{H}), variable fragments (Fv), Fab' fragments, F(ab')2 fragments, Fab fragments, single chain antibodies (scAb), single chain variable regions (scFv), complementarity determining regions (CDRs), domain antibodies (dAbs), single domain heavy chain immunoglobulins of the BHH or BNAR type and single domain light chain immunoglobulins.

The masking moiety functions to reduce the binding activity of the antibody in the probody to the target senescent cells, in comparison with the binding activity of the same antibody without the masking moiety (e.g. after the masking moiety is cleaved from the probody). The binding activity of the antibody to the target senescent cell may be reduced by the masking moiety by at least 50%, at least 55%, at least 60%, at least 65%, at least 70%, at least 75%, at least 80%, at least 85%, at least 90%, at least 92%, at least 93%, at least 94%, at least 95%, at least 96%, at least 97%, at least 98%, at least 99% or even 100%. The reduction in binding activity may be, for example, for a period of at least 2, 4, 6, 8, 12, 28, 24, 30, 36, 48, 60, 72, 84, or 96 hours.

In one embodiment, the masking moiety (MM) is conjugated through a linker (L) to one or more variable regions of the antibody (Ab) to create a barrier between the antibody and the target senescent cells. For example, the masking moiety may be conjugated to the N-terminus of the one or more variable regions. The masking moiety and the linker form a single chain conjugated to the N-terminus of the one or more variable regions. In another example, the masking moiety may be conjugated to a side chain of an amino acid of the one or more variable regions in which case the masking moiety and the linker form a single chain conjugated to a side chain of an amino acid in the one or more variable regions. In yet another example, the masking moiety is conjugated to the C-terminus of the one or more variable regions when the probody comprises only a fragment of an antibody (such as only the variable regions). In some embodiments, the probody has a structure from the N-terminus to the C-terminus of MM-L-Ab. In other embodiments, the probody has a structure from the N-terminus to the C-terminus of Ab-L-MM.

In some embodiments, the masking moiety may be identified by screening a library of diverse peptides for a peptide that binds to one or more of the variable regions of the antibody (Desnoyers et al., "Tumor-specific activation of an EGFR-targeting probody enhances therapeutic index," Sci Transl Med., vol. 5, 207ra144, 2013). The peptide that can specifically bind to the antibody and block the binding of the antibody to the target senescent cell when conjugated to the antibody through the linker is selected as the masking moiety. The screening may be conducted using known techniques including, but not limited to, panning, fluorescence activated cell sorting and magnetic selection with streptavidin-coated magnetic beads (Rice et al., "Bacterial display using circularly permuted outer membrane protein OmpX yields high affinity peptide ligands," Protein Sciences, vol. 15, pp. 825-36, 2006).

In some embodiments, a random peptide library (e.g., peptides having from about 2 to about 40 amino acids, or about 5 to about 30 amino acids, or about 8 to about 20 amino acids, or more than 40 amino acids) may be used in the screening method to identify a suitable masking moiety. For example, a masking moiety with a specific binding affinity for the antibody can be identified through a screening procedure that includes providing a library of peptide scaffolds wherein each scaffold is made up of a transmembrane protein and a candidate. The library is then contacted with the antibody for identifying one or more suitable masking moieties having detectable binding activity to the antibody. Screening can include one more rounds of magnetic-activated sorting or fluorescence activated cell sorting.

Thus, the masking moiety may be specific for the antibody in the probody. One masking moiety that works well for a particular antibody may be less than optimal for another antibody. Thus, screening of a diverse peptide library using the antibody in the probody for a masking moiety best for the antibody may be important for some embodiments of the present invention.

In some embodiments, the masking moiety is screened from a diverse library of synthetic peptides. This type of masking moiety may have a certain level of similarity to the target senescent cell (the natural binding partner of the antibody). In certain embodiments, the masking moiety may be modeled after the natural binding partner of the antibody. For example, the natural binding partner may be modified by changing one or more amino acid residues to slightly decrease its binding activity to the antibody. In other embodiments, the masking moiety has no more than 5%, no more than 7%, no more than 10%, no more than 15%, no more than 20%, no more than 25%, no more than 30%, no more than 35%, no more than 40%, no more than 45%, no more than 50%, no more than 55%, no more than 60%, no more than 65%, no more than 70%, no more than 75%, or no more than 80% sequence identity with the natural binding partner of the antibody.

The structural properties of the masking moiety depend on several factors such as the minimum amino acid sequence required for interference with antibody binding to the target senescent cell, the size of the antibody (full antibody or fragment), the length of the linker, and the like. In some embodiments, the masking moiety is coupled to the antibody by covalent bonding. In one example, the antibody is coupled to the masking moiety by cysteine-cysteine disulfide bridges between the linker and the antibody. In another example, the antibody is coupled to the masking moiety by a peptide bond between the linker and the antibody.

In some embodiments, the masking moiety may not specifically bind to the antibody, but rather will only interfere with the binding of the antibody to the target senescent cell through one or more non-specific interactions such as steric hindrance. For example, the masking moiety may be positioned in the probody such that the structure of the probody allows the masking moiety to mask the antibody through charge-based interaction, thereby holding the masking moiety in place to interfere with access to the binding site on the antibody.

The linker of the probody is positioned between the masking moiety and the antibody. The linker comprises a cleavage site (CS) where a protease present in the extracellular environment of senescent cells will cleave the linker to release the masking moiety from the probody. The antibody will then be unmasked and available to bind to the target senescent cell. The linker may further comprise one or more flexible regions (FR) that flank one or both sides of the cleavage site. For example, the linker may have the structure of: -FR-CS-FR-, -FR-CS-, -CS-FR-, -FR-FR-CS-, -CS-FR-FR-, -FR-FR-CS-FR-, -FR-CS-FR-FR-, -FR-FR-CS-FR-FR-.

The flexible region provides flexibility to the conformation of the masking moiety to allow the masking moiety to reach the binding site of the antibody and interfere with its binding. The flexible region consists essentially of small amino acids such as glycine, serine, and alanine that have small side chains to provide maximal flexibility. Glycine and glycine-serine polymers are relatively unstructured, and therefore may be able to serve as a neutral tether between components. Glycine accesses significantly more phi-psi space than even alanine, and is much less restricted than residues with longer side chains (see Scheraga, Rev. Computational Chem., pp. 11173-11142, 1992).

Suitable flexible regions can have different lengths, such as from 1 amino acid to 20 amino acids, from 2 amino acids to 15 amino acids, from 3 amino acids to 12 amino acids, from 4 amino acids to 10 amino acids, from 5 amino acids to 9 amino acids, from 6 amino acids to 8 amino acids, or from 7 amino acids to 8 amino acids, and may be 1, 2, 3, 4, 5, 6, or 7 amino acids in length.

Exemplary flexible regions include glycine polymers (G)n, glycine-serine polymers (including, for example, (GS)n (SEQ ID NO: 14), (GGS)n (SEQ ID NO: 15), (GSGGS)n (SEQ ID NO: 16), (GSGGS)n (SEQ ID NO: 17), and (GGGS)n (SEQ ID NO: 18), where n is an integer of at least one, glycine-alanine polymers, alanine-serine polymers, and other flexible regions known in the art. More examples of flexible regions include GGSG (SEQ ID NO: 19), GGSGG (SEQ ID NO: 20), GSGSG (SEQ ID NO: 21), GSGGG (SEQ ID NO: 22), GGGSG (SEQ ID NO: 23), and GSSSG (SEQ ID NO: 24), GSSGGSGGSGGSG (SEQ ID NO: 25), GSSGGSGGSGG (SEQ ID NO: 26), GSSGGSGGSGGS (SEQ ID NO: 27), GSSGGSGGSGGSGGGS (SEQ ID NO: 28), GSSGGSGGSG (SEQ ID NO: 29), or GSSGGSGGSGS (SEQ ID NO: 30), GSSGT (SEQ ID NO: 31) or GSSG (SEQ ID NO: 32).

The cleavage site is a substrate for a protease in the extracellular environment of senescent cells. The cleavage site is commonly included as a part of the linker. But in some cases, the cleavage site may be part of the masking moiety, such that all or a portion of the cleavage site facilitates masking of the antibody when the probody is in the inhibited or uncleaved or masked state.

The cleavage site may be selected based on the protease in the extracellular environment of senescent cells. The senescent cells are known to secret proteases into their extracellular environment, such as the matrix metalloproteinases (MMPs). Examples of MMP family members include stromelysin-1 and -2 (MMP-3 and -10, respectively) and collagenase-1 (MMP-1). Other MMPs include MMP1, MMP2, MMP7, MMP8, MMP9, MMP13, and MMP14. The natural substrates of these proteases are also known, which can assist to design the cleavage site used in the probodies. For example, these MMPs can cleave MCP-1, -2, and -4 and IL-8. A variety of other CXCL/CCL family members can also be cleaved by MMP-9, -2, or -7. Serine proteases are also present in the extracellular environment of senescent cells. Members of serine proteases include urokinase- or tissue-type plasminogen activators (uPA or tPA, respectively). See Coppe et al., "The Senescence-Associated Secretory Phenotype: The Dark Side of Tumor Suppression," Annu Rev Pathol., vol. 5, pp. 99-118, 2010.

In one exemplary embodiment, the cleavage site is a substrate of a matrix metalloprotease, and thus is cleavable by the MMP to release the making moiety. In another embodiment, the cleavage site is a substrate of a serine uPA, or PSA. In some embodiments, the probody can comprises more than one cleavage site, and each can be a substrate of a different protease. Exemplary cleavage sites that may be substrates of proteases include: ADAM10, ADAM12, ADAM17, ADAMTS, ADAMTS5, BACE, Caspase 1-14, Cathepsin A, Cathepsin B, Cathepsin D, Cathepsin E, Cathepsin K, Cathepsin S, FAP, MT1-MMP, Granzyme B, Guanidinobenzoatase, Hepsin, Human Neutrophil Elastase, Legumain, Matriptase 2, Meprin, MMP1-17, MT-SP1, Neprilysin, NS3/4A, Plasmin, PSA, PSMA, TRACE, TMPRSS 3, TMPRSS 4, and uPA. Some exemplary cleavage sites are PLGLWA (SEQ ID NO: 33) that can be cleaved by MMPs and GPQGIAGQ (SEQ ID NO: 34) that can be cleaved by collagenase. Other examples of cleavage sites include YGLLGIAGPPGP (SEQ ID NO: 35), SPGRVVRG (SEQ ID NO: 36), VRG (SEQ ID NO: 37).

In some embodiments, the antibody in the probody is itself conditionally active. Particularly, the antibody itself may have a higher binding activity to its target in a condition in the extracellular environment of senescent cells in comparison with the same binding activity to the target at a normal physiological condition. Such a probody provides a double boost once the probody reaches the extracellular environment of the senescent cells by (1) cleaving the masking moiety to free the binding site of the antibody from the masking moiety, and (2) the antibody having an increased binding activity to the target at the condition in the extracellular environment of senescent cells in comparison with the binding activity at the normal physiological condition.

In one embodiment, the conditionally active senolytic antibody or antibody fragment is an antibody that is intended to be conjugated with another agent. The conditionally active antibody may have a high ratio of the activity at the extracellular pH of the senescent cell to the activity at the normal physiological pH of at least about 10:1, or at least about 11:1, or at least about 12:1, or at least about 13:1, or at least about 14:1, or at least about 15:1, or at least about 16:1, or at least about 17:1, or at least about 18:1, or at least about 19:1, or at least about 20:1, or at least about 40:1, or at least about 60:1, or at least about 80:1, or at least about 100:1. This may be particularly important when the conjugated agent is, for example, toxic or radioactive, since such a conjugated agent is desirably concentrated at the disease or treatment site.

In some embodiments, the conjugated agent is a D retro inverso peptide ("DRI peptide"). The DRI peptide, because of the D amino acids in a reverse sequence, can maintain the side chain topology of the amino acids similar to that of the natural protein from which it is derived. In addition, the DRI peptide is more resistant to proteolytic degradation, thus tends to have a much longer half-life than the natural protein from which it is derived. Furthermore, the DRI peptide has a structure that is similar to the structure of the natural protein from which it is derived. Finally, the DRI peptide has a bioavailability that is comparable with the natural protein from which it is derived. Thus, the DRI peptide can be functional replacement for the natural protein from which it is derived and can compete with the natural protein from which it is derived. DRI peptides are thus viewed as promising pharmaceutical agents.

FOXO4 is a molecular pivot that decides whether damaged cells undergo senescence or apoptosis. The FOXO protein family, including FOXO1, 3, and 4, are negatively regulated by growth factor signaling, but can also be activated by oxidative stress (Brunet, A. et al., Science, vol. 303, pp. 2011-2015 (2004); de Keizer, P. L. et al., Cancer Res, vol. 70, pp. 8526-8536 (2010); Essers, M. A. et al., EMBO J., vol. 23, pp. 4802-4812 (2004)). Constitutive foxo1-/- mice are embryonic lethal and foxo3-/- mice show reproductive deficiencies, but foxo4-/- mice do not harbor a significantly defective phenotype (Hosaka, T. et al., Proc. Natl. Acad. Sci. U.S.A, vol. 101, pp. 2975-2980 (2004); Castrillon, D. H. et al., Science, vol. 301, pp. 215-218 (2003)). Individual conditional somatic foxo3-/- mice show a slightly shortened lifespan, whereas conditional somatic foxo1-/- and foxo4-/- do not (Paik, J. H. et al., Cell, vol. 128, pp. 309-323 (2007)). Somatic triple foxo1,3,4-/- mice show an increase in lymphoma thus indicating that in this respect FOXO proteins are functionally redundant (*Id*.). Notably however, single somatic foxo4-/- mice do not show any shortened lifespan, nor any changes in tumor-free survival. Further, unlike its counterparts FOXO1 and FOXO3, FOXO4 mRNA and protein expression rise significantly in response to senescence-inducing levels of DNA damage.

Senescence caused by ionizing radiation (XRAY)-induced DNA damage is characterized by the formation of persistent nuclear foci termed DNA-SCARS (or DNA Segments with Chromatin Alterations Reinforcing Senescence), which are required for the growth arrest (Rodier, F. et al., J Cell Sci, vol. 124, pp. 68-81 (2011)). Under these DNA-damaging conditions, a loss of FOXO4 expression using stable short hairpin-based RNA interference (shRNA) induced apoptosis instead of senescence. This shows that FOXO4 is a pivotal factor in the molecular decision of whether cells senesce or apoptosis occurs in response to genotoxic stress.

The mechanism by which FOXO4 restrains apoptosis in favor of senescence involves its physical association with the p53 tumor suppressor protein. p53 is well known to regulate cell fate after DNA damage (Rodier, F. et al., Nucleic Acids Res, vol. 35, pp. 7475-7484 (2007)) and is a major component of DNA-SCARS (Rodier, F. et al., Nat. Cell Biol., vol. 11, pp. 973-979 (2009)). p53 can induce senescence as well as apoptosis, depending on its post-translational modifications and its interaction partners (Vousden, K. H. et al., Nat. Rev. Mol. Cell Biol., vol. 8, pp. 275-283 (2007)). When phosphorylated on Ser46, p53 strongly favors apoptosis over cell cycle arrest (Bulavin, D. V. et al., EMBO J., vol. 18, pp. 6845-6854 (1999)). However, Ser46 is phosphorylated in response to several senescence-inducing stimuli, including activated oncogenes (Feng, L. et al., Cell Cycle, vol. 5, pp. 2812-2819 (2006); Bischof, O. et al., EMBO J., vol. 21, pp. 3358-3369 (2002)). Under DNA damaging conditions, Ser46-phosphorylation of p53 becomes elevated and that interference with the HIPK2 kinase, which is responsible for Ser46-phosphorylation (Dauth, I. et al., Cancer Res, vol. 67, pp. 2274-2279 (2007)), impairs the apoptotic response caused by FOXO4 depletion. Thus, FOXO4 restrains apoptosis in senescent cells by repressing the apoptotic function of p53 signaling in favor of senescence. Inhibition of FOXO4, especially its interaction with p53, will switch senescent cells into apoptosis.

Human FOXO4 protein has two variants (SEQ ID NOS: 1 and 2). In some embodiments, any fragment of the FOXO4 protein may be used as the basis to design a FOXO4 DRI peptide. In one embodiment, the FOXO4 fragment comprises at least a portion of a functional domain of the FOXO4 protein, such as its DNA binding domain (SEQ ID NO:3) or p53 interaction domain (SEQ ID NO:4).

Any FOXO4 DRI peptide that can inhibit the function of FOXO4 and/or interfere with its interaction with p53 may be used as the conjugate agent to a conditionally active antibody. Particularly, three FOXO4 DRI peptides are preferred for effectively interfering with the interaction between FOXO4 and p53: LTLRKEPASE IAQSILEAYS QNGWANRRSG GKRP (SEQ ID NO:5), LTLRKEPASE IAQSILEAYS QNGWANRRSG GKRPPPRRRQ RRKKRG (SEQ ID NO:6), and SEIAQSILEAYSQNGW (SEQ ID NO:7). These three FOXO4 DRI peptides all consist of D amino acid residues. At least some of the D amino acid residues in these FOXO4 DRI peptides may be replaced with L amino acid residues without significantly diminishing their ability to induce apoptosis in senescent cells. These FOXO4 DRI peptides interfere with the interaction between FOXO4 and p53 thereby inhibiting FOXO4's function of suppressing p53, which leads to apoptosis in senescent cells.

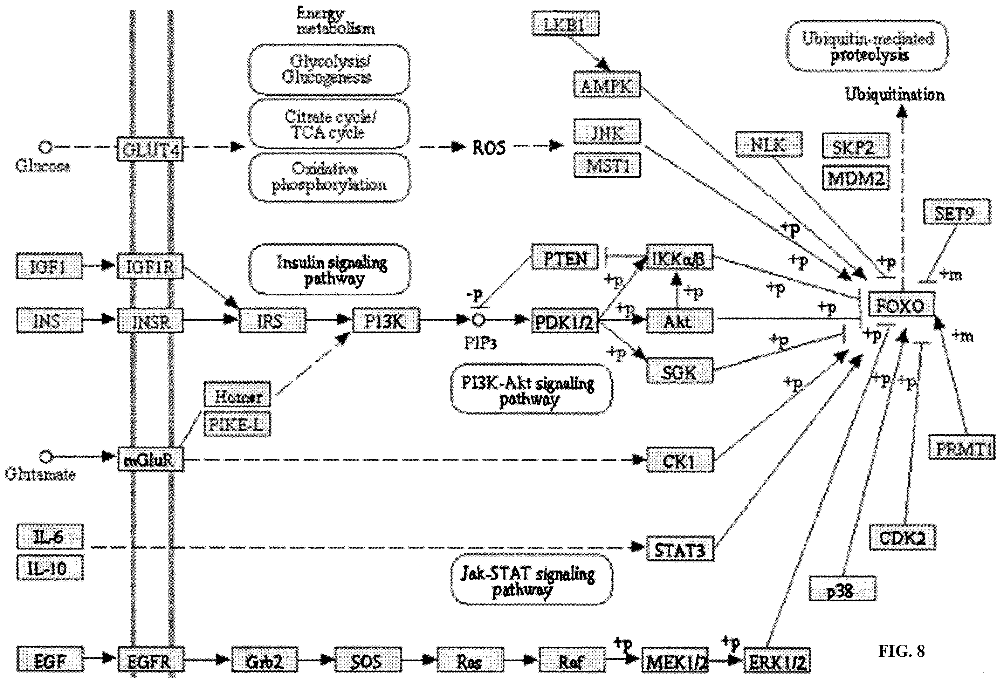

FOXO4 is itself regulated by other proteins. Referring to FIG. 8, the members of the FOXO family, including FOXO4, are activated through phosphorylation or methylation by other proteins: AMPK, JNK, MST1, CK1, STAT3, p38 through phosphorylation, and PRMT1 through methylation. The stress-activated c-Jun N-terminal kinase (JNK) and the energy sensing AMP-activated protein kinase (AMPK), upon exposure to oxidative and nutrient stress stimuli, phosphorylate and activate FOXOs. Any protein that activates FOXO4 may be the basis (i.e., the natural or wild-type protein) for design of a DRI peptide useful in the invention. In some embodiments, the natural protein is selected from the group consisting of AMPK, JNK, MST1, CK1, STAT3, p38 and PRMT1.

Taking the JNK protein as an example. JNK is a c-Jun N-terminal kinase that can phosphorylate and activate FOXO4. Human JNK has an amino acid sequence of SEQ ID NO: 8. A DRI peptide based on the JNK protein can modulate JNK allosterically and selectively by blocking access to its substrates using a competitive mechanism (Bonny, C. et al. Diabetes, vol. 50, pp. 77-82 (2001); Borsello, T. et al. Trends Mol Med, vol. 10, pp. 239-244, (2004); and Borsello, T. et al. Nat Med, vol. 9, pp. 1180-1186, (2003)). One exemplary JNK DRI peptide is DQSRPVQPFLQLTTPRKP (SEQ ID NO:9).

Furthermore, activators of AMPK, JNK, MST1, CK1, STAT3, p38 and PRMT1 may also be used as the natural protein for design of the DRI peptide. For example, ASKl is an apoptosis signal-regulating kinase 1, which activates JNK. Human ASKl has a GenBank accession number No. NP_005914. The ASK1 protein may be the natural protein for design of DRI peptide. Such a DRI peptide can inhibit ASK1, thus suppressing the activity of JNK, which will lead to inhibition of FOXO4.

In some embodiments, the natural proteins for design of the DRI peptides are human proteins, such as human FOXO4, AMPK, JNK, MST1, CK1, STAT3, p38, PRMT1, and ASK1. In some other embodiments, the natural proteins for design of the DRI peptides are mammalian proteins, such as primate or mouse proteins of FOXO4, AMPK, JNK, MST1, CK1, STAT3, p38, PRMT1, and ASK1. It is commonly understood that an ortholog protein may also function in another species, which means that a DRI peptide designed based on an ortholog may function in another species. For example, a DRI peptide designed based on the mouse FOXO4 will likely function on human FOXO4, and thus may be used as a conjugate for inducing apoptosis of senescent cells in humans.

In one embodiment, a fragment of the natural protein is used to design the DRI peptides. In another embodiment, the full length of the natural protein is used to design the DRI peptide. In these embodiments, the amino acid sequences of the DRI peptides are the exact reverse of the amino acid sequences of the fragments or the full length of the natural proteins of FOXO4, AMPK, JNK, MST1, CK1, STAT3, p38, PRMT1, and ASK1.

In some embodiments, the amino acid sequences of the DRI peptides are not the exact reverse of the amino acid sequences of the fragments or the full length of the natural proteins of FOXO4, AMPK, JNK, MST1, CK1, STAT3, p38, PRMT1, and ASK1. In such embodiments, the amino acid sequences of the DRI peptides may have at least 51, 52, 53, 54, 55, 56, 57, 58, 59,60 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100% sequence identity to the reversed sequence of the fragment or full length of the natural protein.

The DRI peptides may for example be small peptides for enabling entry of the DRI peptides into the senescent cells. In some embodiments, the DRI peptides contain 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59,60 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, or more amino acid residues.

Though the DRI peptides in some embodiments consist of all D amino acid residues, some functional DRI peptides may contain a combination of L-amino acid residues and D amino acid residues. In some embodiments, the DRI peptides have at most 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, 55, or 60 % amino acid residues that are L amino acid residues.

In some embodiments, the DRI peptides may further comprise one or more functional domains that are not part of a natural protein that serves as the basis used for design the DRI peptides. In one embodiments, the DRI peptides comprise the sequence "PPRRRQRRKKRG" (SEQ ID NO:10), which facilitates entry of the DRI peptides into the senescent cells to induce apoptosis. The skilled person understands that this functional domain can be replaced by any other protein domains that facilitate entry of the DRI peptide into the senescent cells.

Some other functional domains that may be included in the DRI peptides include a cell permeable peptide ("CPP"), such as the primary amphipatic peptide MPG (GALFLGFLGA AGSTMGAWSQ PKKKRKV, SEQ ID NO:11), Pep-1 (KETWWETWWT EWSQPKKKRKV, SEQ ID NO:12), a secondary amphipathic peptide CADY (Ac-GLWRALWRLLRSLWRLLWRA-Cya, SEQ ID NO:13) or octa-arginine (R(8)).

The functional domains in the DRI peptides do not themselves have any apoptosis-inducing activity, but may serve to increase the apoptosis-inducing activity of another portion of the DRI peptides. The functional domains comprise at least 1, 2, 3, 4, 5, 6, 7, or 10 D amino acid residues, more preferably all amino acid residues of the functional domains are D amino acid residues.

The DRI peptides have apoptosis-inducing activity in senescent cells if they kill, clear, remove, inactivate or reduce the viability of senescent cells. In some embodiments, the DRI peptides can kill, clear, remove, inactivate or reduce the viability of at least 5, 10, 15, 20, 25, 30, 40, 50, 60, 70, 80, 90 or 95% of the cells in a senescent cell culture.

In some embodiments, the DRI peptides selectively exhibit apoptosis-inducing activity in senescent cells, and thus have little or no apoptosis-inducing activity in non-senescent cells. The DRI peptides may favor apoptosis in senescent cells over apoptosis in non-senescent cells by at least a ratio of 1.1, 1.2, 1.3, 1.4, 1.5, 1.6, 1.7, 1.8, 1.9, 2.0, 2.5, 3, 4, 5, 6, 7, 8, 9, 10, or higher.

Using common general knowledge, one skilled in the art can assess via a standard *in vitro* test whether a DRI peptide exhibits apoptosis-inducing activity in senescent cells. For example, a cell culture of senescent cells can be obtained by subjecting said cell culture to ionizing radiation or a chemotherapeutic agent and then mixed with non-senescent cells. Other ways of providing senescent cells are (i) continuous passaging until replicative senescence occurs (=telomere shortening), (ii) via the use oxidative stressors such as H₂O₂ and Rotenone, (iii) chromatin remodelers as Sodium dibutyrate, or (iv) expression of hyperactivated oncogenes such as RASG12V or BRAFV600E. The presence of senescence cells can be established by testing for SA-B-GAL.

The second step is to administer to the cell culture a peptide and measure one or more markers of apoptosis, such as (i) staining for cytoplasmic cytochrome C or (ii) staining for TUNEL. Cytochrome C data can be quantified by counting the number of cells (DAPI can be used to indicate a cell) in which Cytochrome C has been released from the mitochondria to the cytosol or (at later stages) the number of cells that have disappeared completely. This assay can be done in the presence of a caspase-inhibitor so that the cells that are about to undergo apoptosis (indicated by release of Cytochrome C into the cytosol) are not allowed to actually die as caspases are required for that. The benefit of this assay is that it is possible to get a cumulative count on the amount of senescence over several days (for example 5 days). In TUNEL staining, the percentage of nuclei (DAPI-positive) which stain positive for TUNEL are counted. This can easily be performed by eye, but it is also possible to use a software tool called Cellprofiler (freeware).

In some embodiments, the conditionally active senolytic antibody or antibody fragment comprises a prodrug that is covalently bonded to a peptide linker, which in turn is conjugated to the conditionally active senolytic antibody or antibody fragment. A prodrug is a drug that is conjugated to the peptide linker. Due to the presence of the covalently bonded peptide linker, the drug is not in an active form. The peptide linker can be cleaved by a protease in the extracellular environment of senescent cells, thus releasing the covalently bonded drug from the conditionally active senolytic antibody or antibody fragment in an active form.

The peptide linker between the drug and the conditionally active senolytic antibody or antibody fragment may comprise the same cleavage sites that are used in the probodies described in this application (e.g., cleavage sites with SEQ ID NOS: 33-37). The same proteases in the extracellular environment of senescent cells that can release the antibody in the probodies will also cleave the peptide linkers to release the prodrug in an active form from the conditionally active senolytic antibody or antibody fragment in the extracellular environment of senescent cells.

In some embodiment, the peptide linker may be cleaved by the enzyme legumain. Such a peptide linker comprises a cleavage site for the legumain. Some exemplary cleavage sites are: PTN (SEQ ID NO: 38); PNN (SEQ ID NO: 39); PAN (SEQ ID NO: 40); PPN (SEQ ID NO: 41); TTN (SEQ ID NO: 42); TNN (SEQ ID NO: 43); TAN (SEQ ID NO: 44); TPN (SEQ ID NO: 45); NTN (SEQ ID NO: 46); NNN (SEQ ID NO: 47); NAN (SEQ ID NO: 48); NPN (SEQ ID NO: 49); ATN (SEQ ID NO: 50); ANN (SEQ ID NO: 51); AAN (SEQ ID NO: 52); APN (SEQ ID NO: 53); TTNL (SEQ ID NO: 54); TTNA (SEQ ID NO: 55); PTNL (SEQ ID NO: 56); PTNA (SEQ ID NO: 57); PNNL (SEQ ID NO: 58); PNNA (SEQ ID NO: 59); TNNL (SEQ ID NO: 60); TNNA (SEQ ID NO: 61); NK (SEQ ID NO: 62); NL (SEQ ID NO: 63); NA (SEQ ID NO: 64); NE (SEQ ID NO: 65); ND (SEQ ID NO: 66); and NN (SEQ ID NO: 67).

The drug covalently bonded to the peptide linker in the prodrug may be a cytotoxic drug, a cytostatic drug or an antiproliferative drug. These drugs are exemplified by:
Alkaloids: Docetaxel, Etoposide, Irinotecan, Paclitaxel, Teniposide, Topotecan, Vinblastine, Vincristine, Vindesine.
Alkylating agents: Busulfan, Improsulfan, Piposulfan, Benzodepa, Carboquone, Meturedepa, Uredepa, Altretamine, triethylenemelamine, Triethylenephosphoramide, Triethylenethiophosphoramide, Chlorambucil, Chloranaphazine, Cyclophosphamide, Estramustine, Ifosfamide, Mechlorethamine, Mechlorethamine Oxide Hcl, Melphalan, Novemebichin, Perfosfamide Phenesterine, Prednimustine, Trofosfamide, Uracil Mustard, Carmustine, Chlorozotocin, Fotemustine, Lomustine, Nimustine, Semustine Ranimustine, Dacarbazine, Mannomustine, Mitobronitol, Mitolactol, Pipobroman, Temozolomide.
Antibiotics and analogs: Aclacinomycins, Actinomycins, Anthramycin, Azaserine, Bleomycins, Cactinomycin, Carubicin, Carzinophilin, Cromomycins, Dactinomycins, Daunorubicin, 6-Diazo-5-oxo-L-norleucine, Doxorubicin, Epirubicin, Idarubicin, Menogaril, Mitomycins, Mycophenolic Acid, Nogalamycine, Olivomycins, Peplomycin, Pirarubicin, Plicamycin, Porfiromycin, Puromycine, Streptonigrin, Streptozocin, Tubercidin, Zinostatin, Zorubicin.
Antimetabolites: Denopterin, Edatrexate, Methotrexate, Piritrexim, Pteropterin, Tomudex, Trimetrexate, Cladridine, Fludarabine, 6-Mercaptopurine, Pentostatine Thiamiprine, Thioguanine, Ancitabine, Azacitidine, 6-Azauridine, Carmofur, Cytarabine, Doxifluridine, Emitefur, Floxuridine, Fluorouracil, Gemcitabine, Tegafur;
Platinum complexes: Caroplatin, Cisplatin, Miboplatin, Oxaliplatin;
Others: Aceglatone, Amsacrine, Bisantrene, Defosfamide, Demecolcine, Diaziquone, Eflomithine, Elliptinium Acetate, Etoglucid, Etopside, Fenretinide, Gallium Nitrate, Hdroxyurea, Lonidamine, Miltefosine, Mitoguazone, Mitoxantrone, Mopidamol, Nitracrine, Pentostatin, Phenamet, Podophillinic acid 2-Ethyl-Hydrazide, Procarbazine, Razoxane, Sobuzoxane, Spirogermanium, Teniposide Tenuazonic Acid, Triaziquone, 2,2',2"-Trichlorotriethylamine, Urethan.

The drug covalently bonded to the peptide linker in the prodrug may also be a chemotherapeutic drug. Chemotherapeutic drugs may inhibit senescent cells in different ways. Chemotherapeutic drugs can damage the DNA template by alkylation, by cross-linking, or by double-strand cleavage of DNA. Other chemotherapeutic drugs can block RNA synthesis by intercalation. Some chemotherapeutic drugs are spindle poisons, or anti-metabolites that inhibit enzyme activity, or hormonal and anti-hormonal agents. Chemotherapeutic drugs may be selected from various groups of agents, including but not limited to alkylating agents, antimetabolites, antitumor antibiotics, vinca alkaloids, epipodophyllotoxins, nitrosoureas, hormonal and antihormonal agents, and toxins. Some examples are the follows:
Examples of alkylating agents include cyclophosphamide, chlorambucil, busulfan, melphalan, thiotepa, ifosphamide, Nitrogen mustard.
Examples of antimetabolites include methotrexate, 5-Fluorouracil, cytosine arabinoside, 6-thioguanine, 6-mercaptopurin.
Examples of antitumor antibiotics include doxorubicin, daunorubicin, idorubicin, nimitoxantron, dactinomycin, bleomycin, mitomycin, plicamycin.
Examples of vinca alkaloids and epipodophyllotoxins include vincristin, vinblastin, vindestin, etoposide, teniposide.
Examples of nitrosoureas include carmustin, lomustin, semustin, streptozocin.
Examples of hormonal and antihormonal agents include adrenocorticorticoids, estrogens, antiestrogens, progestins, aromatas inhibitors, androgens, antiandrogens.
Examples of random synthetic agents include dacarbazin, hexamethylmelamine, hydroxyurea, mitotane, procarbazide, cisplastin, carboplatin.

The conditionally active senolytic antibodies or antibody fragments may be further engineered as described in WO 2016/138071. The conditionally active senolytic antibody or antibody fragment may be engineered through antibody conjugation, engineered to produce multispecific antibodies, engineering to produce a bi-specific conditionally active antibody against an immune effector-cell surface antigen, engineered to produce a masked conditionally active senolytic antibody or antibody fragment, and/or the Fc region of the antibodies may be engineered, each as described in WO 2016/138071. The conditionally active senolytic antibody or antibody fragment may also be used for engineering conditionally active viral particles, as described in WO 2015/175375.

T cells are used by the mammalian immune system for combating substances or cells having foreign antigens. CAR-T technology uses genetic engineering methods to reprogram natural circulating T cells by inserting a chimeric antigen receptor (CAR) into the T cells to produce highly specific CAR-T cells in which the CAR directs the engineered CAR-T cells to the target tissue by specifically binding to an antigen on the surface of the target tissue. Thus, the CAR-T cells can specifically target tumor cells, making the CAR-T cells much more effective than naturally circulating T cells. The CAR-T cells may be engineered to target senescent cells.

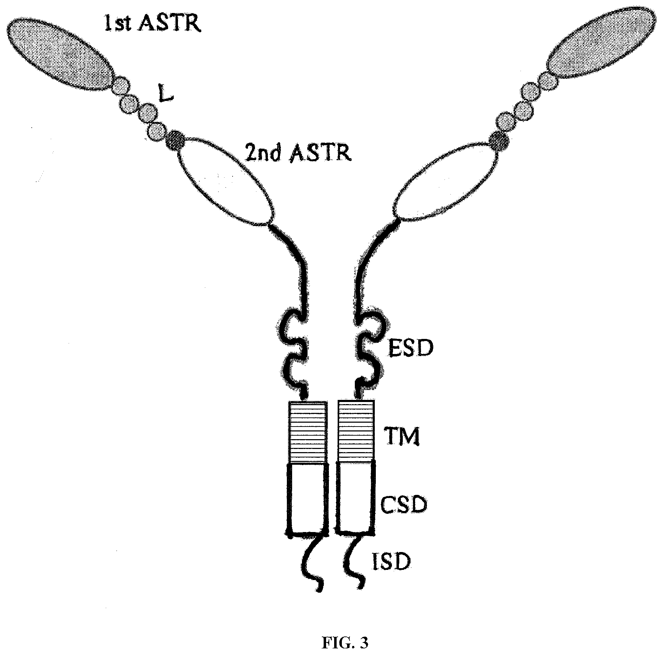

The CARs include at least one antigen specific targeting region (ASTR), an extracellular spacer domain (ESD), a transmembrane domain (TM), one or more co-stimulatory domains (CSD), and an intracellular signaling domain (ISD), see FIG. 3 and Jensen et al., "Design and implementation of adoptive therapy with chimeric antigen receptor-modified T cells," Immunol Rev., vol. 257, pp. 127-144, 2014. After the ASTR binds specifically to a target antigen, the ISD activates intracellular signaling in the CAR-T cells. For example, the ISD can redirect the CAR-T cell specificity and reactivity toward a selected target in a non-MHC-restricted manner, exploiting the antigen-binding properties of antibodies. The non-MHC-restricted antigen recognition gives the CAR-T cells the ability to recognize a senescent cell and initiate antigen processing. In an embodiment, the ESD and/or CSD are optional. In another embodiment, the ASTR has a bispecificity, which allows it to specifically bind with two different antigens or epitopes. The conditionally active senolytic or antibody fragment of the present invention may be engineered as the ASTR or portion thereof, in order to render the CARs more active in an extracellular environment of a senescent cell. Such CARs can preferentially deliver the T cells to the senescent cells thus dramatically reducing side-effects caused by T cells attacks on normal tissue. This allows higher doses of T cells to be used to increase therapeutic efficacy and improves the tolerance of a subject to the treatment.

The ASTR may comprise a conditionally active senolytic antibody or antibody fragment, such as antibody, especially a single-chain antibody, or a fragment thereof that binds specifically to an antigen on senescent cells. Some examples of the proteins suitable for ASTRs include linked cytokines (which leads to recognition of cells bearing the cytokine receptor), affibodies, ligand binding domains from naturally occurring receptors, and soluble protein/peptide ligands for a receptor on a senescent cell.

In some embodiments, the CAR includes at least two ASTRs which target at least two different antigens or two epitopes on the same antigen. In one embodiment, the CAR includes three or more ASTRs which target at least three or more different antigens or epitopes. When a plurality of ASTRs is present in the CAR, the ASTRs may be arranged in tandem and may be separated by linker peptides (FIG. 3).

In yet another embodiment, an ASTR includes a diabody. In a diabody, the scFvs are created with linker peptides that are too short for the two variable regions to fold together, driving the scFvs to dimerize. Still shorter linkers (one or two amino acids) lead to the formation of trimers, the so-called triabodies or tribodies. Tetrabodies may also be used in the ASTR.

Target antigens include surface proteins found on senescent cells such as the surface proteins discussed above.

In some embodiments, the extracellular spacer domain and the transmembrane domain may be ubiquitylation-resistant, which can enhance CAR-T cell signaling and thus augment their activity (Kunii et la., "Enhanced function of redirected human t cells expressing linker for activation of t cells that is resistant to ubiquitylation," Human Gene Therapy, vol. 24, pp. 27-37, 2013). Within this region, the extracellular spacer domain is outside of the CAR-T cells, and thus is exposed to different conditions and can potentially be made conditionally ubiquitylation-resistant.

The conditionally active senolytic antibodies or antibody fragments may be included in pharmaceutical compositions, medical devices, kits, or articles of manufacture for human pharmaceutical or diagnostic use, as described in detail in WO 2016/138071.

The conditionally active senolytic antibodies or antibody fragments and the pharmaceutical composition may be used to treat senescent cell-associated diseases and disorders, which include age-related diseases and disorders, in a subject in need thereof. Examples of senescent cell-associated conditions, disorders, or diseases that may be treated by administering the conditionally active senolytic antibody or antibody fragment or pharmaceutical composition described herein include, cognitive diseases (e.g., mild cognitive impairment (MCI), Alzheimer's disease and other dementias; Huntington's disease); cardiovascular disease (e.g., atherosclerosis, cardiac diastolic dysfunction, aortic aneurysm, angina, arrhythmia, cardiomyopathy, congestive heart failure, coronary artery disease, myocardial infarction, endocarditis, hypertension, carotid artery disease, peripheral vascular diseases, cardiac stress resistance, cardiac fibrosis); metabolic diseases and disorders (e.g., obesity, diabetes, metabolic syndrome); neurological diseases and disorders including neurodegenerative diseases and disorders (e.g., Parkinson's disease, motor neuron dysfunction (MND)); cerebrovascular disease; emphysema; benign prostatic hypertrophy; pulmonary diseases (e.g., idiopathic pulmonary fibrosis, chronic obstructive pulmonary disease (COPD), emphysema, obstructive bronchiolitis, asthma); pulmonary insufficiency; inflammatory/autoimmune diseases and disorders (e.g., osteoarthritis, eczema, psoriasis, osteoporosis, mucositis, transplantation related diseases and disorders); ophthalmic diseases or disorders (e.g., age-related macular degeneration, cataracts, glaucoma, vision loss, presbyopia); diabetic ulcer; metastasis; chemotherapeutic side effects, radiotherapy side effects; aging-related diseases and disorders (e.g., kyphosis, renal failure or dysfunction, frailty, hair loss, hearing loss, muscle fatigue, skin conditions, sarcopenia, and herniated intervertebral disc) and other age-related diseases that are induced by senescence (e.g., diseases/disorders resulting from irradiation, chemical exposure, smoking tobacco, eating a high fat/high sugar diet, and environmental factors); wound healing; skin nevi; and fibrotic diseases and disorders (e.g., cystic fibrosis, renal fibrosis, liver fibrosis, pulmonary fibrosis, oral submucous fibrosis, cardiac fibrosis, and pancreatic fibrosis).

In a more specific embodiment, methods are provided for treating a senescent cell-associated disease or disorder by killing or removing senescent cells (i.e., established senescent cells) associated with the disease or disorder in a subject who has the disease or disorder by administering the conditionally active senolytic antibody or antibody fragment or pharmaceutical composition. In certain exemplary embodiments, the present invention is used to treat osteoarthritis; idiopathic pulmonary fibrosis; chronic obstructive pulmonary disease (COPD); or atherosclerosis.

Subjects (i.e., patients, individuals (human or non-human animals)) who may benefit from use of the methods described herein that comprise administering the conditionally active senolytic antibody or antibody fragment or pharmaceutical composition include those who may also have cancer. The subject treated by these methods may be considered to be in partial or complete remission (also called cancer remission). As discussed in detail herein, the conditionally active senolytic antibody or antibody fragment or pharmaceutical composition for use in methods for selective killing or removal of senescent cells are not intended to be used as a treatment for cancer, that is, in a manner that kills or destroys the cancer cells in a statistically significant manner. Therefore, the methods disclosed herein do not encompass use of the conditionally active senolytic antibody or antibody fragment or pharmaceutical composition in a manner that would be considered a primary therapy for the treatment of a cancer. Even though the conditionally active senolytic antibody or antibody fragment, alone or with other chemotherapeutic or radiotherapy agents, are not used in a manner that is sufficient to be considered as a primary cancer therapy, the conditionally active senolytic antibody or antibody fragment or pharmaceutical composition described herein may be used in a manner (e.g., a short term course of therapy) that is useful for inhibiting metastases. In certain embodiments, the subject to be treated with the conditionally active senolytic antibody or antibody fragment or pharmaceutical composition does not have a cancer (i.e., the subject has not been diagnosed as having a cancer by a person skilled in the medical art).

### Cardiovascular Diseases and Disorders

The senescent cell-associated disease or disorder treated by the conditionally active senolytic antibody or antibody fragment or pharmaceutical composition may be a cardiovascular disease. The cardiovascular disease may be any one or more of angina, arrhythmia, atherosclerosis, cardiomyopathy, congestive heart failure, coronary artery disease, carotid artery disease, endocarditis, heart attack (coronary thrombosis, myocardial infarction), high blood pressure/hypertension, aortic aneurysm, brain aneurysm, cardiac fibrosis, cardiac diastolic dysfunction, hypercholesterolemia/hyperlipidemia, mitral valve prolapse, peripheral vascular disease (e.g., peripheral artery disease), cardiac stress resistance, and stroke.

In certain embodiments, methods are provided for treating senescence cell-associated cardiovascular disease that is associated with or caused by arteriosclerosis (i.e., hardening of the arteries). The cardiovascular disease may be any one or more of atherosclerosis (e.g., coronary artery disease (CAD) and carotid artery disease); angina, congestive heart failure, and peripheral vascular disease (e.g., peripheral artery disease (PAD)). The methods for treating a cardiovascular disease that is associated with or caused by arteriosclerosis may reduce the likelihood of occurrence of high blood pressure/hypertension, angina, stroke, and heart attack (i.e., coronary thrombosis, myocardial infarction (MI)). In certain embodiments, methods are provided for stabilizing atherosclerotic plaque(s) in a blood vessel (e.g., artery) of a subject, thereby reducing the likelihood of occurrence or delaying the occurrence of a thrombotic event, such as stroke or MI. In certain embodiments, these methods comprising administration of a conditionally active senolytic antibody or antibody fragment reduce (i.e., cause decrease of) the lipid content of an atherosclerotic plaque in a blood vessel (e.g., artery) of the subject and/or increase the fibrous cap thickness (i.e., cause an increase, enhance or promote thickening of the fibrous cap).

In one embodiment, methods are provided for inhibiting the formation of atherosclerotic plaques (or reducing, diminishing, causing decrease in formation of atherosclerotic plaques) by administering the conditionally active senolytic antibody or antibody fragment or pharmaceutical composition. In other embodiments, methods are provided for reducing (decreasing, diminishing) the amount (i.e., level) of plaque. Reduction in the amount of plaque in a blood vessel (e.g., artery) may be determined, for example, by a decrease in surface area of the plaque, or by a decrease in the extent or degree (e.g., percent) of occlusion of a blood vessel (e.g., artery), which can be determined by angiography or other visualizing methods used in the cardiovascular art. Also provided herein are methods for increasing the stability (or improving, promoting, enhancing stability) of atherosclerotic plaques that are present in one or more blood vessels (e.g., one or more arteries) of a subject, which methods comprise administering to the subject the conditionally active senolytic antibody or antibody fragment or pharmaceutical composition.

The effectiveness of the conditionally active antibody or antibody fragment or pharmaceutical composition for treating or preventing (i.e., reducing or decreasing the likelihood of developing or occurrence of) a cardiovascular disease (e.g., atherosclerosis) can readily be determined by a person skilled in the medical and clinical arts. One or any combination of diagnostic methods, including physical examination, assessment and monitoring of clinical symptoms, and performance of analytical tests and methods described herein and practiced in the art (e.g., angiography, electrocardiography, stress test, non-stress test), may be used for monitoring the health status of the subject. The effects of the treatment by the conditionally active senolytic antibody or antibody fragment or pharmaceutical composition can be analyzed using techniques known in the art, such as comparing symptoms of patients suffering from or at risk of cardiovascular disease that have received the treatment with those of patients without such a treatment or with placebo treatment.

### Inflammatory and Autoimmune Diseases and Disorders

In certain embodiments, a senescent cell-associated disease or disorder is an inflammatory disease or disorder, such as by way of non-limiting example, osteoarthritis, that may be treated or prevented (i.e., likelihood of occurrence is reduced) according to the methods described herein that comprise administration of the conditionally active senolytic antibody or antibody fragment or pharmaceutical composition. Other inflammatory or autoimmune diseases or disorders include osteoporosis, psoriasis, oral mucositis, rheumatoid arthritis, inflammatory bowel disease, eczema, kyphosis, herniated intervertebral disc, and the pulmonary diseases, COPD and idiopathic pulmonary fibrosis.

Unexpectedly, by selectively killing senescent cells, the conditionally active senolytic antibody or antibody fragment or pharmaceutical composition reduces the likelihood of occurrence, reduces or inhibits loss or erosion of proteoglycan layers in a joint, reduces inflammation in the affected joint, and promotes (i.e., stimulates, enhances, induces) production of collagen (e.g., type 2 collagen). Removal of senescent cells may cause a reduction in the amount (i.e., level) of inflammatory cytokines, such as IL-6, produced in joint and inflammation is reduced. Methods are provided herein for treating osteoarthritis, by selectively killing or removing senescent cells possibly located in an osteoarthritic joint of a subject, and/or inducing collagen (such as Type 2 collagen) production in the joint of a subject in need thereof by administering at least one conditionally active senolytic antibody or antibody fragment to the subject. The conditionally active senolytic antibody or antibody fragment also may be used for decreasing (inhibiting, reducing) production of metalloproteinase 13 (MMP-13), which degrades collagen in a joint, and for restoring proteoglycan layer or inhibiting loss and/or degradation of the proteoglycan layer. Treatment with the conditionally active senolytic antibody or antibody fragment or pharmaceutical composition may thereby prevent or reduce likelihood of occurrence of, inhibit, or decrease erosion, or slow erosion of the bone. As described in detail herein, in certain embodiments, the conditionally active senolytic antibody or antibody fragment or pharmaceutical composition is administered directly to an osteoarthritic joint (e.g., by intra-articular, topical, transdermal, intradermal, or subcutaneous delivery). Treatment with the conditionally active senolytic antibody or antibody fragment or pharmaceutical composition can also restore, improve, or inhibit deterioration of strength of a joint. In addition, the methods comprising administering the conditionally active senolytic antibody or antibody fragment or pharmaceutical composition can reduce joint pain and are therefore useful for pain management of osteoarthritic joints.

The effectiveness of one or more conditionally active senolytic antibodies or antibody fragments for treatment or prophylaxis of osteoarthritis in a subject and monitoring of a subject who receives one or more senolytic agents can readily be determined by a person skilled in the medical and clinical arts. One or any combination of diagnostic methods, including physical examination (such as determining tenderness, swelling or redness of the affected joint), assessment and monitoring of clinical symptoms (such as pain, stiffness, mobility), and performance of analytical tests and methods described herein and practiced in the art (e.g., determining the level of inflammatory cytokines or chemokines; X-ray images to determine loss of cartilage as shown by a narrowing of space between the bones in a joint; magnetic resonance imaging (MRI), providing detailed images of bone and soft tissues, including cartilage), may be used for monitoring the health status of the subject. The effects of the treatment of one or more senolytic agents can be analyzed by comparing symptoms of patients suffering from or at risk of an inflammatory disease or disorder, such as osteoarthritis, who have received the treatment with those of patients who have not received such a treatment or who have received a placebo treatment.

In certain embodiments, the conditionally active senolytic antibody or antibody fragment or pharmaceutical composition may be used for treating and/or preventing (i.e., decreasing or reducing the likelihood of occurrence) rheumatoid arthritis (RA).

Chronic inflammation may also contribute to other age-related or aging related diseases and disorders, such as kyphosis and osteoporosis. Kyphosis has been associated with cellular senescence. The capability of a senolytic agent for treating kyphosis may be determined in pre-clinical animal models used in the art. By way of example, TTD mice develop kyphosis (see, e.g., de Boer et al. Science, vol. 296, pp. 1276-1279, 2002); other mice that may be used include BubRl^{H/H} mice, which are also known to develop kyphosis (see, e.g., Baker et al. Nature, vol. 479, pp. 232-36, 2011). Kyphosis formation is visually measured over time. The level of senescent cells decreased by treatment with the senolytic agent can be determined by detecting the presence of one or more senescent cell associated markers such as by SA-P-Gal staining.

In still other embodiments, an inflammatory/autoimmune disorder that may be treated or prevented (i.e., likelihood of occurrence is reduced) with the conditionally active senolytic antibody or antibody fragment or pharmaceutical composition described herein include irritable bowel syndrome (IBS) and inflammatory bowel diseases, such as ulcerative colitis and Crohn's disease. Diagnosis and monitoring of the diseases is performed according to methods and diagnostic tests routinely practiced in the art, including blood tests, colonoscopy, flexible sigmoidoscopy, barium enema, CT scan, MRI, endoscopy, and small intestine imaging.

In other embodiments, the methods described herein may be useful for treating a subject who has herniated intervertebral discs. Subjects with these herniated discs exhibit elevated presence of cell senescence in the blood and in vessel walls (see e.g., Roberts et al. Eur. Spine J., 15 Suppl 3: S312-316, 2006). Increased levels of proinflammatory molecules and matrix metalloproteases are also found in aging and degenerating discs tissues, suggesting a role for senescence cells (see e.g., Chang-Qing et al. Ageing Res. Rev., vol. 6, pp. 247-61, 2007). Animal models may be used to characterize the effectiveness of a senolytic agent in treating herniated intervertebral discs; degeneration of the intervertebral disc is induced in mice by compression and disc strength evaluated (see e.g., Lotz et al. Spine, vol. 23, pp. 2493-506, 1998).

Other inflammatory or autoimmune diseases that may be treated or prevented (i.e., likelihood of occurrence is reduced) by using the conditionally active senolytic antibody or antibody fragment or pharmaceutical composition include eczema, psoriasis, osteoporosis, and pulmonary diseases (e.g., chronic obstructive pulmonary disease (COPD), idiopathic pulmonary fibrosis (IPF), asthma), inflammatory bowel disease, and mucositis (including oral mucositis, which in some instances is induced by radiation). Certain fibrosis or fibrotic conditions of organs such as renal fibrosis, liver fibrosis, pancreatic fibrosis, cardiac fibrosis, skin wound healing, and oral submucous fibrosis may be treated with using the conditionally active senolytic antibody or antibody fragment or pharmaceutical composition.

In certain embodiments, the senescent cell associated disorder is an inflammatory disorder of the skin, such as by way of a non-limiting examples, psoriasis and eczema that may be treated or prevented (i.e., likelihood of occurrence is reduced) according to the methods described herein that comprise administration of the conditionally active senolytic antibody or antibody fragment or pharmaceutical composition. The effectiveness of the conditionally active senolytic antibody or antibody fragment or pharmaceutical composition for treatment of psoriasis and eczema and monitoring of a subject who receives such treatment can be readily determined by a person skilled in the medical or clinical arts. One or any combination of diagnostic methods, including physical examination (such as skin appearance), assessment of and/or monitoring of clinical symptoms (such as itching, swelling, and pain), and performance of analytical tests and methods described herein and practiced in the art (i.e., determining the level of pro-inflammatory cytokines).

### Pulmonary Diseases and Disorders

In one embodiment, methods are provided for treating or preventing (i.e., reducing the likelihood of occurrence of) a senescent cell-associated disease or disorder that is a pulmonary disease or disorder by killing or removing senescent cells (i.e., established senescent cells) associated with the disease or disorder in a subject who has the disease or disorder by administering the conditionally active senolytic antibody or antibody fragment or pharmaceutical composition. Senescence associated pulmonary diseases and disorders include, for example, idiopathic pulmonary fibrosis (IPF), chronic obstructive pulmonary disease (COPD), asthma, cystic fibrosis, bronchiectasis, and emphysema. The involvement of cellular senescence in IPF is suggested by the observations that the incidence of the disease increases with age and that lung tissue in IPF patients is enriched for SA-P-Gal-positive cells and contains elevated levels of the senescence marker p21 (see, e.g., Minagawa et al, Am. J. Physiol. Lung Cell. Mol. Physiol., vol. 300, pp. L391-L401, 2011). Short telomeres are a risk factor common to both IPF and cellular senescence (see, e.g., Alder et al, Proc. Natl. Acad. Sci. USA, vol. 105, pp. 13051-56, 2008). Without wishing to be bound by theory, the contribution of cellular senescence to IPF is suggested by the report that SASP components of senescent cells, such as IL-6, IL-8, and IL-Iβ, promote fibroblast-to-myofibroblast differentiation and epithelial-mesenchymal transition, resulting in extensive remodeling of the extracellular matrix of the alveolar and interstitial spaces (see, e.g., Minagawa et al, *supra).*

Other pulmonary diseases or disorders that may be treated by using the conditionally active senolytic antibody or antibody fragment or pharmaceutical composition include, for example, emphysema, asthma, bronchiectasis, and cystic fibrosis (see, e.g., Fischer et al, Am J Physiol Lung Cell Mol Physiol., vol. 304, pp. L394-400, 2013).

The methods described herein for treating or preventing (i.e., reducing the likelihood of occurrence of) a senescence associate pulmonary disease or disorder may also be used for treating a subject who is aging and has loss (or degeneration) of pulmonary function (i.e., declining or impaired pulmonary function compared with a younger subject) and/or degeneration of pulmonary tissue. By administering a senolytic agent to an aging subject (which includes a middle-aged adult who is asymptomatic), the decline in pulmonary function may be decelerated or inhibited by killing and removing senescent cells from the respiratory tract. The effects of the treatment by the conditionally active senolytic antibody or antibody fragment or pharmaceutical composition can be analyzed using techniques known in the art, such as comparing symptoms of patients suffering from or at risk of the pulmonary disease that have received the treatment with those of patients without such a treatment or with placebo treatment. In addition, methods and techniques that evaluate mechanical functioning of the lung, for example, techniques that measure lung capacitance, elastance, and airway hypersensitivity may be performed. To determine lung function and to monitor lung function throughout treatment, any one of numerous measurements may be obtained, expiratory reserve volume (ERV), forced vital capacity (FVC), forced expiratory volume (FEV) (e.g., FEV in one second, FEV1), FEV1/FEV ratio, forced expiratory flow 25% to 75%, and maximum voluntary ventilation (MVV), peak expiratory flow (PEF), slow vital capacity (SVC). Total lung volumes include total lung capacity (TLC), vital capacity (VC), residual volume (RV), and functional residual capacity (FRC). Gas exchange across alveolar capillary membrane can be measured using diffusion capacity for carbon monoxide (DLCO). Peripheral capillary oxygen saturation (SpO₂) can also be measured.

### Neurological Diseases and Disorders

Senescent cell-associated diseases or disorders treatable by administering the conditionally active senolytic antibody or antibody fragment or pharmaceutical composition include neurological diseases or disorders. Such senescent cell-associated diseases and disorders include Parkinson's disease, Alzheimer's disease (and other dementias), motor neuron dysfunction (MND), mild cognitive impairment (MCI), Huntington's disease, and diseases and disorders of the eyes, such as age-related macular degeneration. Other diseases of the eye that are associated with increasing age are glaucoma, vision loss, presbyopia, and cataracts.

Senescence of dopamine-producing neurons is thought to contribute to the observed cell death in PD through the production of reactive oxygen species (see, e.g., Cohen et al, J. Neural Transm. Suppl. 19:89-103 (1983)); therefore, the conditionally active senolytic antibody or antibody fragment and pharmaceutical composition described herein are useful for treatment and prophylaxis of Parkinson's disease.

Methods for detecting, monitoring or quantifying neurodegenerative deficiencies and/or locomotor deficits associated with Parkinson's diseases are known in the art, such as histological studies, biochemical studies, and behavioral assessment (see, e.g., U.S. 2012/0005765). Symptoms of Parkinson's disease are known in the art and include, but are not limited to, difficulty starting or finishing voluntary movements, jerky, stiff movements, muscle atrophy, shaking (tremors), and changes in heart rate, but normal reflexes, bradykinesia, and postural instability.

The effectiveness of the conditionally active senolytic antibody or antibody fragment or pharmaceutical composition described herein in a subject who receives one or more senolytic agents can readily be determined by a person skilled in the medical and clinical arts. One or any combination of diagnostic methods, including physical examination, assessment and monitoring of clinical symptoms, and performance of analytical tests and methods described herein, may be used for monitoring the health status of the subject. The effects of administering the conditionally active senolytic antibody or antibody fragment or pharmaceutical composition can be analyzed using techniques known in the art, such as comparing symptoms of patients suffering from or at risk of Alzheimer's disease that have received the treatment with those of patients without such a treatment or with placebo treatment.

### Mild Cognitive Impairment (MCI)

MCI is a brain-function syndrome involving the onset and evolution of cognitive impairment beyond those expected based on age and education of the individual, but which are not significant enough to interfere with this individual's daily activities. Administration of the conditionally active senolytic antibody or antibody fragment may reduce or inhibit MCI by killing or removing senescent cells. Methods for detecting, monitoring, quantifying or assessing neuropathological deficiencies associated with MCI are known in the art, including astrocyte morphological analyses, release of acetylcholine, silver staining for assessing neurodegeneration, and PiB PET imaging to detect beta amyloid deposits (see, e.g., U.S. 2012/0071468). Methods for detecting, monitoring, quantifying or assessing behavioral deficiencies associated with MCI are also known in the art, including eight-arm radial maze paradigm, non-matching-to-sample task, allocentric place determination task in a water maze, Morris maze test, visuospatial tasks, and delayed response spatial memory task, olfactory novelty test (see, *id.).*

### Motor Neuron Dysfunction (MND)

MND is a group of progressive neurological disorders that destroy motor neurons, the cells that control essential voluntary muscle activity such as speaking, walking, breathing and swallowing. Examples of MNDs include, but are not limited to Amyotrophic Lateral Sclerosis (ALS), also known as Lou Gehrig's Disease, progressive bulbar palsy, pseudobulbar palsy, primary lateral sclerosis, progressive muscular atrophy, lower motor neuron disease, and spinal muscular atrophy (SMA) (e.g., SMA1 also called Werdnig-Hoffmann Disease, SMA2, SMA3 also called Kugelberg-Welander Disease, and Kennedy's disease), post-polio syndrome, and hereditary spastic paraplegia. Administration of the conditionally active senolytic antibody or antibody fragment may reduce or inhibit MNDs by killing or removing senescent cells. Methods for detecting, monitoring or quantifying locomotor and/or other deficits associated with Parkinson's diseases, such as MND, are known in the art (see, e.g., U.S. 20120005765). Methods for detecting, monitoring, quantifying or assessing motor deficits and histopathological deficiencies associated with MND are known in the art, including histopathological, biochemical, and electrophysiological studies and motor activity analysis (see, e.g., Rich et al., J Neurophysiol, vol. 88, pp. 3293-3304, 2002; Appel et al, Proc. Natl. Acad. Sci. USA, vol. 88, pp. 647-51, 1991).

### Ophthalmic Diseases and Disorders

In certain embodiments, a senescent cell-associated disease or disorder is an ocular disease, disorder, or condition, for example, presbyopia, macular degeneration, or cataracts. In other certain embodiments, the senescent cell-associated disease or disorder is glaucoma. Macular degeneration is a neurodegenerative disease that causes the loss of photoreceptor cells in the central part of retina, called the macula. While the exact causes of age-related macular degeneration are still unknown, the number of senescent retinal pigmented epithelial (RPE) cells increases with age. Age and certain genetic factors and environmental factors are risk factors for developing ARMD (see, e.g., Lyengar et al, Am. J. Hum. Genet., vol. 74, pp. 20-39, 2004; Kenealy et al, Mol. Vis., vol. 10, pp. 57-61, 2004; Gorin et al, Mol. Vis., vol. 5, p. 29, 1999). Decreased micro RNAs contribute to a senescent cell profile; and DICERI ablation induces premature senescence. Diagnosing and monitoring of a subject with macular degeneration may be accomplished by a person skilled in the ophthalmic art according to art-accepted periodic eye examination procedures and report of symptoms by the subject.

Age-related changes in the mechanical properties of the anterior lens capsule and posterior lens capsule suggest that the mechanical strength of the posterior lens capsule decreases significantly with age (see, e.g., Krag et al, Invest. Ophthalmol. Vis. Sci., vol. 44, pp. 691-96, 2003; Krag et al, Invest. Ophthalmol. Vis. Sci., vol. 38, pp. 357-63, 1997). The laminated structure of the capsule also changes and may result, at least in part, from a change in the composition of the tissue.

Research has suggested that collagen IV influences cellular function which is inferred from the positioning of basement membranes underneath epithelial layers, and data support the role of collagen IV in tissue stabilization. Posterior capsule opacification (PCO) develops as a complication in approximately 20-40% of patients in subsequent years after cataract surgery (see, e.g., Awasthi et al, Arch Ophthalmol., vol. 127, pp. 555-62, 2009). PCO results from proliferation and activity of residual lens epithelial cells along the posterior capsule in a response akin to wound healing. Growth factors, such as fibroblast growth factor, transforming growth factor β, epidermal growth factor, hepatocyte growth factor, insulin-like growth factor, and interleukins IL-1 and IL-6 may also promote epithelial cell migration. As discussed herein, production of these factors and cytokines by senescent cells contribute to the SASP. In contrast, *in vitro* studies show that collagen IV promotes adherence of lens epithelial cells (see, e.g., Olivero et al, Invest. Ophthalmol. Vis. Sci., vol. 34, pp. 2825-34, 1993). Adhesion of the collagen IV, fibronectin, and laminin to the intraocular lens inhibits cell migration and may reduce the risk of PCO (see, e.g., Raj et al, Int. J. Biomed. Sci., vol. 3, pp. 237-50, 2007).

Without wishing to be bound by any particular theory, selective killing or removal of senescent cells by the conditionally active senolytic antibody or antibody fragment described herein may slow or impede (delay, inhibit, retard) the disorganization of the type IV collagen network. Removal of senescence cells and thereby removing the inflammatory effects of SASP may decrease or inhibit epithelial cell migration and may also delay (suppress) the onset of presbyopia or decrease or slow the progressive severity of the condition (such as slow the advancement from mild to moderate or moderate to severe). The conditionally active senolytic antibody or antibody fragment and pharmaceutical composition described herein may also be useful for post-cataract surgery to reduce the likelihood of occurrence of PCO.

BubR1 hypomorphic mice develop posterior subcapsular cataracts bilaterally early in life, suggesting that senescence may play a role (see, e.g., Baker et al, Nat. Cell Biol., vol. 10, pp. 825-36, 2008). The presence and severity of a cataract can be monitored by eye exams using methods routinely performed by a person skilled in the ophthalmology art.

In certain embodiments, at least one conditionally active senolytic antibody or antibody fragment that selectively kills senescent cells may be administered to a subject who is at risk of developing presbyopia, cataracts, or macular degeneration. Treatment with the conditionally active senolytic antibody or antibody fragment may be initiated when a human subject is at least 40 years of age to delay or inhibit onset or development of cataracts, presbyopia, and macular degeneration. Because almost all humans develop presbyopia, in certain embodiments, the senolytic agent may be administered in a manner as described herein to a human subject after the subject reaches the age of 40 to delay or inhibit onset or development of presbyopia.

In certain embodiments, the senescence associated disease or disorder is glaucoma. Glaucoma is a broad term used to describe a group of diseases that causes visual field loss, often without any other prevailing symptoms. When the cellular network required for the outflow of fluid was subjected to SA-P-Gal staining, a fourfold increase in senescence has been observed in glaucoma patients (see, e.g., Liton et al, Exp. Gerontol., vol. 40, pp. 745-748, 2005).

For monitoring the effect of a therapy on inhibiting progression of glaucoma, standard automated perimetry (visual field test) is the most widely used technique. In addition, several algorithms for progression detection have been developed (see, e.g., Wesselink et al, Arch Ophthalmol., vol. 127, pp. 270-274, 2009, and references therein). Additional methods include gonioscopy (examines the trabecular meshwork and the angle where fluid drains out of the eye); imaging technology, for example scanning laser tomography (e.g., HRT3), laser polarimetry (e.g., GDX), and ocular coherence tomography); ophthalmoscopy; and pachymeter measurements that determine central corneal thickness.

### Metabolic Disease or Disorder

Senescent cell-associated diseases or disorders treatable by administering the conditionally active senolytic antibody or antibody fragment or pharmaceutical composition include metabolic diseases or disorders. Such senescent cell associated diseases and disorders include diabetes, metabolic syndrome, diabetic ulcers, and obesity. The conditionally active senolytic antibodies or antibody fragments described herein may be used for treating type 2 diabetes, particularly age-, diet- and obesity-associated type 2 diabetes.

Involvement of senescent cells in metabolic disease, such as obesity and type 2 diabetes, has been suggested as a response to injury or metabolic dysfunction (see, e.g., Tchkonia et al, Aging Cell, vol. 9, pp. 667-684, 2010). Fat tissue from obese mice showed induction of the senescence markers SA-P-Gal, p53, and p21 (see, e.g., Minamino et al, Nat. Med., vol. 15, pp. 1082-1087, 2009). A concomitant up-regulation of pro-inflammatory cytokines, such as tumor necrosis factor-alpha and Ccl2/MCPl, was observed in the same fat tissue (see, e.g., Minamino et al., supra). Induction of senescent cells in obesity potentially has clinical implications because pro-inflammatory SASP components are also suggested to contribute to type 2 diabetes (see, e.g., Tchkonia et al, supra). A similar pattern of up-regulation of senescence markers and SASP components are associated with diabetes, both in mice and in humans (see, e.g., Minamino et al, supra). Accordingly, the methods described herein that comprise administering a senolytic agent may be useful for treatment or prophylaxis of type 2 diabetes, as well as obesity and metabolic syndrome. Without wishing to be bound by theory, contact of senescent pre-adipocytes with a senolytic agent thereby killing the senescent pre-adipocytes may provide clinical and health benefit to a person who has any one of diabetes, obesity, or metabolic syndrome.

A condition or disorder associated with diabetes and senescence is a diabetic ulcer (i.e., diabetic wound). An ulcer is a breakdown in the skin, which may extend to involve the subcutaneous tissue or even muscle or bone. These lesions occur, particularly, on the lower extremities. Patients with diabetic venous ulcer exhibit elevated presence of cellular senescence at sites of chronic wounds (see, e.g., Stanley et al. J. Vas. Surg., vol. 33, pp. 1206-1211, 2001). Chronic inflammation is also observed at sites of chronic wounds, such as diabetic ulcers (see, e.g., Goren et al. Am. J. Pathol., vol. 168, pp. 65-77), suggesting that the proinflammatory cytokine phenotype of senescent cells has a role in the pathology.

The effectiveness of the conditionally active senolytic antibody or antibody fragment can readily be determined by a person skilled in the medical and clinical arts. One or any combination of diagnostic methods, including physical examination, assessment and monitoring of clinical symptoms, and performance of analytical tests and methods, such as those described herein, may be used for monitoring the health status of the subject. A subject who is receiving one or more senolytic agents described herein for treatment or prophylaxis of diabetes can be monitored, for example, by assaying glucose and insulin tolerance, energy expenditure, body composition, fat tissue, skeletal muscle, and liver inflammation, and/or lipotoxicity (muscle and liver lipid by imaging in vivo and muscle, liver, bone marrow, and pancreatic β-cell lipid accumulation and inflammation by histology). Other characteristic features or phenotypes of type 2 diabetes are known and can be assayed as described herein and by using other methods and techniques known and routinely practiced in the art.

Subjects who have type 2 diabetes or who are at risk of developing type 2 diabetes may have metabolic syndrome. Metabolic syndrome in humans is typically associated with obesity and characterized by one or more of cardiovascular disease, liver steatosis, hyperlipidemia, diabetes, and insulin resistance. A subject with metabolic syndrome may present with a cluster of metabolic disorders or abnormalities which may include, for example, one or more of hypertension, type-2 diabetes, hyperlipidemia, dyslipidemia (e.g., hypertriglyceridemia, hypercholesterolemia), insulin resistance, liver steatosis (steatohepatitis), hypertension, atherosclerosis, and other metabolic disorders.

### Dermatological Disease or Disorder

Senescent cell-associated diseases or disorders treatable by administering the conditionally active senolytic antibody or antibody fragment or pharmaceutical composition described herein include dermatological diseases or disorders. Such senescent cell associated diseases and disorders include psoriasis and eczema, which are also inflammatory diseases and are discussed in greater detail above. Other dermatological diseases and disorders that are associated with senescence include rhytids (wrinkles due to aging); pruritus (linked to diabetes and aging); dysesthesia (chemotherapy side effect that is linked to diabetes and multiple sclerosis); psoriasis (as noted) and other papulosquamous disorders, for example, erythroderma, lichen planus, and lichenoid dermatosis; atopic dermatitis (a form of eczema and associated with inflammation); eczematous eruptions (often observed in aging patients and linked to side effects of certain drugs). Other dermatological diseases and disorders associated with senescence include eosinophilic dermatosis (linked to certain kinds of hematologic cancers); reactive neutrophilic dermatosis (associated with underlying diseases such as inflammatory bowel syndrome); pemphigus (an autoimmune disease in which autoantibodies form against desmoglein); pemphigoid and other immunobullous dermatosis (autoimmune blistering of skin); fibrohistiocytic proliferations of skin, which is linked to aging; and cutaneous lymphomas that are more common in older populations. Another dermatological disease that may be treatable according to the methods described herein includes cutaneous lupus, which is a symptom of lupus erythematosus. Late onset lupus may be linked to decreased (i.e., reduced) function of T-cell and B-cells and cytokines (immunosenescence) associated with aging.

### Metastasis

In a particular embodiment, the conditionally active senolytic antibody or antibody fragment or pharmaceutical composition can be used for treatment or prevention of metastasis (i.e., the spreading and dissemination of cancer or tumor cells) from one organ or tissue to another organ or tissue in the body. A subject who has a cancer may benefit from administration of the conditionally active senolytic antibody or antibody fragment or pharmaceutical composition for inhibiting metastasis. Such the conditionally active senolytic antibody or antibody fragment or pharmaceutical composition may inhibit tumor proliferation. Metastasis of a cancer occurs when the cancer cells (i.e., tumor cells) spread beyond the anatomical site of origin and initial colonization to other areas throughout the body of the subject. Tumor proliferation may be determined by tumor size, which can be measured in various ways familiar to a person skilled in the art, such as by PET scanning, MRI, CAT scan, biopsy, for example. The effect of the therapeutic agent on tumor proliferation may also be evaluated by examining differentiation of the tumor cells.

As used herein and in the art, the terms cancer or tumor are clinically descriptive terms that encompass diseases typically characterized by cells exhibiting abnormal cellular proliferation. The term cancer is generally used to describe a malignant tumor or the disease state arising from the tumor. Alternatively, an abnormal growth may be referred to in the art as a neoplasm. The term tumor, such as in reference to a tissue, generally refers to any abnormal tissue growth that is characterized, at least in part, by excessive and abnormal cellular proliferation. A tumor may be metastatic and capable of spreading beyond its anatomical site of origin and initial colonization to other areas throughout the body of the subject. A cancer may comprise a solid tumor or may comprise a "liquid" tumor (e.g., leukemia and other blood cancers).

Cells are induced to senesce by cancer therapies, such as radiation and certain chemotherapy drugs. The presence of senescent cells increases secretion of inflammatory molecules (see description herein of senescent cells), promotes tumor progression, which may include promoting tumor growth and increasing tumor size, promoting metastasis, and altering differentiation. When senescent cells are destroyed, tumor progression is significantly inhibited, resulting in tumors of small size and with little or no observed metastatic growth (see, e.g., WO 2013/090645). Thus, the conditionally active senolytic antibody or antibody fragment or pharmaceutical composition may be administered after the chemotherapy or radiotherapy to kill or remove these senescent cells. As discussed herein and understood in the art, establishment of senescence, such as shown by the presence of a senescent cell-associated secretory phenotype (SASP), occurs over several days; therefore, administering a senolytic agent to kill senescent cells, and thereby reduce the likelihood of occurrence or reduce the extent of metastasis, is initiated when senescence has been established.

In a certain particular embodiment when chemotherapy or radiotherapy is administered in a treatment cycle of at least one day on-therapy (i.e., chemotherapy or radiotherapy)) followed by at least one week off-therapy, the conditionally active senolytic antibody or antibody fragment or pharmaceutical composition is administered on one or more days during the off-therapy time interval beginning on or after the second day of the off-therapy time interval and ending on or before the last day of the off-therapy time interval. In a more specific embodiment, when chemotherapy or radiotherapy is administered in a treatment cycle of at least one day on-therapy (i.e., chemotherapy or radiotherapy)) followed by at least one week off-therapy, the conditionally active senolytic antibody or antibody fragment or pharmaceutical composition is administered on one day that is the sixth day of the off-therapy time interval. In other specific embodiments, when chemotherapy or radiotherapy is administered in a treatment cycle of at least one day on-therapy (i.e., chemotherapy or radiotherapy)) followed by at least two weeks off-therapy, the conditionally active senolytic antibody or antibody fragment or pharmaceutical composition is administered beginning on the sixth day of the off-chemo- or radio-therapy time interval and ending at least one day or at least two days prior to the first day of a subsequent chemotherapy or radiation therapy treatment course.

In another embodiment for treating metastasis, the conditionally active senolytic antibody or antibody fragment or pharmaceutical composition may be administered after the treatment regimen of chemotherapy or radiotherapy has been completed. In a particular embodiment, the conditionally active senolytic antibody or antibody fragment or pharmaceutical composition is administered after the chemotherapy or radiotherapy has been completed on one or more days within treatment window (i.e., senolytic agent treatment course) of no longer than 14 days.

The methods described herein are also useful for inhibiting, retarding or slowing progression of metastatic cancer of any one of the types of tumors described in the medical art. Types of cancers (tumors) include the following: adrenocortical carcinoma, childhood adrenocortical carcinoma, aids-related cancers, anal cancer, appendix cancer, basal cell carcinoma, childhood basal cell carcinoma, bladder cancer, childhood bladder cancer, bone cancer, brain tumor, childhood astrocytomas, childhood brain stem glioma, childhood central nervous system atypical teratoid/rhabdoid tumor, childhood central nervous system embryonal tumors, childhood central nervous system germ cell tumors, childhood craniopharyngioma brain tumor, childhood ependymoma brain tumor, breast cancer, childhood bronchial tumors, carcinoid tumor, childhood carcinoid tumor, gastrointestinal carcinoid tumor, carcinoma of unknown primary, childhood carcinoma of unknown primary, childhood cardiac (heart) tumors, cervical cancer, childhood cervical cancer, childhood chordoma , chronic myeloproliferative disorders, colon cancer, colorectal cancer, childhood colorectal cancer, extrahepatic bile duct cancer , ductal carcinoma in situ (DCIS), endometrial cancer, esophageal cancer, childhood esophageal cancer, childhood esthesioneuroblastoma, eye cancer, malignant fibrous histiocytoma of bone, gallbladder cancer, gastric (stomach) cancer, childhood gastric (stomach) cancer, gastrointestinal stromal tumors (GIST), childhood gastrointestinal stromal tumors (GIST), childhood extracranial germ cell tumor, extragonadal germ cell tumor, gestational trophoblastic tumor, glioma, head and neck cancer, childhood head and neck cancer, hepatocellular (liver) cancer, hypopharyngeal cancer, kidney cancer, renal cell kidney cancer, Wilms tumor, childhood kidney tumors, Langerhans cell histiocytosis, laryngeal cancer, childhood laryngeal cancer, leukemia, acute lymphoblastic leukemia (ALL), acute myeloid leukemia (AML), chronic lymphocytic leukemia (CLL), chronic myelogenous leukemia (cml), hairy cell leukemia, lip cancer, liver cancer (primary), childhood liver cancer (primary), lobular carcinoma in situ (LCIS), lung cancer, non-small cell lung cancer, small cell lung cancer, lymphoma, aids-related lymphoma, Burkitt lymphoma, cutaneous t-cell lymphoma, Hodgkin lymphoma, non-Hodgkin lymphoma, primary central nervous system lymphoma (CNS), melanoma, childhood melanoma, intraocular (eye) melanoma, Merkel cell carcinoma, malignant mesothelioma, childhood malignant mesothelioma, metastatic squamous neck cancer with occult primary, midline tract carcinoma involving NUT gene, mouth cancer, childhood multiple endocrine neoplasia syndromes, mycosis fungoides, myelodysplasia syndromes, myelodysplasia neoplasms, myeloproliferative neoplasms, multiple myeloma, nasal cavity cancer, nasopharyngeal cancer, childhood nasopharyngeal cancer, neuroblastoma, oral cancer, childhood oral cancer, oropharyngeal cancer, ovarian cancer, childhood ovarian cancer, epithelial ovarian cancer, low malignant potential tumor ovarian cancer, pancreatic cancer, childhood pancreatic cancer, pancreatic neuroendocrine tumors (islet cell tumors) , childhood papillomatosis , paraganglioma, paranasal sinus cancer, parathyroid cancer, penile cancer, pharyngeal cancer, pheochromocytoma, pituitary tumor, plasma cell neoplasm , childhood pleuropulmonary blastoma, prostate cancer, rectal cancer, renal pelvis transitional cell cancer, retinoblastoma, salivary gland cancer, childhood salivary gland cancer, Ewing sarcoma family of tumors, Kaposi Sarcoma, osteosarcoma, rhabdomyosarcoma, childhood rhabdomyosarcoma, soft tissue sarcoma, uterine sarcoma, Sezary syndrome, childhood skin cancer, nonmelanoma skin cancer, small intestine cancer, squamous cell carcinoma, childhood squamous cell carcinoma, testicular cancer, childhood testicular cancer, throat cancer, thymoma and thymic carcinoma, childhood thymoma and thymic carcinoma, thyroid cancer, childhood thyroid cancer, ureter transitional cell cancer, urethral cancer, endometrial uterine cancer, vaginal cancer, vulvar cancer, Waldenstrom macroglobulinemia.

### Chemotherapy and Radiotherapy Side Effects

In another embodiment, the senescence cell associated disorder or condition is a chemotherapeutic side effect or a radiotherapy side effect. Examples of chemotherapeutic agents that induce non-cancer cells to senesce include anthracyclines (such as doxorubicin, daunorubicin); taxols (e.g., paclitaxel); gemcitabine; pomalidomide; and lenalidomide. One or more of the senolytic agents administered as described herein may be used for treating and/or preventing {i.e., reducing the likelihood of occurrence of) a chemotherapeutic side effect or a radiotherapy side effect. Removal or destruction of senescent cells may ameliorate acute toxicity, including acute toxicity comprising energy imbalance, of a chemotherapy or radiotherapy. Acute toxic side effects include but are not limited to gastrointestinal toxicity (e.g., nausea, vomiting, constipation, anorexia, diarrhea), peripheral neuropathy, fatigue, malaise, low physical activity, hematological toxicity (e.g., anemia), hepatotoxicity, alopecia (hair loss), pain, infection, mucositis, fluid retention, dermatological toxicity (e.g., rashes, dermatitis, hyperpigmentation, urticaria, photosensitivity, nail changes), mouth (e.g., oral mucositis), gum or throat problems, or any toxic side effect caused by a chemotherapy or radiotherapy. For example, toxic side effects caused by radiotherapy or chemotherapy (see, e.g., National Cancer Institute web site) may be ameliorated by the methods described herein. Accordingly, in certain embodiments, methods are provided herein for ameliorating (reducing, inhibiting, or preventing occurrence (i.e., reducing the likelihood of occurrence)) acute toxicity or reducing severity of a toxic side effect (i.e., deleterious side effect) of a chemotherapy or radiotherapy or both in a subject who receives the therapy, wherein the method comprises administering to the subject an agent that selectively kills, removes, or destroys or facilitates selective destruction of senescent cells.

Administration of the conditionally active senolytic antibody or antibody fragment or pharmaceutical composition for treating or reducing the likelihood of occurrence, or reducing the severity of a chemotherapy or radiotherapy side effect may be accomplished by the same treatment courses described above for treatment/prevention of metastasis. As described for treating or preventing (i.e., reducing the likelihood of occurrence of) metastasis, the conditionally active senolytic antibody or antibody fragment or pharmaceutical composition is administered during the off-chemotherapy or off-radiotherapy time interval or after the chemotherapy or radiotherapy treatment regimen has been completed.

In a more specific embodiment, the acute toxicity is an acute toxicity comprising energy imbalance and may comprise one or more of weight loss, endocrine change(s) (e.g., hormone imbalance, change in hormone signaling), and change(s) in body composition. In certain embodiments, an acute toxicity comprising energy imbalance relates to decreased or reduced ability of the subject to be physically active, as indicated by decreased or diminished expenditure of energy than would be observed in a subject who did not receive the medical therapy. By way of non-limiting example, such an acute toxic effect that comprises energy imbalance includes low physical activity. In other particular embodiments, energy imbalance comprises fatigue or malaise.

In one embodiment, a chemotherapy side effect to be treated or prevented (i.e., likelihood of occurrence is reduced) by the conditionally active senolytic antibody or antibody fragment or pharmaceutical composition is cardiotoxicity. A subject who has a cancer that is being treated with an anthracycline (such as doxorubicin, daunorubicin) may be treated with one or more senolytic agents described herein that reduce, ameliorate, or decrease the cardiotoxicity of the anthracycline. As is well understood in the medical art, because of the cardiotoxicity associated with anthracyclines, the maximum lifetime dose that a subject can receive is limited even if the cancer is responsive to the drug. Administration of one or more of the conditionally active senolytic antibodies or antibody fragments may reduce the cardiotoxicity such that additional amounts of the anthracycline can be administered to the subject, resulting in an improved prognosis related to cancer disease. In one embodiment, the cardiotoxicity results from administration of an anthracyline, such as doxorubicin. Doxorubicin is an anthracycline topoisomerase that is approved for treating patients who have ovarian cancer after failure of a platinum based therapy; Kaposi's sarcoma after failure of primary systemic chemotherapy or intolerance to the therapy; or multiple myeloma in combination with bortezomib in patients who have not previously received bortezomib or who have received at least one prior therapy. Doxorubicin may cause myocardial damage that could lead to congestive heart failure if the total lifetime dose to a patient exceeds 550 mg/m². Cardiotoxicity may occur at even lower doses if the patient also receives mediastinal irradiation or another cardiotoxic drug. See drug product inserts (e.g., doxil, adriamycin).

In other embodiments, the conditionally active senolytic antibody or antibody fragment or pharmaceutical composition described herein may be used in the methods as provided herein for ameliorating chronic or long term side effects. Chronic toxic side effects typically result from multiple exposures to or administrations of a chemotherapy or radiotherapy over a longer period of time. Certain toxic effects appear long after treatment (also called late toxic effects) and result from damage to an organ or system by the therapy. Organ dysfunction (e.g., neurological, pulmonary, cardiovascular, and endocrine dysfunction) has been observed in patients who were treated for cancers during childhood (see, e.g., Hudson et al, JAMA, vol. 309, pp. 2371-81, 2013). Without wishing to be bound by any particular theory, by destroying senescent cells, particular normal cells that have been induced to senescence by chemotherapy or radiotherapy, the likelihood of occurrence of a chronic side effect may be reduced, or the severity of a chronic side effect may be reduced or diminished, or the time of onset of a chronic side effect may be delayed. Chronic and/or late toxic side effects that occur in subjects who received chemotherapy or radiation therapy include by way of non-limiting example, cardiomyopathy, congestive heart disease, inflammation, early menopause, osteoporosis, infertility, impaired cognitive function, peripheral neuropathy, secondary cancers, cataracts and other vision problems, hearing loss, chronic fatigue, reduced lung capacity, and lung disease.

In addition, by killing or removing senescent cells in a subject who has a cancer by administering the conditionally active senolytic antibody or antibody fragment or pharmaceutical composition, the sensitivity to the chemotherapy or the radiotherapy may be enhanced in a clinically or statistically significant manner than if the conditionally active senolytic antibody or antibody fragment or pharmaceutical composition was not administered. In other words, development of chemotherapy or radiotherapy resistance may be inhibited when the conditionally active senolytic antibody or antibody fragment or pharmaceutical composition is administered to a subject treated with the respective chemotherapy or radiotherapy.

### Age-Related Diseases and Disorders

The conditionally active senolytic antibody or antibody fragment or pharmaceutical composition may also be useful for treating or preventing (i.e., reducing the likelihood of occurrence) of an age-related disease or disorder that occurs as part of the natural aging process or that occurs when the subject is exposed to a senescence inducing agent or factor (e.g., irradiation, chemotherapy, smoking tobacco, high-fat/high sugar diet, other environmental factors). An age-related disorder or disease or an age-sensitive trait may be associated with a senescence-inducing stimulus. The efficacy of a method of treatment described herein may be manifested by reducing the number of symptoms of an age-related disorder or age-sensitive trait associated with a senescence-inducing stimulus, decreasing the severity of one or more symptoms, or delaying the progression of an age-related disorder or age-sensitive trait associated with a senescence-inducing stimulus. In other particular embodiments, preventing an age-related disorder or age-sensitive trait associated with a senescence-inducing stimulus refers to preventing (i.e., reducing the likelihood of occurrence) or delaying onset of an age-related disorder or age-sensitive trait associated with a senescence-inducing stimulus, or reoccurrence of one or more age-related disorder or age-sensitive trait associated with a senescence-inducing stimulus.

Age related diseases or conditions include, for example, renal dysfunction, kyphosis, herniated intervertebral disc, frailty, hair loss, hearing loss, vision loss (blindness or impaired vision), muscle fatigue, skin conditions, skin nevi, diabetes, metabolic syndrome, and sarcopenia. Vision loss refers to the absence of vision when a subject previously had vision. Various scales have been developed to describe the extent of vision and vision loss based on visual acuity. Age-related diseases and conditions also include dermatological conditions, for example without limitation, treating one or more of the following conditions: wrinkles, including superficial fine wrinkles; hyperpigmentation; scars; keloid; dermatitis; psoriasis; eczema (including seborrheic eczema); rosacea; vitiligo; ichthyosis vulgaris; dermatomyositis; and actinic keratosis.

Frailty has been defined as a clinically recognizable state of increased vulnerability resulting from aging-associated decline in reserve and function across multiple physiologic systems that compromise a subject's ability to cope with every day or acute stressors. In certain embodiments, aging and diseases and disorders related to aging may be treated or prevented (i.e., the likelihood of occurrence of is reduced) by administering the conditionally active senolytic antibody or antibody fragment or pharmaceutical composition. The conditionally active senolytic antibody or antibody fragment or pharmaceutical composition may inhibit senescence of adult stem cells or inhibit accumulation, kill, or facilitate removal of adult stem cells that have become senescent. See, e.g., Park et al, J. Clin. Invest., vol. 113, pp. 175-79, 2004 and Sousa-Victor, Nature, vol. 506, pp. 316-21, 2014) describing importance of preventing senescence in stem cells to maintain regenerative capacity of tissues.

The effectiveness of the conditionally active senolytic antibody or antibody fragment or pharmaceutical composition with respect to treating a senescent cell-associated disease or disorder described herein can readily be determined by a person skilled in the medical and clinical arts. One or any combination of diagnostic methods appropriate for the particular disease or disorder, which methods are well known to a person skilled in the art, including physical examination, patient self-assessment, assessment and monitoring of clinical symptoms, performance of analytical tests and methods, including clinical laboratory tests, physical tests, and exploratory surgery, for example, may be used for monitoring the health status of the subject and the effectiveness of the senolytic agent. The effects of the methods of treatment described herein can be analyzed using techniques known in the art, such as comparing symptoms of patients suffering from or at risk of a particular disease or disorder that have received the conditionally active senolytic antibody or antibody fragment or pharmaceutical composition with those of patients who were not treated with the conditionally active senolytic antibody or antibody fragment or pharmaceutical composition or who received a placebo treatment.

The effectiveness of the conditionally active senolytic antibody or antibody fragment or pharmaceutical composition may include beneficial or desired clinical results that comprise, but are not limited to, abatement, lessening, or alleviation of symptoms that result from or are associated with the disease to be treated; decreased occurrence of symptoms; improved quality of life; longer disease-free status (i.e., decreasing the likelihood or the propensity that a subject will present symptoms on the basis of which a diagnosis of a disease is made); diminishment of extent of disease; stabilized (i.e., not worsening) state of disease; delay or slowing of disease progression; amelioration or palliation of the disease state; and remission (whether partial or total), whether detectable or undetectable; and/or overall survival. The effectiveness of the conditionally active senolytic antibody or antibody fragment or pharmaceutical composition may also mean prolonging survival when compared to expected survival if a subject were not receiving the conditionally active senolytic antibody or antibody fragment or pharmaceutical composition.

A subject, patient, or individual in need of treatment with the conditionally active senolytic antibody or antibody fragment or pharmaceutical composition as described herein may be a human or may be a non-human primate or other animal (i.e., veterinary use) who has developed symptoms of a senescence cell-associated disease or disorder or who is at risk for developing a senescence cell-associated disease or disorder. Non-human animals that may be treated include mammals, for example, non-human primates (e.g., monkey, chimpanzee, gorilla, and the like), rodents (e.g., rats, mice, gerbils, hamsters, ferrets, rabbits), lagomorphs, swine (e.g., pig, miniature pig), equine, canine, feline, bovine, elephants, bears and other domestic, farm, and zoo animals.

### EXAMPLES

**Examples 1-9** for making conditionally active protein are described in WO 2016/138071.

### Example 10: Activity of conditionally active antibodies in different buffers

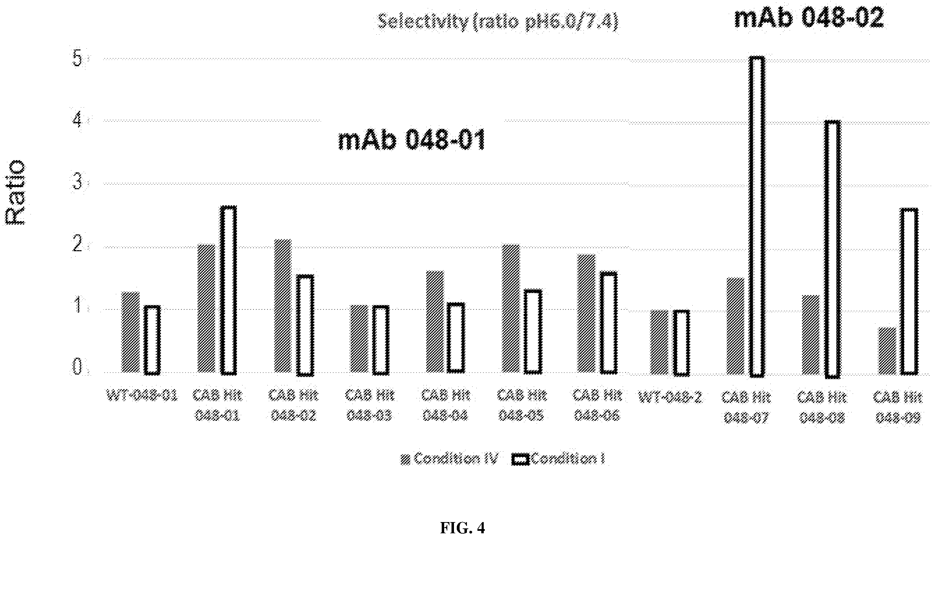

The activity of conditionally active antibodies evolved from two monoclonal antibodies (mAb 048-01 and mAb 048-02 as parent antibodies) respectively, were measured in two different buffers (FIG. 4). The two buffers were phosphate buffer (Condition IV) and Krebs buffer (Condition I). Six conditionally active antibodies were evolved from mAb 048-01: CAB Hit 048-01, CAB Hit 048-02, CAB Hit 048-03, CAB Hit 048-04, CAB Hit 048-05, and CAB Hit 048-06. Three conditionally active antibodies were evolved from mAb 048-02: CAB Hit 048-07, CAB Hit 048-08, and CAB Hit 048-09.

This study showed that the selectivity (the ratio of the activity in the assay at pH 6.0 to the activity in the assay at pH/7.4) of the conditionally active antibodies was affected by the buffer used in the assay. The conditionally active antibodies evolved from wild-type mAb 048-02 showed a significantly higher selectivity in the Krebs buffer than in the phosphate buffer (FIG. 4).

### Example 11: Selectivity of conditionally active antibodies and bicarbonate

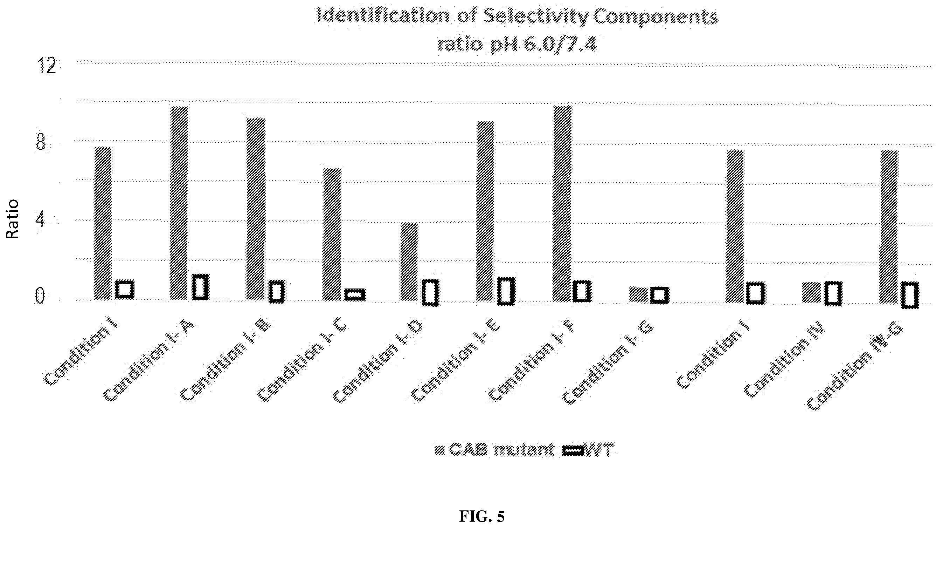

In Example 10 higher selectivity of the conditionally active antibodies was observed in Krebs buffer (Condition I) than in phosphate buffer (Condition IV). This was directed to identification of the component in the Krebs buffer that made the most significant contribution to the higher selectivity observed in Example 10. The selectivity of one conditionally active antibody was retested in buffers that were derived from Krebs buffer with various components subtracted therefrom one at a time (FIG. 5, left group of bars). When the complete Krebs buffer was used, the selectivity of the conditionally active antibody is high with an activity ratio of pH 6.0/7.4 of about 8. As components A-F were each subtracted from the Krebs buffer, the selectivity of the conditionally active antibody was not lost, though the conditionally active antibody became less selective when each of components C and D was subtracted. However, when component G (bicarbonate) was subtracted from Krebs buffer, the selectivity of the conditionally active antibody was completely lost. See FIG. 5. This indicates that bicarbonate is at least partially responsible for the high selectivity of the conditionally active antibodies in the Krebs buffer.

The selectivity of the same conditionally active antibody was then measured in phosphate buffer (Condition IV), which does not have bicarbonate and it was observed that he selectivity of the conditionally active antibody was completely lost in the phosphate buffer. When bicarbonate was added to the phosphate buffer, the selectivity of the conditionally active antibody was restored to the level observed in the Krebs buffer. This confirmed that bicarbonate was required for the selectivity of this conditionally active antibody.

### Example 12: Bicarbonate suppresses binding at pH 7.4

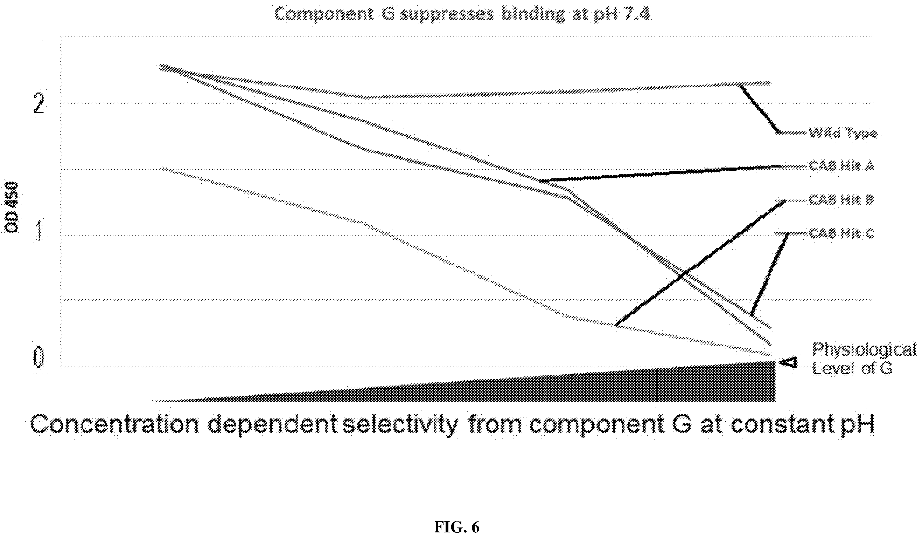

This example measured the binding activity at pH 7.4 for three conditionally active antibodies (CAB Hit A, CAB Hit B, and CAB Hit C) in buffers having different concentrations of bicarbonate ranging from 0 to the physiological concentration of bicarbonate (about 20 mM, FIG. 6). It was observed that the binding activity of all three conditionally active antibodies at pH 7.4 decreased in a dose-dependent manner as the concentration of bicarbonate increased from 0 to the physiological concentration (FIG. 6). On the other hand, the binding activity of the wild-type antibody was not affected by the bicarbonate. This study showed that the selectivity of the conditionally active antibodies in the presence of bicarbonate was likely due at least in part to loss of binding activity for the conditionally active antibodies at pH 7.4 due to interaction with the bicarbonate.

### Example 13: Induction of Senescent Cells

Cell plating: In 6-well plates, cells were seeded as: MDA-MB468 (P10), MDA-MB231 (Px) at 1.0x10⁵ cells and MCF-7 (Px) 2.0x10⁵ cells for blanks and treatment in 2 mL culture medium per well. Cells were cultured overnight.
- MCF-7 is an ERa+ cell line. Palbociclib has anti-proliferative activity in this cell line arresting cell growth and inducing senescent cells.
- MDA-MB231 is an ERa- cell line. Palbociclib has anti-proliferative activity in this cell line arresting cell growth and inducing senescent cells.
- MDA-MB468 is another ERa- cell line. Palbociclib has no anti-proliferative effect in this cell line, and thus does not arrest cell growth and fails to induce senescent cells.

Preparation of Palbociclib solution: 25 mg Palbociclib Isethionate (PD-0332991, Selechchem, Cat. S1579, Batch 4, 25mg) was added to 0.5 mL of H₂O, producing a solution with a concentration of 87.15 mM Palbociclib as a stock solution. 2.3 µL of stock solution was mixed with 198 µL H₂O, which produced a Palbociclib solution of 1 mM.

Induction of senescent cells: add 2 uL of 1 mM Palbociclib solution into 2 mL culture medium to yield a final concentration of 1 uM Palbociclib for treatment of the cultured cells (MCF-7, MDA-MB231, and MDA-MB468). The cultured cells were treated with this culture medium for 7 days to attempt to induce senescent cells.

Detection of senescent cells by FACs (co-staining of B-gal and antibodies): after 7 days treatment with Palbociclib, the cells were co-stained with SA-B-gal fluorescent substrate (C12FDG) and a panel of antibodies, and Zombie NIR live/dead dye was applied.
1. Wash the cells with PBS twice and detach cells with Detachin^{™} cell detachment solution.
2. Stop the Detachin^{™} reaction with DMEM and count the cells.
3. Stain with 2mM C12FDG (final 33uM), antibodies (5uL to 1x10^6 cells) and Zombie NIR dye (1:1000) in PBS for 1hr on ice.
4. Wash the cells with PBS twice, and fix with 4% PFA for 10 min at room temperature.
5. Wash with PBS and collect FACs in 100uL PBS.
6. Apply FITC-PE-APC/Cy7.
7. Co-Stain the cells with the following antibodies to detect expression of the corresponding antigens:
   **a)** PE anti-human CD54 Clone HCD54, 200 ug/mL, isotype: Ms IgG1. Biolegend, Catalog 322707, lot B232865, 5ul/10⁶ cells
   **b)** PE anti-human CD73 Clone AD2, isotype: Ms IgG1. Biolegend, Catalog 344004, lot B216193, 5ul/10⁶ cells
   **c)** PE anti-human CD261 (DR4, TRAIL-R1) Clone DJR1, 200 ug/mL, isotype: Ms IgG1. Biolegend, Catalog 307205, lot B189821, 5ul/10⁶ cells
   **d)** PE anti-human CD95 (Fas) Clone DX2, 100 ug/mL, isotype: Ms IgG1. Biolegend, Catalog 305607, lot B203942, 5ul/10⁶ cells
   **e)** PE anti-human CD39 Clone A1, 50 ug/mL, isotype: Ms IgG1, Biolegend, Catalog 328208, lot B199643, 5ul/10⁶ cells
   **f)** PE anti-human Nectin4, isotype: Ms IgG1. R&D systems, Catalog FAB2659P, lot AAAO0217031, 5ul/10⁶ cells
   **g)** PE-isotype mouse anti-IgG1, k: Clone MOPC-21, 0.2 mg/mL. Biolegend, Catalog 400112, lot B220359, 5ul/10⁶ cells

The induced senescent cells were detected by FACS. Stained cells were washed with PBS and fixed with 4% paraformaldehyde (PFA) for 10 min. at room temperature and used for FACS analysis. SA-B-gal (Senescence Associated B-Gal) staining using CBA-230 kit from Cell Biolabs was also performed as a control.

The cell lines (MCF-7, MDA-MB231 and MDA-MB468 cells) were observed under a microscope after the Palbociclib treatment. Further, the target profile expressed in the cell lines after the Palbociclib treatment was also analyzed by staining with corresponding antibodies. The targets profiled were Target 1 (CD54), Target 2 (CD73), Target 3 (CD261), Target 4 (CD95), Target 5 (CD39), and Target 6 (Nectin 4).

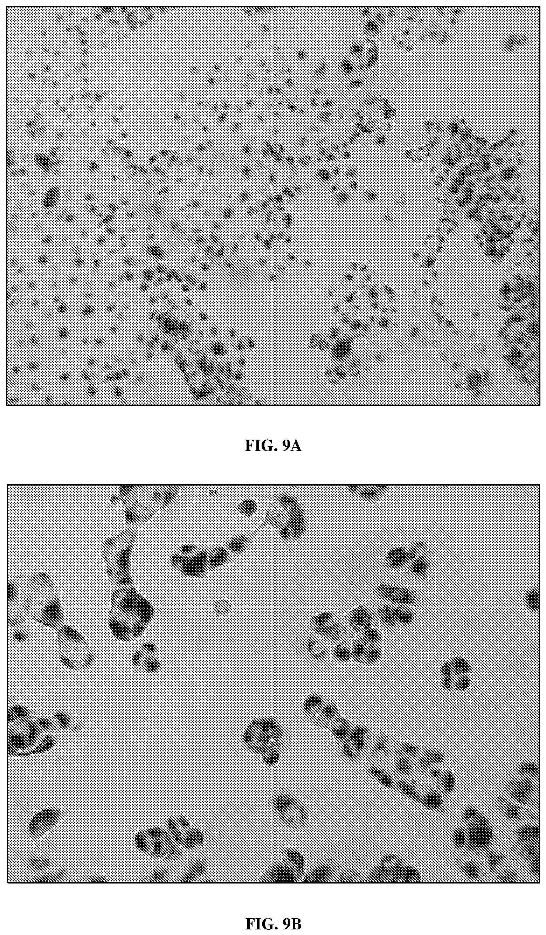

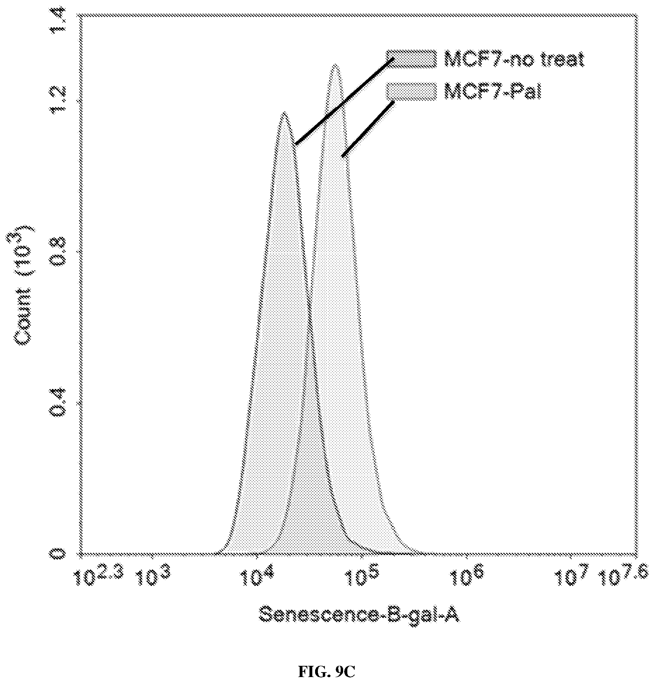

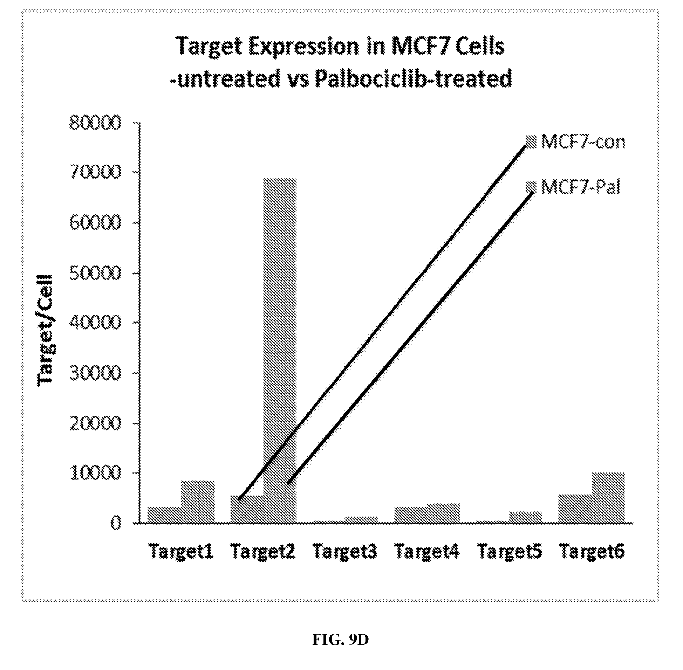

MCF-7 cells were responsive to treatment with Palbociclib, which induced the cells to become senescent cells (FIGS. 9A-9B). The MCF-7 cells formed clusters which had an extracellular environment for the senescent cells (FIG. 9B). FACS analysis clearly showed that the Palbociclib treated cells (senescent cells) were different from the untreated cells (non-senescent cells, FIG. 9C). The target profile of the Palbociclib treated cells (senescent cells) was found to be different from the untreated cells (non-senescent cells, FIG. 9D). Specifically, Targets 1, 2, and 6 were more abundantly expressed in the senescent cells, with target 2 having the greatest increase in expression level.

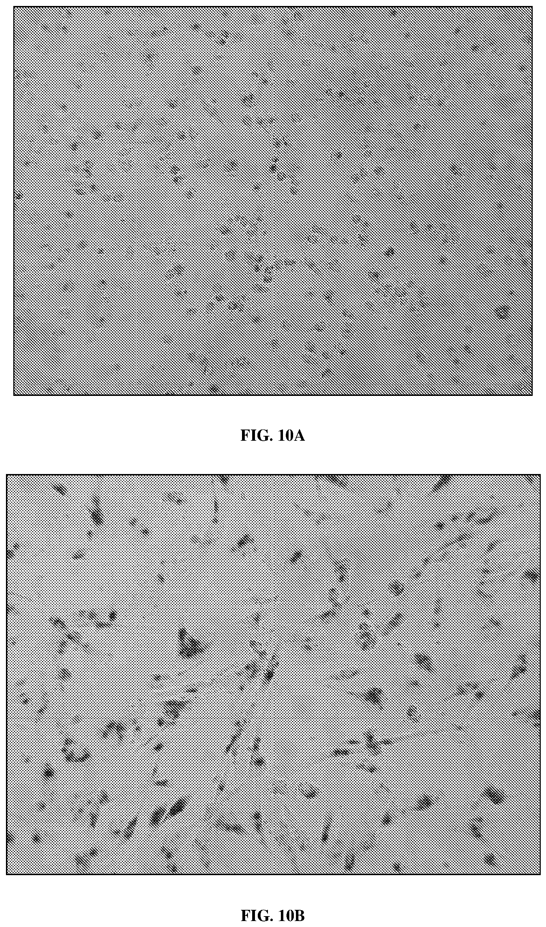

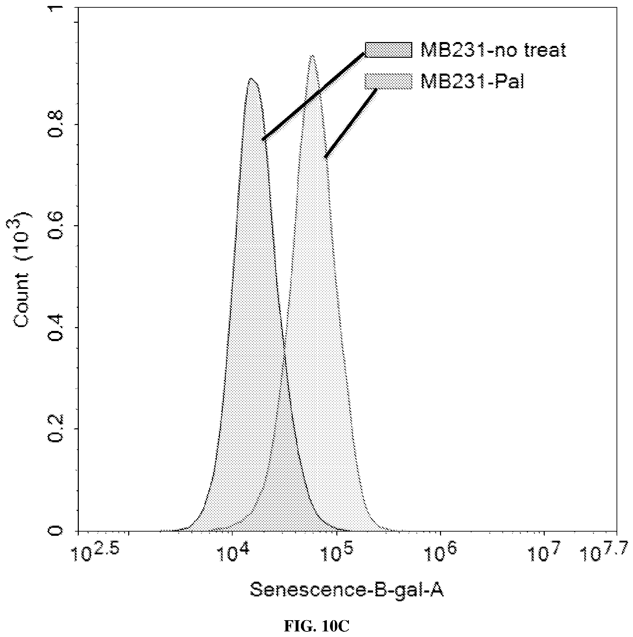

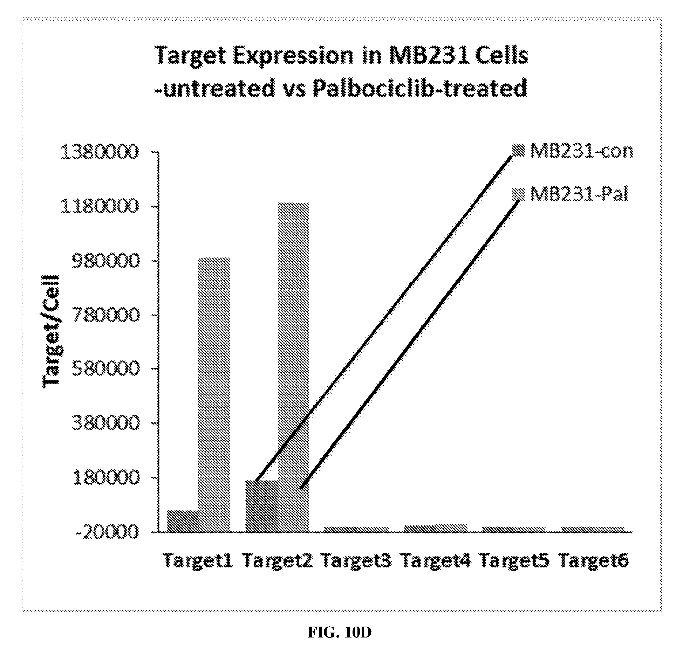

Similarly, MDA-MB231 cells were also responsive to treatment of Palbociclib, which induced the cells to become senescent cells (FIGS. 10A-10B). The MDA-MB231 cells also formed clusters which had an extracellular environment (FIG. 10B). FACS analysis clearly showed that the Palbociclib treated cells (senescent cells) were different from the untreated cells (non-senescent cells, FIG. 10C). The target profile of the Palbociclib treated cells (senescent cells) was found to be different from the untreated cells (non-senescent cells, FIG. 10D). Specifically, Targets 1 and 2 exhibited a significantly higher expression level in the senescent cells as compared to the untreated, non-senescent cells.

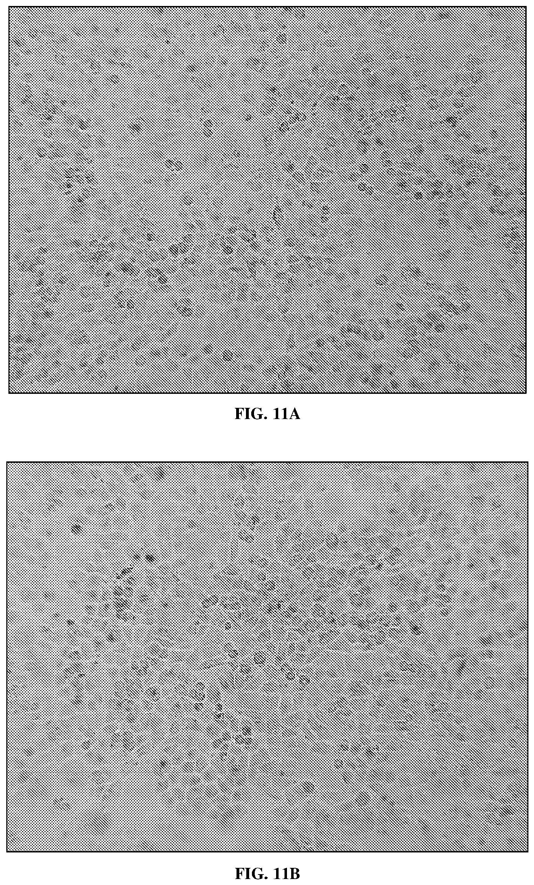

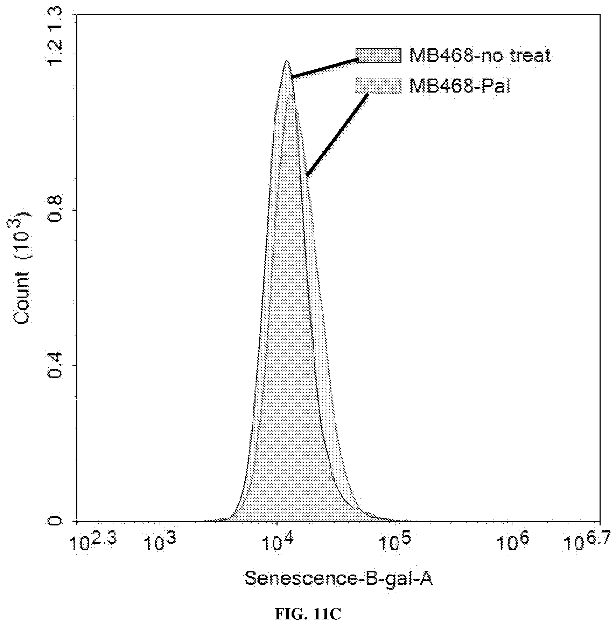

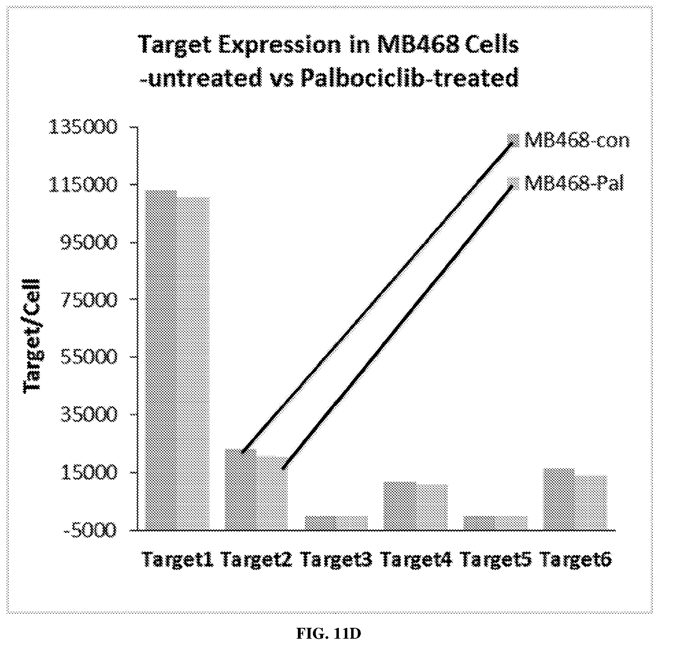

The control MDA-MB468 cells, were not responsive to treatment with Palbociclib, and thus this treatment did not induce the control cells to become senescent cells (FIGS. 11A-11B). The FACS and target profile analyses did not show any significant differences between the treated cells and untreated cells.

### Example 14: Palbociclib treatment and Beta-Galactosidase staining of MDA-MB231 Cells

MDA-MB231 cells were plated at 1x10⁵ cells/well in a 6-well plate, and cultured overnight. The cultured cells were separated into two batches: one batch was treated with 1 µM of Palbociclib Isethionate for 7 days and the other batch remained untreated. Both batches were harvested by detaching the cells from the wells.

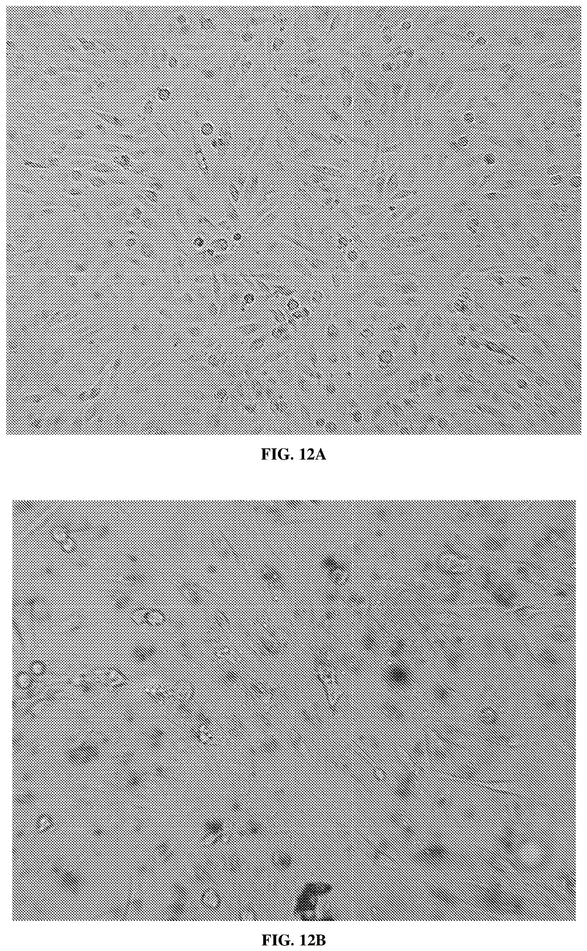

The harvested cells were stained with beta-galactosidase (B-gal) substrate (FITC) and a target antibody (anti-CD73 antibody) and live/dead dye (APC/Cy7) in PBS buffer for 1 hr on ice. The B-gal staining was performed using Cell Signaling Technologies, Cat#9860S kit. The stained MDA-MB231 cells were observed under a microscope. FIG. 12A shows that among the untreated MDA-MB231 cells there are few senescent cells since no cell clusters were observed. FIG. 12B shows the MDA-MB231 cells treated with Palbociclib. Some of the cells were induced into senescent cells that formed clusters and an extracellular environment was also present.

The stained cells, both untreated and treated, were washed with PBS and fixed with 4% paraformaldehyde for 10 min at room temperature. The fixed cells were used in FACS cell sorting.

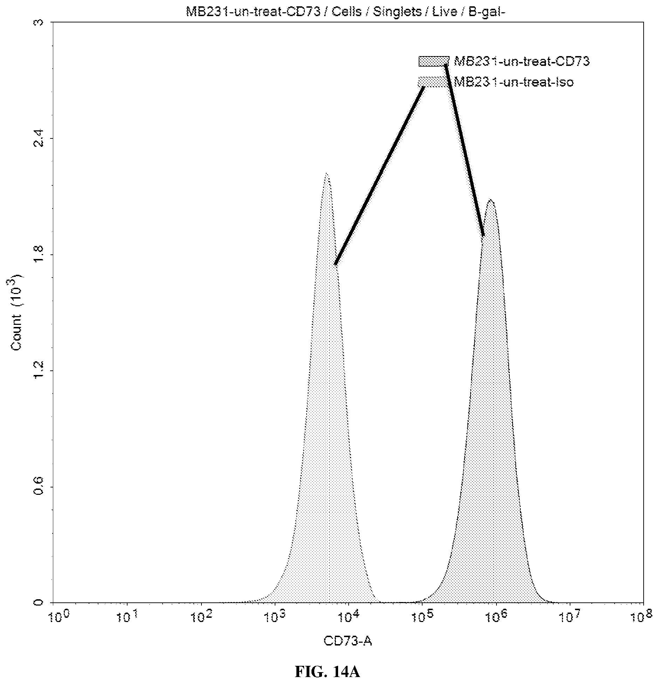

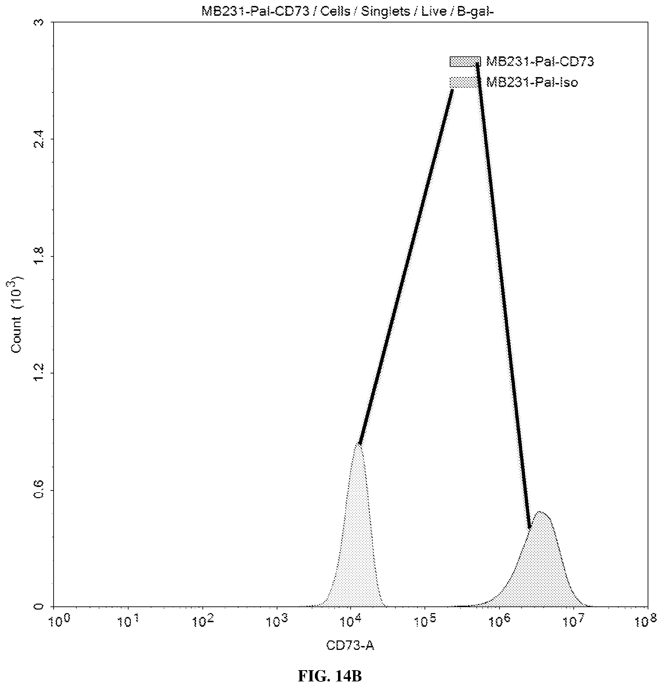

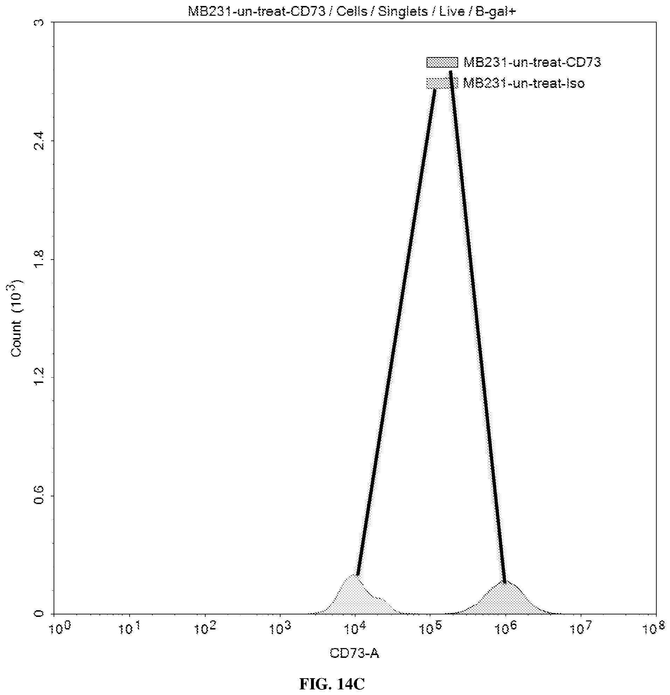

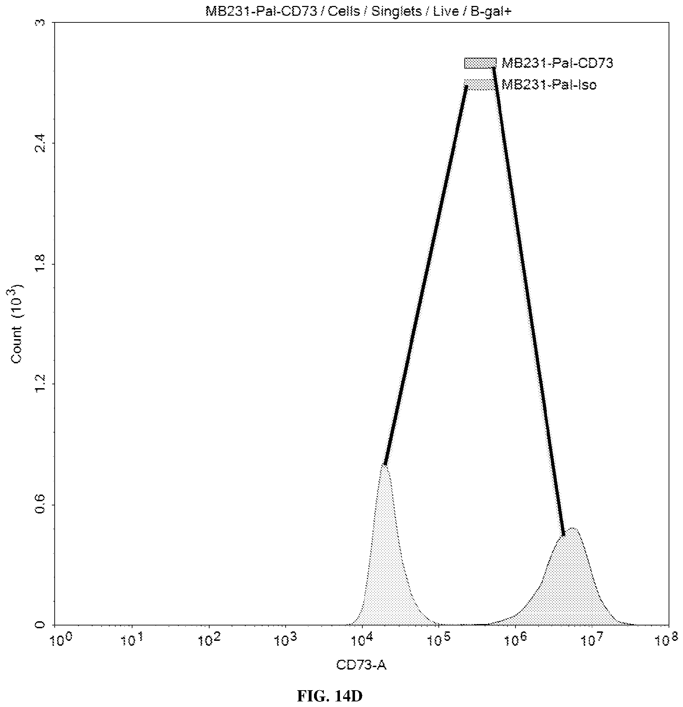

The untreated cells were mostly B-gal staining negative, though they were separated by their CD73 activities by FACS sorting (FIG. 14A). B-gal positive cells were present in significantly smaller numbers, though they were also separated by their CD73 activities by FACS sorting (FIG. 14C). In contrast, the Palbociclib treated cells had about the same number of B-gal negative cells and B-gal positive cells (FIG. 14B and 14D). Likewise, the treated cells, whether B-gal negative or B-gal positive, were separated by their CD73 activities by FACS sorting (FIG. 14B and 14D).

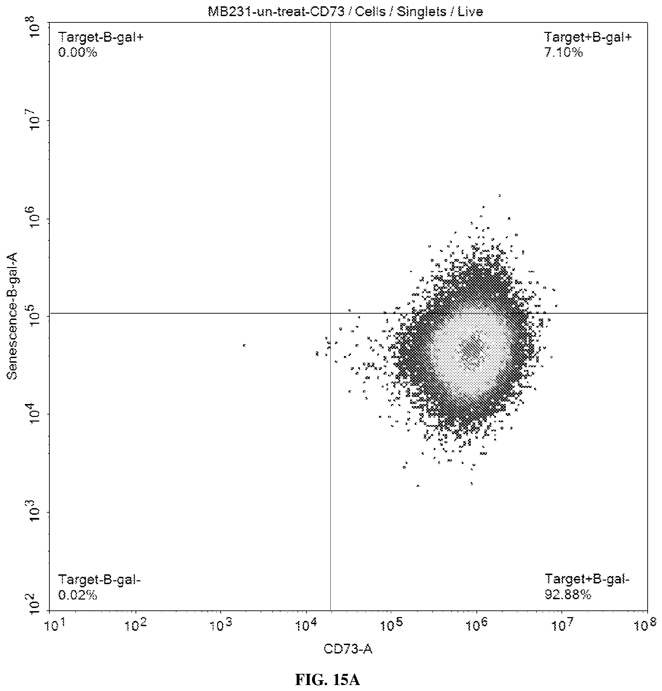

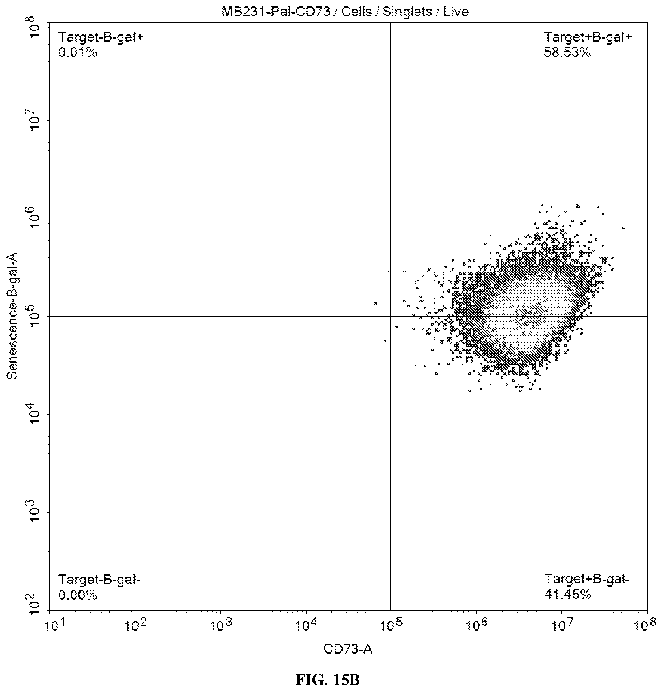

The FACS sorting results for the MDA-MB231 cells are summarized in FIGS. 15A-15B. FIG. 15A shows the untreated cells where the number of senescent cells was much smaller and the CD73 activity of the cells was at a much lower level, in comparison with the treated cells (FIG. 15B) that included a larger number of senescent cells and a higher CD73 activity.

### Example 15: Palbociclib treatment and Beta-Galactosidase staining of MDA-MB468 Cells

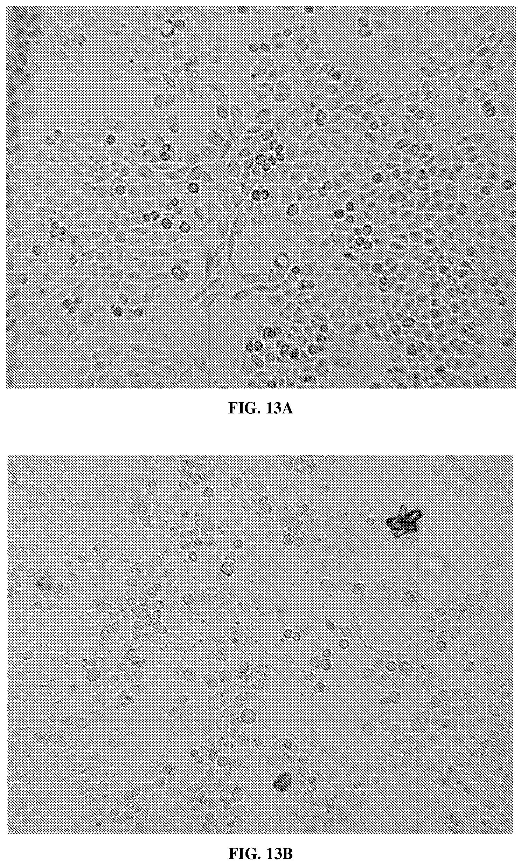

MDA-MB468 cells were cultured, stained and harvested as described for the MDA-MD231 cells in Example 14. The stained MDA-MB468 cells were observed under a microscope. FIG. 13A shows the untreated MDA-MB468 cells. FIG. 13B shows the MDA-MB468 cells treated with Palbociclib. No significant senescent cells (cell clusters) were observed after the treatment. The untreated and treated cells appeared to be similar in morphology as observed under microscope.

The stained cells, both untreated and treated with Palbociclib, were washed with PBS and fixed with 4% paraformaldehyde for 10 min at room temperature. The fixed cells were used in FACS cell sorting.

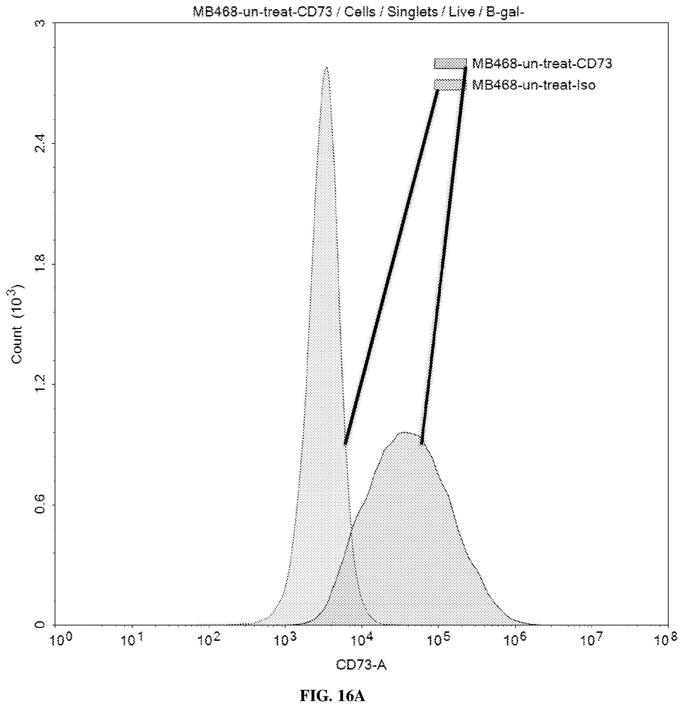

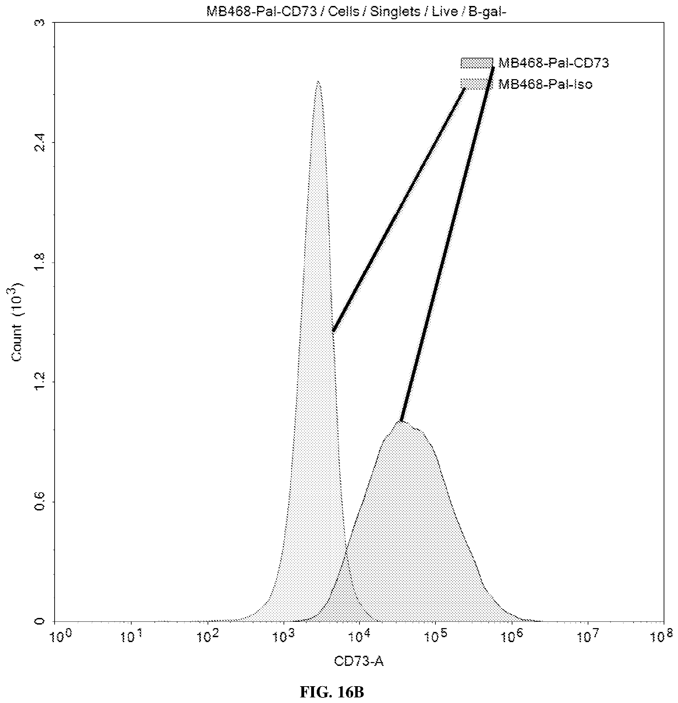

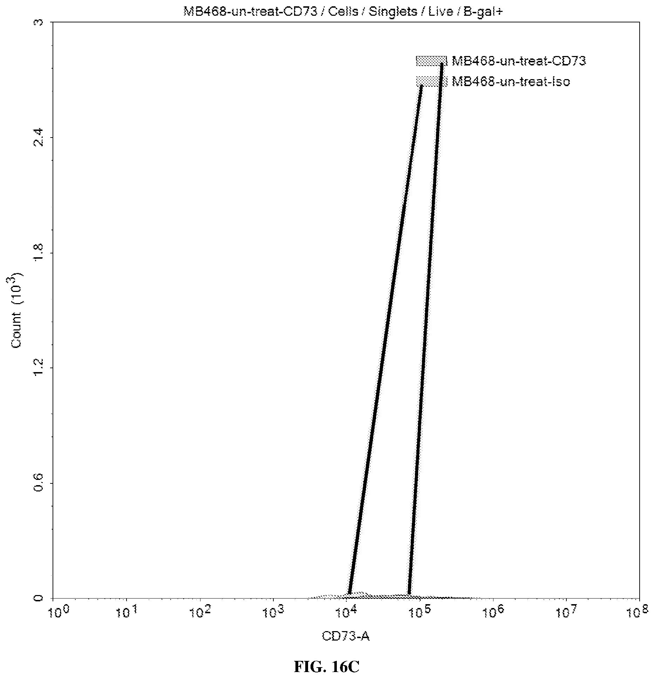

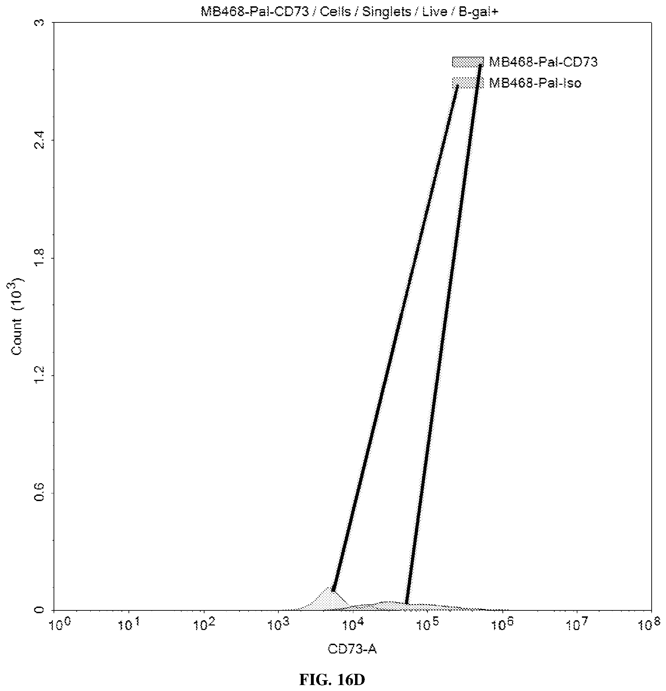

The untreated cells were mostly B-gal staining negative, though they were separated by their CD73 activities by FACS sorting (FIG. 16A). The separation was not as clear-cut as the MDA-MB231 cells in Example 14. B-gal positive cells were present in a significantly smaller number, though they were also separated by their CD73 activities by FACS sorting (FIG. 16C). Similarly, the treated cells were also mostly B-gal negative (FIGS. 16B and 16D). Likewise, the treated cells, whether B-gal negative or B-gal positive, were separated by their CD73 activities by FACS sorting, though less clear-cut than for the MDA-MB231 cells in Example 14 (FIG. 14B and 14D).

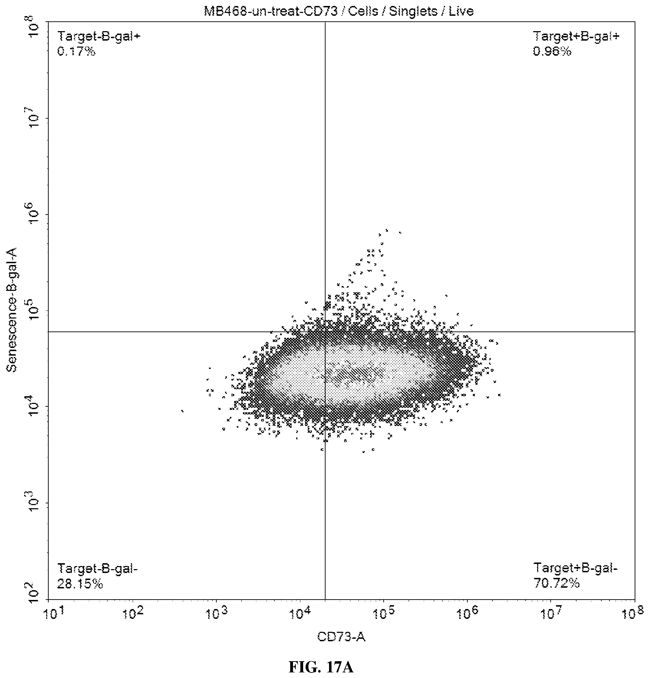

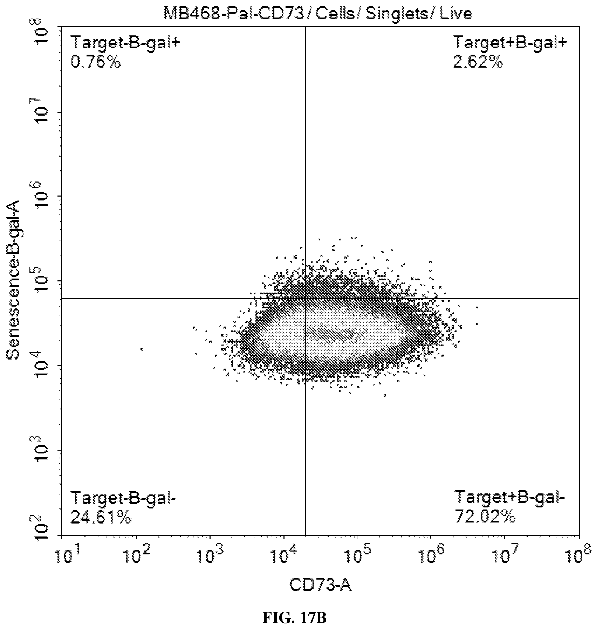

The FACS sorting results for the MDA-MB468 cells are summarized in FIGS. 17A-17B. The treated and untreated cells had similar numbers of senescent cells and levels of CD73 activity. These results indicate that the Palbociclib treatment did not induce a significant number of senescent cells.

### Example 16: Expression of CD73 in MDA-MB231 and MDA-MB468 Cells

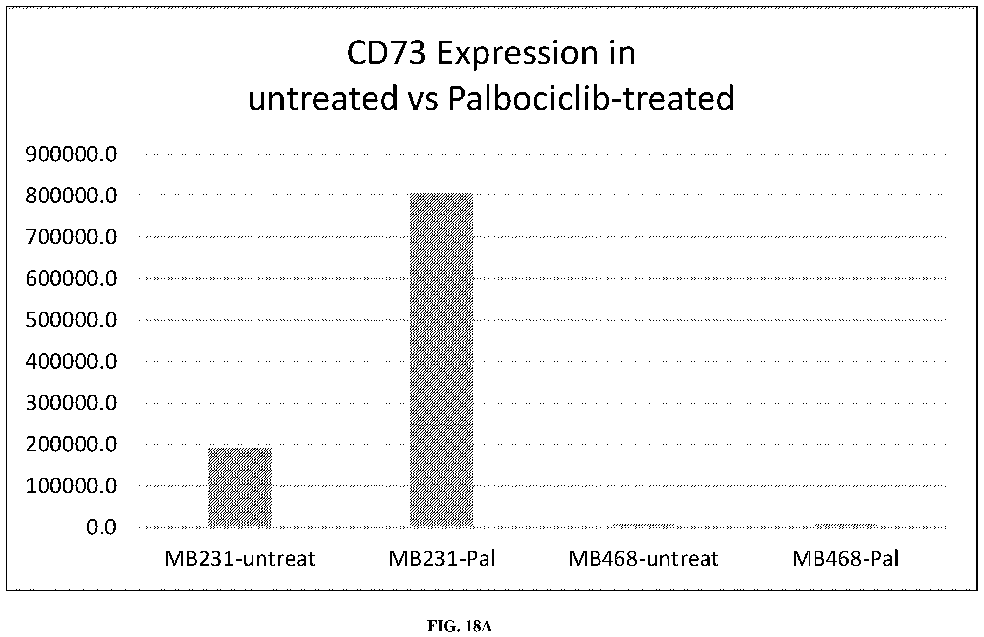

The CD73 expression levels in MDA-MB231 and MDA-MB468 cells after the Palbociclib treatment were measured (FIG. 18A). In MDA-MB231 cells, the Palbociclib treatment significantly increased the expression level of CD73 (left two bars in FIG. 18A). The untreated MDA-MB468 cells had a lower CD73 expression level than the untreated MDA-MB231 cells (first and third bars in FIG. 18A). Further, the Palbociclib treatment did not significantly increase the expression level of CD73 in the MDA-MB468 cells (right two bars in FIG. 18A).

### Example 17: Senescent cell killing as measured by a ZAP assay

ZAP assays were performed according to the protocol recommended by the manufacturer of the ZAP assay kit, Advanced Targeting Systems.

Since Palbociclib treatment was observed to induce senescent cells with increased CD73 expression in the MDA-MB231 cells, the cell killing assay (ZAP assay) was performed on the MDA-MB231 cells. Briefly, the cells were plated at 4x10³ cells/well in a 96-well plate and cultured overnight. The cultured cells were separated into two batches: one batch to be treated with 1 µM of Palbociclib Isethionate for 7 days another batch that was not treated with Palbociclib Isethionate.

Both types of MDA-MB231 cells were used in the ZAP assay. Each type of cells was assayed in four groups: ZAP assays with BAP147-CD73 (conditionally active anti-CD73 antibody), B12 (isotype negative control), Saporin (negative control), and media only (negative control). The ZAP assay was performed for 72 hrs.

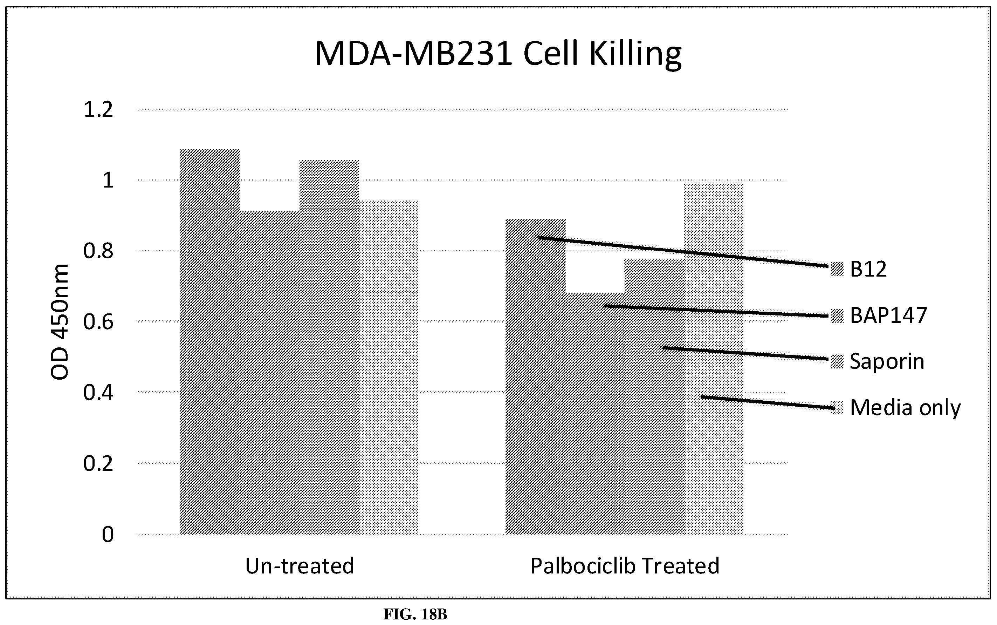

The cell killing results using the conditionally active anti-CD73 antibody and the negative controls are presented in FIG. 18B. The OD₄₅₀ₙₘ value of the Y-axis represents the total number of living cells. The media had a similar effect on the cells treated with Palbociclib and the untreated cells, which indicated that the media had no cell killing activity towards senescent cells. The conditionally active anti-CD73 antibody induced a significant reduction in the number of cells for the cells treated with Palbociclib in comparison with the untreated cells, which indicated that the conditionally active anti-CD73 antibody had a significant cell killing activity on senescent cells.

B12 appeared to have a small effect on the cells treated with Palbociclib in comparison with the untreated cells, which indicated the B12 had a small and less significant cell killing ability for senescent cells. Interestingly, Saporin also caused a similar small reduction in the number of senescent cells as compared to B12. See FIG. 18B.

This example demonstrates that conditionally active anti-CD73 antibody can target the CD73 that was overexpressed in the senescent cells induced by Palbociclib, thereby killing a significant number of these senescent cells.

## Claims

1. A method of producing a conditionally active senolytic antibody or antibody fragment that binds to a target associated with a senescent cell from a parent antibody or antibody fragment that binds to the target associated with the senescent cell, said method comprising steps of:
(i) evolving a DNA encoding the parent antibody or antibody fragment using one or more evolutionary techniques to create mutant DNAs;
(ii) expressing the mutant DNAs to obtain mutant antibodies or antibody fragments;
(iii) subjecting the mutant antibodies or antibody fragments to an assay of binding activity to the target under an extracellular pH of the senescent cell of from 5.5-7.0 and an assay of binding activity to the target under a normal physiological pH of 7.2-7.8; and
(iv) selecting the conditionally active senolytic antibody or antibody fragment from the mutant antibodies or antibody fragments subjected to the assays in step (iii) that exhibits at least one of:
(a) a decrease in binding activity to the target in the assay under the normal physiological pH of 7.2-7.8 compared to the same binding activity of the parent antibody or antibody fragment in the same assay, and an increase in binding activity to the target in the assay under the extracellular pH of 5.5-7.0 of the senescent cell compared to the same binding activity to the target of the conditionally active senolytic antibody or antibody fragment in the assay under the normal physiological pH of 7.2-7.8; and
(b) a decrease in binding activity to the target in the assay under the normal physiological pH of 7.2-7.8 compared to the same binding activity to the target of the parent antibody or antibody fragment in the same assay and an increase in the binding activity to the target in the assay under the extracellular pH of 5.5-7.0 of the senescent cell compared to the same binding activity to the target of the parent antibody or antibody fragment in the assay under the extracellular pH of 5.5-7.0 of the senescent cell; and
wherein the target is selected from at least one of APC, ARHGAP1, ARMCX-3, AXL, B2MG, BCL2L1, CAPNS2, CD261, CD39, CD54, CD73, CD95, CDC42, CDKN2C, CLYBL, COPG1, CRKL, DCR1, DCR2, DCR3, DEP1, DGKA, EBP, EBP50, FASL, FGF1, GBA3, GIT2, ICAM1, ICAM3, IGF1, ISG20, ITGAV, KITLG, LaminB1, LANCL1, LCMT2, LPHN1, MADCAM1, MAG, MAP3K14, MAPK, MEF2C, miR22, MMP3, MTHFD2, NAIP, NAPG, NCKAP1, Nectin4, NNMT, NOTCH3, NTAL, OPG, OSBPL3, p16, p16INK4a, p19, p21, p53, PAI1, PARK2, PFN1, PGM, PLD3, PMS2, POU5F1, PPP1A, PPP1CB, PRKRA, PRPF19, PRTG, RAC1, RAPGEF1, RET, Smurf2, STX4, VAMP3, VIT, VPS26A, WEE1, YAP1, YH2AX, and YWHAE.

2. The method of claim 1, wherein a ratio of the binding activity of the conditionally active senolytic antibody or antibody fragment in the assay under the extracellular pH 5.5-7.0 of the senescent cell to the binding activity of the conditionally active senolytic antibody or antibody fragment in the assay under the normal physiological pH of 7.2-7.8 is at least 1.3:1, or at least 2:1, or at least 3:1, or at least 4:1, or at least 5:1, or at least 6:1, or at least 7:1, or at least 8:1, or at least 9:1, or at least 10:1.

3. The method of any one of claims 1-2, wherein the extracellular pH of the senescent cell is a pH in a range of from 6.0 to 7.0 or from 6.2 to 6.8, and
the normal physiological pH is a pH in a range of from 7.2 to 7.6 or from 7.4 to 7.6.

4. The method of claim 1, wherein
the one or more assays are performed in assay solutions containing at least one species selected from histidine, histamine, hydrogenated adenosine diphosphate, hydrogenated adenosine triphosphate, citrate, bicarbonate, acetate, lactate, bisulfide, hydrogen sulfide, ammonium, and dihydrogen phosphate.

5. The method of any one of claims 1-3, wherein the conditionally active senolytic antibody or antibody fragment is a conditionally active antibody.

6. The method of any one of claims 1-3, further comprising a step of conjugating the conditionally active senolytic antibody or antibody fragment to a cytotoxic drug, a cytostatic drug, or an anti-proliferative drug through a linker.

7. The method of any one of claims 1-3, further comprising a step of conjugating the conditionally active senolytic antibody or antibody fragment to an agent selected from a toxic agent, radioactive agent, or D retro inverso peptide.

8. The method of claim 7, wherein the conditionally active senolytic antibody or antibody fragment is a conditionally active antibody.

## Patentansprüche

1. Verfahren zur Herstellung eines bedingt aktiven senolytischen Antikörpers oder Antikörperfragments, der bzw. das an ein mit einer seneszenten Zelle assoziiertes Zielmolekül bindet, ausgehend von einem Ausgangsantikörper oder -antikörperfragment, der bzw. das an das mit der seneszenten Zelle assoziierte Zielmolekül bindet, wobei das Verfahren die folgenden Schritte umfasst:
(i) Evolvieren einer DNA, die den Ausgangsantikörper oder das Ausgangsantikörperfragment kodiert, unter Verwendung einer oder mehrerer evolutionärer Techniken, um mutierte DNAs zu erzeugen;
(ii) Exprimieren der mutierten DNAs, um mutierte Antikörper oder Antikörperfragmente zu erhalten;
(iii) Unterziehen der mutierten Antikörper oder Antikörperfragmente einem Assay zur Bestimmung der Bindungsaktivität an das Zielmolekül bei einem extrazellulären pH-Wert der seneszenten Zelle von 5,5-7,0 sowie einem Assay zur Bestimmung der Bindungsaktivität an das Zielmolekül bei einem normalen physiologischen pH-Wert von 7,2-7,8; und
(iv) Auswählen des bedingt aktiven senolytischen Antikörpers oder Antikörperfragments aus den mutierten Antikörpern oder Antikörperfragmenten, die den Assays in Schritt (iii) unterzogen wurden, der bzw. das mindestens eines von Folgendem aufweist:
(a) eine Abnahme der Bindungsaktivität an das Zielmolekül im Assay bei dem normalen physiologischen pH-Wert von 7,2-7,8 im Vergleich zur gleichen Bindungsaktivität des Ausgangsantikörpers oder -antikörperfragments im selben Assay und eine Erhöhung der Bindungsaktivität an das Zielmolekül im Assay bei dem extrazellulären pH-Wert von 5,5-7,0 der seneszenten Zelle im Vergleich zur gleichen Bindungsaktivität an das Zielmolekül des bedingt aktiven senolytischen Antikörpers oder Antikörperfragments im Assay bei dem normalen physiologischen pH-Wert von 7,2-7,8; und
(b) eine Abnahme der Bindungsaktivität an das Zielmolekül im Assay bei dem normalen physiologischen pH-Wert von 7,2-7,8 im Vergleich zur gleichen Bindungsaktivität an das Zielmolekül des Ausgangsantikörpers oder Antikörperfragments im selben Assay und eine Erhöhung der Bindungsaktivität an das Zielmolekül im Assay bei dem extrazellulären pH-Wert von 5,5-7,0 der seneszenten Zelle im Vergleich zur gleichen Bindungsaktivität des Ausgangsantikörpers oder Antikörperfragments an das Zielmolekül im Assay bei dem extrazellulären pH-Wert von 5,5-7,0 der seneszenten Zelle; und
wobei das Zielmolekül aus mindestens einem von APC, ARHGAP1, ARMCX-3, AXL, B2MG, BCL2L1, CAPNS2, CD261, CD39, CD54, CD73, CD95, CDC42, CDKN2C, CLYBL, COPG1, CRKL, DCR1, DCR2, DCR3, DEP1, DGKA, EBP, EBP50, FASL, FGF1, GBA3, GIT2, ICAM1, ICAM3, IGF1, ISG20, ITGAV, KITLG, LaminB1, LANCL1, LCMT2, LPHN1, MADCAM1, MAG, MAP3K14, MAPK, MEF2C, miR22, MMP3, MTHFD2, NAIP, NAPG, NCKAP1, Nectin4, NNMT, NOTCH3, NTAL, OPG, OSBPL3, p16, p16INK4a, p19, p21, p53, PAI1, PARK2, PFN1, PGM, PLD3, PMS2, POU5F1, PPP1A, PPP1CB, PRKRA, PRPF19, PRTG, RAC1, RAPGEF1, RET, Smurf2, STX4, VAMP3, VIT, VPS26A, WEE1, YAP1, YH2AX und YWHAE ausgewählt ist.

2. Verfahren nach Anspruch 1, wobei das Verhältnis der Bindungsaktivität des bedingt aktiven senolytischen Antikörpers oder Antikörperfragments im Assay bei dem extrazellulären pH-Wert von 5,5-7,0 der seneszenten Zelle zur Bindungsaktivität des bedingt aktiven senolytischen Antikörpers oder Antikörperfragments im Assay bei dem normalen physiologischen pH-Wert von 7,2-7,8 mindestens 1,3:1, oder mindestens 2:1, oder mindestens 3:1, oder mindestens 4:1, oder mindestens 5:1, oder mindestens 6:1, oder mindestens 7:1, oder mindestens 8:1, oder mindestens 9:1, oder mindestens 10:1 beträgt.

3. Verfahren nach einem der Ansprüche 1 bis 2, wobei der extrazelluläre pH-Wert der seneszenten Zelle im Bereich von 6,0 bis 7,0 oder von 6,2 bis 6,8 liegt und der normale physiologische pH-Wert im Bereich von 7,2 bis 7,6 oder von 7,4 bis 7,6 liegt.

4. Verfahren nach Anspruch 1, wobei der eine oder die mehreren Assays in Assay-Lösungen durchgeführt werden, die mindestens eine chemische Spezies enthalten, ausgewählt aus Histidin, Histamin, hydriertem Adenosindiphosphat, hydriertem Adenosintriphosphat, Citrat, Bicarbonat, Acetat, Lactat, Bisulfid, Schwefelwasserstoff, Ammonium und Dihydrogenphosphat.

5. Verfahren nach einem der Ansprüche 1 bis 3, wobei der bedingt aktive senolytische Antikörper oder das bedingt aktive senolytische Antikörperfragment ein bedingt aktiver Antikörper ist.

6. Verfahren nach einem der Ansprüche 1 bis 3, ferner umfassend einen Schritt des Konjugierens des bedingt aktiven senolytischen Antikörpers oder des bedingt aktiven senolytischen Antikörperfragments über einen Linker an ein zytotoxisches Medikament, ein zytostatisches Medikament oder ein antiproliferatives Medikament.

7. Verfahren nach einem der Ansprüche 1 bis 3, ferner umfassend einen Schritt des Konjugierens des bedingt aktiven senolytischen Antikörpers oder des bedingt aktiven senolytischen Antikörperfragments an einen Wirkstoff, der aus einem toxischen Wirkstoff, einem radioaktiven Wirkstoff oder einem D-Retro-Inverso-Peptid ausgewählt ist.

8. Verfahren nach Anspruch 7, wobei der bedingt aktive senolytische Antikörper oder das bedingt aktive senolytische Antikörperfragment ein bedingt aktiver Antikörper ist.

## Revendications

1. Procédé de production d'un anticorps ou d'un fragment d'anticorps sénolytique conditionnellement actif se liant à une cible associée à une cellule sénescente, à partir d'un anticorps ou d'un fragment d'anticorps parent se liant à la cible associée à la cellule sénescente, ledit procédé comprenant des étapes suivantes :
(i) faire évoluer un ADN codant pour l'anticorps ou le fragment d'anticorps parent à l'aide d'une ou de plusieurs techniques évolutives pour créer des ADN mutants ;
(ii) exprimer les ADN mutants pour obtenir des anticorps ou fragments d'anticorps mutants ;
(iii) soumettre les anticorps ou fragments d'anticorps mutants à un dosage d'activité de liaison à la cible à un pH extracellulaire de la cellule sénescente compris entre 5,5 et 7,0 et à un dosage d'activité de liaison à la cible à un pH physiologique normal compris entre 7,2 et 7,8 ; et
(iv) sélectionner, parmi les anticorps ou fragments d'anticorps mutants soumis aux dosages à l'étape (iii), l'anticorps ou le fragment d'anticorps sénolytique conditionnellement actif qui présente au moins une des caractéristiques suivantes :
(a) une diminution de l'activité de liaison à la cible dans le dosage à un pH physiologique normal compris entre 7,2 et 7,8 par rapport à la même activité de liaison de l'anticorps ou du fragment d'anticorps parent dans le même dosage, et une augmentation de l'activité de liaison à la cible dans le dosage au pH extracellulaire compris entre 5,5 et 7,0 de la cellule sénescente par rapport à la même activité de liaison à la cible de l'anticorps ou du fragment d'anticorps sénolytique conditionnellement actif dans le dosage au pH physiologique normal compris entre 7,2 et 7,8 ; et
(b) une diminution de l'activité de liaison à la cible dans le dosage au pH physiologique normal compris entre 7,2 et 7,8 par rapport à la même activité de liaison à la cible de l'anticorps ou du fragment d'anticorps parent dans le même dosage, et une augmentation de l'activité de liaison à la cible dans le dosage au pH extracellulaire compris entre 5,5 et 7,0 de la cellule sénescente par rapport à la même activité de liaison à la cible de l'anticorps ou du fragment d'anticorps parent dans le dosage au pH extracellulaire compris entre 5,5 et 7,0 de la cellule sénescente ; et
dans lequel la cible est sélectionnée parmi au moins un des éléments suivants : APC, ARHGAP1, ARMCX-3, AXL, B2MG, BCL2L1, CAPNS2, CD261, CD39, CD54, CD73, CD95, CDC42, CDKN2C, CLYBL, COPG1, CRKL, DCR1, DCR2, DCR3, DEP1, DGKA, EBP, EBP50, FASL, FGF1, GBA3, GIT2, ICAM1, ICAM3, IGF1, ISG20, ITGAV, KITLG, LaminB1, LANCL1, LCMT2, LPHN1, MADCAM1, MAG, MAP3K14, MAPK, MEF2C, miR22, MMP3, MTHFD2, NAIP, NAPG, NCKAP1, Nectin4, NNMT, NOTCH3, NTAL, OPG, OSBPL3, p16, p16INK4a, p19, p21, p53, PAI1, PARK2, PFN1, PGM, PLD3, PMS2, POU5F1, PPP1A, PPP1CB, PRKRA, PRPF19, PRTG, RAC1, RAPGEF1, RET, Smurf2, STX4, VAMP3, VIT, VPS26A, WEE1, YAP1, YH2AX et YWHAE.

2. Procédé selon la revendication 1, dans lequel un rapport entre l'activité de liaison de l'anticorps ou du fragment d'anticorps sénolytique conditionnellement actif dans le dosage au pH extracellulaire compris entre 5,5 et 7,0 de la cellule sénescente et l'activité de liaison de l'anticorps ou du fragment d'anticorps sénolytique conditionnellement actif dans le dosage à un pH physiologique normal compris entre 7,2 et 7,8 est d'au moins 1,3:1, ou d'au moins 2:1, ou d'au moins 3:1, ou d'au moins 4:1, ou d'au moins 5:1, ou d'au moins 6:1, ou d'au moins 7:1, ou d'au moins 8:1, ou d'au moins 9:1, ou d'au moins 10:1.

3. Procédé selon l'une quelconque des revendications 1 à 2, dans lequel le pH extracellulaire de la cellule sénescente est un pH compris entre 6,0 et 7,0 ou entre 6,2 et 6,8, et le pH physiologique normal est un pH compris entre 7,2 et 7,6 ou entre 7,4 et 7,6.

4. Procédé selon la revendication 1, dans lequel lesdits un ou plusieurs dosages sont réalisés dans des solutions de dosage contenant au moins une espèce sélectionnée parmi l'histidine, l'histamine, l'adénosine diphosphate hydrogénée, l'adénosine triphosphate hydrogénée, le citrate, le bicarbonate, l'acétate, le lactate, le bisulfure, le sulfure d'hydrogène, l'ammonium et le dihydrogénophosphate.

5. Procédé selon l'une quelconque des revendications 1 à 3, dans lequel l'anticorps ou le fragment d'anticorps sénolytique conditionnellement actif est un anticorps conditionnellement actif.

6. Procédé selon l'une quelconque des revendications 1 à 3, comprenant en outre une étape consistant à conjuguer l'anticorps ou le fragment d'anticorps sénolytique conditionnellement actif à un médicament cytotoxique, à un médicament cytostatique ou à un médicament antiprolifératif au moyen d'un lieur.

7. Procédé selon l'une quelconque des revendications 1 à 3, comprenant en outre une étape consistant à conjuguer l'anticorps ou le fragment d'anticorps sénolytique conditionnellement actif à un agent sélectionné parmi un agent toxique, un agent radioactif ou un peptide D rétro-inverso.

8. Procédé selon la revendication 7, dans lequel l'anticorps ou le fragment d'anticorps sénolytique conditionnellement actif est un anticorps conditionnellement actif.
